# EUROPÄISCHE PATENTSCHRIFT

(11) **EP 0 657 441 B1**
(45) Veröffentlichungstag und Bekanntmachung des Hinweises auf die Patenterteilung: **01.03.2000**
(21) Anmeldenummer: 94116758.7
(22) Anmeldetag: 24.10.1994
(51) Int. Cl.: C07D 263/56, C07D 327/06, C07D 407/04, C07D 411/04, C07D 413/04, C07D 405/04, A01N 43/14, A01N 43/40, A01N 43/90

(54) **Cyclische Acetale, Verfahren zu deren Herstellung und deren Umsetzung zu Pflanzenschutzmitteln**
Cyclic acetals, processes for their production and conversion into herbicides
Acétals cycliques, procédé pour leur préparation et leur conversion en herbicides

(30) Priorität: 02.11.1993 DE 4337321
(43) Veröffentlichungstag der Anmeldung: 14.06.1995
(73) Patentinhaber: BASF Aktiengesellschaft, 67063 Ludwigshafen (DE)
(72) Erfinder: Rheinheimer, Joachim, Dr., D-67063 Ludwigshafen (DE); Vogelbacher, Uwe Josef, Dr., D-67071 Ludwigshafen (DE); Baumann, Ernst, Dr., D-67373 Dudenhofen (DE); König, Hartmann, Dr., D-69115 Heidelberg (DE); Gerber, Matthias, Dr., D-67117 Limburgerhof (DE); Westphalen, Karl-Otto, Dr., D-67346 Speyer (DE); Walter, Helmut, Dr., D-67283 Obrigheim (DE)

(56) Entgegenhaltungen:
- EP-A- 0 272 223
- EP-A- 0 490 224
- EP-A- 0 527 378
- CHEMICAL ABSTRACTS, vol. 120, no. 22, 30.Mai 1994 Columbus, Ohio, US; abstract no. 273469c, A. HADFIELD ET AL. 'Practical, large-scale synthesis of 2,2-dimethyl-5-hydroxy-4-oxobenzo-1,3-diox in' Seite 154;
- CHEMICAL ABSTRACTS, vol. 115, no. 12, 23.September 1991 Columbus, Ohio, US; abstract no. 119912g, M. HUNDEWADT, A. SENNING 'Enzymic and nonenzymic in vitro hydrolysis of 2-methyl-2-(2-(methoxy)phenoxy)-4H-1,3-ben zodioxin-4-one and 2-methoxyphenyl O-acetylsalicylate' Seite 345;
- CHEMICAL ABSTRACTS, vol. 97, no. 15, 11.Oktober 1982 Columbus, Ohio, US; abstract no. 127582z, J. AL-RAWI, K. F. AL-SHAHIRY 'Heterocyclic synthesis of some 2,2-dimethyl-4-oxo-substituted benzo-1,3-dioxin, and the analysis of their proton and carbon-13 NMR spectra' Seite 722;
- CHEMICAL ABSTRACTS, vol. 91, no. 23, 3.Dezember 1979 Columbus, Ohio, US; abstract no. 193238u, L. BONSIGNORE ET AL. 'Preparation of 1,3-benzodioxole and 1,3-benzodioxane derivatives' Seite 640;

## Beschreibung

Die vorliegende Erfindung betrifft cyclische Acetale der Formel I 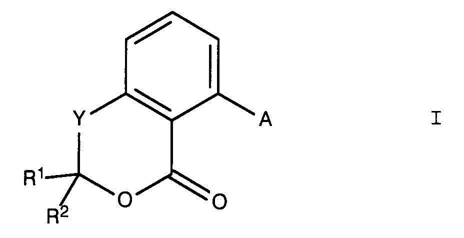 wobei die Substituenten folgende Bedeutung haben:
R¹, R²
   Wasserstoff;
C₁-C₄-Alkyl, wobei dieser Rest jeweils ein bis fünf Halogenatome und/oder ein bis zwei C₁-C₄-Alkoxy-Gruppen tragen kann;
Phenyl, wobei dieser Rest jeweils ein bis fünf Halogenatome und/oder ein bis zwei der folgenden Gruppen tragen kann: C₁-C₄-Alkyl, C₁-C₄-Alkoxy, C₁-C₄-Halogenalkyl oder Nitro;
ferner können die beiden Reste gemeinsam eine C₂-C₆-Alkylenkette sein, die die durch ein bis fünf Halogenatome und/oder C₁-C₄-Alkylreste substituiert sein kann;
Y
   Sauerstoff oder Schwefel;
A
   ein Rest A¹ bis A⁶;
A¹
   Hydroxy;
A²
   ein Halogenatom, C₁-C₄-Halogenalkylsulfonyloxy, C₁-C₄-Alkylsulfonyloxy oder Fluorsulfonyloxy;
A³
   Cyano, Nitro, Formyl;
A⁴
   ein Phenylring oder ein 5- oder 6-gliedriger gesättigter oder ungesättigter Heterocyclus mit bis zu vier Heteroatomen aus der Gruppe: Stickstoff, Schwefel, Sauerstoff im Ring, die jeweils durch bis zu fünf Reste R³¹ bis R³⁵ substituiert sein können;
Naphthyl oder ein benzokondensierter 5- oder 6-gliedriger Heteroaromat mit 1 bis 3 Heteroatomen aus der Gruppe: Stickstoff, Schwefel, Sauerstoff im Ring, der durch bis zu fünf Reste R³¹ bis R³⁵ substituiert sein kann;
A⁵
   eine C₂-C₆-Alkenyl-, C₃-C₈-Cycloalkenyl-, C₅-C₈-Cycloalkadienyl- oder C₂-C₆-Alkinyl-Gruppe, die jeweils bis zu sieben Substituenten R³¹ bis R³⁷ tragen können;
A⁶
   eine C₁-C₈-Alkyl- oder C₃-C₈-Cycloalkyl-Gruppe, die jeweils bis zu sieben Substituenten R³¹ bis R³⁷ tragen können;
R³¹, R³², R³³, R³⁴, R³⁵, R³⁶, R³⁷
   a) eine C₃-C₈-Cycloalkylgruppe, welche ein bis drei C₁-C₄-Alkylreste tragen kann;
   b) eine C₁-C₈-Alkylgruppe, welche ein bis fünf Halogenatome und/oder einen der folgenden Reste tragen kann:
      C₁-C₄-Alkoxy, C₁-C₄-Halogenalkoxy, C₁-C₄-Alkylthio, Cyano, C₃-C₈-Cycloalkyl oder Di-C₁-C₄-Alkylamino;
   c) eine C₁-C₈-Alkoxygruppe oder eine C₃-C₅-Cycloalkoxygruppe, welche jeweils ein bis fünf Halogenatome und/oder einen der folgenden Reste tragen können:
      C₁-C₄-Alkoxy, C₁-C₄-Halogenalkoxy, C₁-C₄-Alkylthio, C₃-C₈-Cycloalkyl oder Di-C₁-C₄-Alkylamino;
   d) eine C₁-C₄-Alkylthiogruppe, welche ein bis fünf Halogenatome und/oder einen der folgenden Reste tragen kann:
      C₁-C₄-Alkoxy, C₁-C₄-Halogenalkoxy, C₁-C₄-Alkylthio, C₃-C₈-Cycloalkyl oder Di-C₁-C₄-Alkylamino;
   e) eine Di-C₁-C₄-Alkylamino-, eine Di-C₁-C₄-Alkylaminoxygruppe, eine C₅-C₈-Cycloalkaniminoxygruppe oder eine C₁-C₁₀-Alkaniminoxygruppe;
   f) eine C₂-C₆-Alkenyl- oder eine C₂-C₆-Alkinylgruppe, welche ein bis fünf Halogenatome und/oder einen der folgenden Reste tragen kann:
      C₁-C₄-Alkoxy, C₁-C₄-Halogenalkoxy oder C₁-C₄-Alkylthio;
   g) Wasserstoff;
   h) Nitro, Halogen, Cyano, Tri-C₁-C₄-alkylsilyl;
mit der Maßgabe, daß A nicht für eine Hydroxy-Gruppe steht, wenn R¹ und R² beide Wasserstoff bedeuten oder wenn R¹ Wasserstoff und R² Phenyl bedeutet.

Die voranstehend genannten Verbindungen dienen insbesondere als Zwischenprodukte zur Herstellung von Pflanzenschutzmitteln, z.B. von substituierten Salicylsäurederivaten, die gemäß dem Stand der Technik (z.B. DE-A 39 19 435, EP-A 346 789, EP-A 490 060, WO 93/03017, EP-A 249 708, 287 072, 287 079 und 315 889) gute herbizide und/oder bioregulatorische Wirkung aufweisen. Sie zeigen aber auch selber eine gute herbizide Wirkung.

Die Herstellung von 1,3-Benzodioxolen und Benzodioxanderivaten wird in Chemical Abstracts 91 (23): 193238u (L. Bonsignore et al., Rend. Semin. Fac. Sci. Univ. Cagliari, Bd. 48, S. 275-283 (1978)) beschrieben.

Der Erfindung lag nun die Aufgabe zugrunde, den Zugang zu verschieden substituierten Salicylsäurederivaten, z.B. den in obengenannten Publikationen beschriebenen Verbindungen, zu erleichtern.

Demgemäß wurden die eingangs genannten cyclischen Acetale I gefunden. Gegenstand der Erfindung sind ferner chemisch eigenartige Verfahren zur Herstellung der Acetale I, herbizide Mittel, enthaltend die Verbindungen I und ihre Verwendung zur Bekämpfung unerwünschten Pflanzenwuchses.

In der Beschreibung haben die unten genannten Substituenten bevorzugt folgende Bedeutung:
C₁-C₄-Alkyl: Methyl, Ethyl, 1-Propyl, 2-Propyl, 2-Methyl-2 propyl, 2-Methyl-1-propyl, 1-Butyl, 2-Butyl;
C₁-C₈-Alkyl: C₁-C₄-Alkyl sowie Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl;
C₁-C₂-Halogenalkyl: Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor 2-fluorethyl, 2,2,2-Trichlorethyl und Pentafluorethyl;
C₁-C₂-Halogenalkoxy: Difluormethoxy, Trifluormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy;
C₁-C₄-Alkoxy: Methoxy, Ethoxy, Propoxy, 1-Methylethoxy, Butoxy, 1-Methylpropoxy, 2-Methylpropoxy, 1,1-Dimethylethoxy, insbesondere Methoxy, Ethoxy, 1-Methylethoxy;
C₁-C₄-Alkylthio: Methylthio, Ethylthio, Propylthio, 1-Methylethylthio, Butylthio, 1-Methylpropylthio, 2-Methylpropylthio, 1,1-Dimethylethylthio, insbesondere Methylthio und Ethylthio;
C₃-C₆-Alkenyl: 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl;
C₃-C₆-Alkinyl: 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-Pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
Halogen: Chlor, Brom, Iod, Fluor;
C₃-C₈-Cycloalkenyl: Cyclopropenyl, Cyclobutenyl, Cyclopentenyl, Cycohexenyl, Cycloheptenyl, Cyclooctenyl, besonders bevorzugt Cyclopentenyl, Cycloheptenyl und Cyclooctenyl;
C₅-C₈-Cycloalkadienyl: Cyclopentadienyl, 1,3-Cyclohexadienyl, 1,4-Cyclohexadienyl, 1,3-Cycloheptadienyl, 1,4-Cycloheptadienyl, 1,3-Cyclooctadienyl, 1,4-Cyclohoctadienyl, 1,5-Cyclohoctadienyl;
C₃-C₈-Cycloalkyl Cyclopropyl, Cyclobutyl, Cyclopentyl, Cyclohexyl, Cycloheptyl und Cycloheptyl, besonders bevorzugt Cyclopropyl, Cyclopentyl und Cyclohexyl;
Di-C₁-C₄-Alkylaminoxy: Dimethylaminoxy, N-Methyl-N-ethylaminoxy, Diethylaminoxy, N-Methyl-N-propylaminoxy, N-Ethyl-N-propylaminoxy, Dipropylaminoxy, Diisopropylaminoxy, N-Isopropyl-N-methylaminoxy, N-Ethyl-N-isopropylaminoxy, N-Isopropyl-N-propylaminoxy, Dibutylaminoxy, Di-2-methylpropylaminoxy, Di-1-methylpropylaminoxy, N-Butyl-N-methylaminoxy sowie Isomere, N-Butyl-N-ethylaminoxy sowie Isomere, N-Butyl-N-propylaminoxy sowie Isomere;
C₁-C₁₀-Alkaniminoxy: Methaniminoxy, Ethaniminoxy, 1-Propaniminoxy, 2-Propaniminoxy, 1-Butaniminoxy, 2-Butaniminoxy, 2-Methylpropan-1-iminoxy, 1-Pentaniminoxy, 2-Pentaniminoxy, 3-Pentaniminoxy, 3-Methylbutan-2-iminoxy, 3-Methylbutan-1-iminoxy, 2-Methylbutan-1-iminoxy, 2,2-Dimethylpropan-1-iminoxy, Hexaniminoxy sowie Isomere, Heptaniminoxy sowie Isomere, Octaniminoxy sowie Isomere, Nonaniminoxy sowie Isomere, Decaniminoxy sowie Isomere; ganz besonders bevorzugt sind 2-Propaniminoxy, 2-Butaniminoxy, 2-Pentaniminoxy, 3-Pentaniminoxy, 2-Hexaniminoxy, 3-Hexaniminoxy sowie 2,2-Dimethylpropan-1-iminoxy;
C₅-C₈-Cycloalkaniminoxy: Cyclopentaniminoxy, Cyclohexaniminoxy, Cycloheptaniminoxy, Cyclooctaniminoxy;
Di-C₁-C₄-Alkylamino: Dimethylamino, N-Methyl-N-ethylamino, Diethylamino, N-Methyl-N-propylamino, N-Ethyl-N-propylamino, Dipropylamino, Diisopropylamino, N-Isopropyl-N-methylamino, N-Ethyl-N-isopropylamino, N-Isopropyl-N-propylamino, Dibutylamino, Di-2-methylpropylamino, Di-1-methylpropylamino, N-Butyl-N-methylamino sowie Isomere, N-Butyl-N-ethylamino sowie Isomere, N-Butyl-N-propylamino sowie Isomere.

Im Hinblick auf die bestimmungsgemäße Verwendung der Endprodukte (z.B. Salicylsäurederivate IV) sind Verbindungen der Formel I als Zwischenprodukte bevorzugt, bei denen die Substituenten folgende Bedeutung haben:
R¹, R²
   Wasserstoff;
C₁-C₄-Alkyl, wobei dieser Rest jeweils ein bis fünf Halogenatome und/oder ein bis zwei C₁-C₄-Alkoxy-Gruppen tragen kann, ganz besonders bevorzugt sind Methyl und Ethyl;
Phenyl, wobei dieser Rest jeweils ein bis fünf Halogenatome und/oder ein bis zwei der folgenden Gruppen tragen kann: C₁-C₄-Alkyl, C₁-C₄-Alkoxy, C₁-C₄-Halogenalkyl, besonders C₁-C₂-Halogenalkyl oder Nitro, ganz besonders bevorzugt ist Phenyl;
ferner können die beiden Reste gemeinsam eine C₂-C₆-Alkylenkette sein, wie eine 1,2-Ethylen-1,3-Propylen, 1,4-Butylen-, 1,5-Pentylen- oder 1,6-Hexylengruppe, die die durch ein bis fünf Halogenatome und/oder C₁-C₄-Alkylreste substituiert sein kann, ganz besonders bevorzugt sind die 1,4-Butylen- und 1,5-Pentylengruppen;
A
   ein Rest A¹ bis A⁶;
A¹
   Hydroxy;
A²
   ein Halogenatom, C₁-C₄-Halogenalkylsulfonyloxy, C₁-C₄-Alkylsulfonyloxy oder Fluorsulfonyloxy;
A³
   Cyano, Nitro, Formyl;
A⁴
   ein Phenylring oder ein 5- oder 6-gliedriger gesättigter oder ungesättigter Heterocyclus mit ein bis vier Heteroatomen aus der Gruppe: Stickstoff, Schwefel, Sauerstoff im Ring, wie Phenyl, Pyridin-2-yl, Pyridin-3-yl, Pyridin-4-yl, Pyrimidin-2-yl, Pyrimidin-4-yl, Pyrimidin-5-yl, Pyrazin-2-yl, Pyridazin-3-yl, Pyridazin-4-yl, 1,3,5-Triazin-2-yl, 1,2,4-Triazin-3-yl, 1,2,4-Triazin-5-yl, 1,2,4-Triazin-6-yl, 1,2,4,5-Tetrazin-3-yl, Tetrahydrothiopyran-2-yl, Tetrahydrothiopyran-3-yl, Tetrahydrothiopyran-4-yl, Tetrahydropyran-2-yl, Tetrahydropyran-3-yl, Tetrahydropyran-4-yl, Dihydropyranyl, Dihydrothiopyranyl, Piperidin-2-yl, Piperidin-3-yl, Piperidin-4-yl, Morpholin-2-yl, Morpholin-3-yl, Furan-2-yl, Furan-3-yl, 2-Thienyl, 3-Thienyl, Tetrahydrofuran-2-yl, Tetrahydrofuran-3-yl, 2,3-Dihydrofuran-3-yl, 2,3-Dihydrofuran-2-yl, 2,3-Dihydrofuran-4-yl, 2,3-Dihydrofuran-5-yl, 3,4-Dihydrofuran-2-yl, 3,4-Dihydrofuran-3-yl, 2,3-Dihydrothien-3-yl, 2,3-Dihydrothien-2-yl, 2,3-Dihydrothien-4-yl, 2,3-Dihydrothien-5-yl, 3,4-Dihydrothien-2-yl, 3,4-Dihydrothien-3-yl, Tetrahydrothien-2-yl, Tetrahydrothien-3-yl, 2-Pyrrolyl, 3-Pyrrolyl, 2-Pyrrolidinyl, 3-Pyrrolidinyl, Imidazol-2-yl, Imidazol-4-yl, Imidazol-5-yl, Pyrazol-3-yl, Pyrazol-4-yl, Pyrazol-5-yl, 1,2,3-Triazol-4-yl, 1,2,3-Triazol-5-yl, 1,2,4-Triazol-3-yl, 1,2,4-Triazol-5-yl, Tetrazol-5-yl, 1,2,3-Thiadiazol-4-yl, 1,2,3-Thiadiazol-5-yl, 1,2,5-Thiadiazol-3-yl, 1,3,4-Thiadiazol-2-yl, 1,2,4-Thiadiazol-3-yl, 1,2,4-Thiadiazol-5-yl, 1,2,3-Oxadiazol-4-yl, 1,2,3-Oxadiazol-5-yl, 1,2,5-Oxadiazol-3-yl, 1,3,4-Oxadiazol-2-yl, 1,2,4-Oxadiazol-3-yl, 1,2,4-Oxadiazol-5-yl, 1,3-Dioxolan-2-yl, 1,3-Dioxolan-4-yl, 1,3-Dithiolan-2-yl, 1,3-Dithiolan-4-yl, Oxazol-2-yl, Oxazol-4-yl, Oxazol-5-yl, Oxazolin-2-yl, Oxazolin-4-yl, Oxazolin-5-yl, Isoxazol-2-yl, Isoxazol-4-yl, Isoxazol-5-yl, Isoxazolin-2-yl, Isoxazolin-4-yl, Isoxazolin-5-yl, Thiazol-2-yl, Thiazol-4-yl, Thiazol-5-yl, Thiazolin-2-yl, Thiazolin-4-yl, Thiazolin-5-yl, Isothiazol-2-yl, Isothiazol-4-yl, Isothiazol-5-yl, Isothiazolin-2-yl, Isothiazolin-4-yl, Isothiazolin-5-yl, die jeweils durch bis zu fünf Reste R³¹-R³⁵ substituiert sein können;
Naphthyl oder ein benzokondensierter 5- oder 6-gliedriger Heteroaromat mit ein bis drei Heteroatomen aus der Gruppe: Stickstoff, Schwefel, Sauerstoff im Ring, wie Benzofuranyl, Benzothienyl, 2,1,3-Benzothiadiazolyl, Indolyl, Indazolyl, Benzotriazolyl, 1,2,3-Benzothiadiazolyl, Benzoxazolyl, Benzisoxazolyl, Benzthiazolyl, Benzisothiazolyl, Chinolinyl, Cinnolinyl, Chinoxalinyl, Phthalazinyl oder Benzofuroxanyl, der durch bis zu fünf Reste R³¹-R³⁵ substituiert sein kann;
A⁵
   eine C₂-C₆-Alkenyl-, C₃-C₈-Cycloalkenyl-, C₅-C₈-Cycloalkadienyl- oder C₂-C₆-Alkinyl-Gruppe, die jeweils bis zu 7 Substituenten R³¹-R³⁷ tragen können;
A⁶
   eine C₁-C₈-Alkyl- oder C₃-C₈-Cycloalkyl-Gruppe, die jeweils bis zu 7 Substituenten R³¹-R³⁷ tragen können;
R³¹, R³², R³³, R³⁴, R³⁵, R³⁶, R³⁷
   a) eine C₃-C₈-Cycloalkylgruppe, welche ein bis drei C₁-C₄-Alkylreste tragen kann;
   b) eine C₁-C₈-Alkylgruppe, welche ein bis fünf Halogenatome und/oder einen der folgenden Reste tragen kann:
      C₁-C₄-Alkoxy, C₁-C₄-Halogenalkoxy, besonders C₁-C₂-Halogenalkoxy, C₁-C₄-Alkylthio, Cyano, C₃-C₈-Cycloalkyl oder Di-C₁-C₄-Alkylamino;
   c) eine C₁-C₈-Alkoxygruppe oder eine C₃-C₅-Cycloalkoxygruppe, welche jeweils ein bis fünf Halogenatome und/oder einen der folgenden Reste tragen können:
      C₁-C₄-Alkoxy, C₁-C₄-Halogenalkoxy, C₁-C₄-Alkylthio, C₃-C₈-Cycloalkyl oder Di-C₁-C₄-Alkylamino;
   d) eine C₁-C₄-Alkylthiogruppe, welche ein bis fünf Halogenatome und/oder einen der folgenden Reste tragen kann:
      C₁-C₄-Alkoxy, C₁-C₄-Halogenalkoxy, besonders C₁-C₂-Halogenalkoxy, C₁-C₄-Alkylthio, C₃-C₈-Cycloalkyl oder Di-C₁-C₄-Alkylamino;
   e) eine Di-C₁-C₄-Alkylamino-, eine Di-C₁-C₄-Alkylaminoxygruppe, eine C₅-C₈-Cycloalkaniminoxygruppe oder eine C₁-C₁₀-Alkaniminoxygruppe;
   f) eine C₂-C₆-Alkenyl- oder eine C₂-C₆-Alkinylgruppe, welche ein bis fünf Halogenatome und/oder einen der folgenden Reste tragen kann:
      C₁-C₄-Alkoxy, C₁-C₄-Halogenalkoxy oder C₁-C₄-Alkylthio;
   g) Wasserstoff;
   h) Nitro, Halogen, Cyano, Tri-C₁-C₄-alkylsilyl;
Y Sauerstoff oder Schwefel.

Die Herstellung der cyclischen Acetale mit A=A¹, A², A³ und A⁶ erfolgt, indem man die zugrunde liegenden 6-Hydroxybenzoesäuren (für Y=0) bzw. 6-Mercaptobenzoesäuren (für Y=S) mit einer Verbindung O=CR¹R² in Gegenwart eines sauren Katalysators umsetzt: 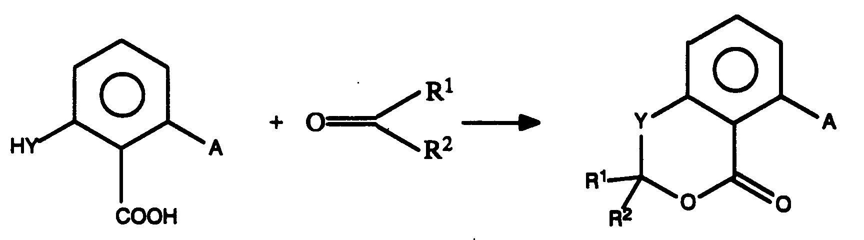
(A = A¹, A², A³ oder A⁶)

Die Ausgangsstoffe sind allgemein bekannt oder in allgemein bekannter Weise zugänglich.

Man kann zur Bindung des bei der Reaktion entstehenden Wassers ein wasserentziehendes Mittel zusetzen wie z.B. Molsieb, Säureanhydride, wie Trifluoracetanhydrid, Acetanhydrid, Natriumsulfat, Calciumchlorid, wenn das den Umsatz fördert. Geeignet ist auch ein reaktionsfähiges Acetal der Verbindung O=CR¹R², wie z.B. ein Dimethylacetal, Diethylacetal, Ethylenacetal oder Propylenacetal. Als Katalysatoren eignen sich besonders saure Ionenaustauscher, Protonensäuren wie z.B. Toluolsulfonsäure, Schwefelsäure, Phosphorsäure usw. oder Lewis-Säuren wie z.B. Aluminiumchlorid, Titantetrachlorid, Zinkchlorid, Calciumchlorid, Magnesiumchlorid, Zinnchloride usw. Oftmals lassen sich das gebildete Wasser oder der aus dem Acetal gebildete Alkohol direkt aus der Reaktionsmischung abdestillieren. Die cyclischen Acetale mit A=A¹ lassen sich an der Hydroxylgruppe weiter modifizieren, indem man sie z.B. mit Sulfonsäureanhydriden oder -halogeniden umsetzt (s. Synthesebeispiele).

Cyclische Acetale I mit A=A⁴ oder A⁵ erhält man besonders vorteilhaft durch Umsetzung von Verbindungen I mit A=A² und Verbindungen X-A⁴ oder X-A⁵ in Gegenwart einer katalytisch wirksamen Palladiumverbindung gemäß folgendem Reaktionsschema:

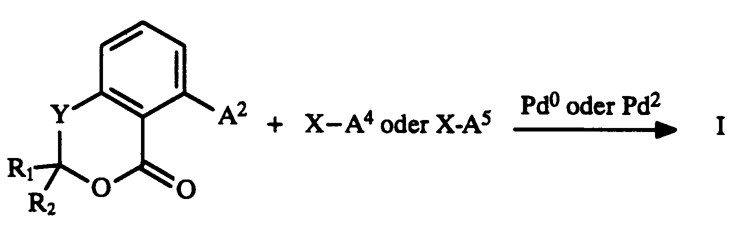

Dabei haben R¹, R², Y und A⁴ oder A⁵ die oben beschriebenen Bedeutungen und A² steht z.B. für Chlor, Brom, Iod, C₁-C₄-Halogenalkylsulfonyloxy, insbesondere Trifluormethylsulfonyloxy, C₁-C₄-Alkylsulfonyloxy oder Fluorsulfonyloxy.

X bedeutet Wasserstoff, insbesondere wenn A für A⁵ steht, Trialkylstannyl, z.B. Tri-C₁-C₈-alkylstannyl wie Trimethylstannyl, Triethylstannyl, Tripropylstannyl, Tributylstannyl, Tripentylstannyl oder Trihexylstannyl, Dihydroxyboranyl, Dialkoxyboranyl, z.B. Di-C₁-C₄-alkoxyboranyl wie Dimethoxyboranyl, Diethoxyboranyl, Dipropoxyboranyl, Diisopropoxyboranyl oder Dibutoxyboranyl oder Isomere oder Alkylendioxyboranyl, z.B. C₁-C₄-Alkylendioxyboranyl wie Ethylendioxyboranyl oder 1,3-Propylendioxyboranyl. Die verwendeten Bor- oder Zinnverbindungen sind entweder bekannt oder analog zu bekannten Verbindungen herstellbar.

Bei diesem neuen Verfahren wird eine katalytisch wirksame Palladiumverbindung eingesetzt. Dabei sind beliebige Palladiumsalze oder -Komplexe geeignet, die in der Reaktionsmischung zumindest teilweise löslich sind. Die Oxidationsstufe des Palladiums kann 0 oder 2 betragen. Bei den Palladiumsalzen kommen u. a. folgende Gegenionen in Betracht: Chlorid, Bromid, Iodid, Sulfat, Acetat, Trifluoracetat, Acetylacetonat oder Hexafluoro-2,4-pentadionat. Es können viele verschiedene Palladiumkomplexe verwendet werden. Voraussetzung ist lediglich, daß die Liganden am Palladium unter den Reaktionsbedingungen vom Substrat verdrängt werden können. Besonders geeignet sind Phosphinliganden wie z. B. Aryl-Alkylphosphine wie u. a. Methyldiphenylphosphin, Isopropyldiphenylphosphin, Triarylphosphine wie u. a. Triphenylphosphin, Tritolylphosphin, Trixylylphosphin, Trihetarylphosphine wie Trifurylphosphin oder dimere Phosphine. Gut geeignet sind auch olefinische Liganden wie u. a. Dibenzylidenaceton oder seine Salze, Cycloocta-1,5-dien oder Amine wie Trialkylamine (z. B. Triethylamin, Tetramethylethylendiamin, N-Methylmorpholin) oder Pyridin.

Man kann den verwendeten Komplex direkt bei der Reaktion einsetzen. So kann man z. B. mit Tetrakistriphenylphosphinpalladium(0), Bistriphenylphosphinpalladiumdichlorid, Bistriphenylphosphinpalladiumdiacetat, einem Dibenzylidenaceton-Palladium(0)-Komplex, Tetrakismethyldiphenylphosphinpalladium(0) oder Bis(1,2-diphenylphosphinoethan)palladiumdichlorid verfahren. Man kann auch ein Palladiumsalz und zusätzlich einen geeigneten Liganden verwenden, die dann erst in situ den katalytisch aktiven Komplex bilden. Diese Vorgehensweise bietet sich z. B. bei den oben genannten Salzen und Phosphinliganden wie z. B. Trifurylphosphin oder Tritolylphosphin an. Auch können Palladiumkomplexe wie z. B. Tris(dibenzylidenaceton)dipalladium, Bis(dibenzylidenaceton)palladium oder 1,5-Cyclooctadienpalladiumdichlorid durch die Zugabe von Liganden wie z. B. Trifurylphosphin oder Tritolylphosphin weiter aktiviert werden.

Üblicherweise werden 0,001 bis 10 mol-%, insbesondere 0,005 bis 5 mol-% der Palladiumverbindung (Salz oder Komplex), bezogen auf die Verbindung X-A⁴ bzw. X-A⁵ verwendet. Höhere Mengen sind möglich, aber eher unwirtschaftlich.

Die Menge an X-A⁴ bzw. X-A⁵, bezogen auf den Ausgangsstoff I-A² (I mit A=A²), liegt im allgemeinen bei 0,8 bis 3, bevorzugt 0,95 bis 1,5 Moläquivalenten.

Für die Reaktion sind alle Lösungsmittel geeignet, die nicht selbst mit den verwendeten Substraten reagieren. Polare Lösungsmittel beschleunigen die Reaktion. Besonders geeignet sind Ether wie Diethylether, Methyl-tert.-butylether, Dimethoxyethan, Tetrahydrofuran, Dioxan, Amide wie Dimethylformamid, Dimethylacetamid, N-Methylpyrrolidon, Dimethylpropylenharnstoff oder Amine wie Triethylamin. Vorteilhaft ist oftmals die Verwendung von Mischungen z. B. von Ethern mit Amiden oder Mischungen der obengenannten Lösungsmittel mit Wasser oder aliphatischen Alkoholen. Die Zugabe von Tetraalkylammoniumhalogeniden oder Alkalimetallhalogeniden wie z. B. Lithiumchlorid ist oft hilfreich und insbesondere anzuraten, wenn A² für Sulfonyloxireste steht.

Wenn X Wasserstoff bedeutet, ist es häufig vorteilhaft, dem Reaktionsgemisch eine anorganische oder organische Base zuzusetzen wie z.B. Kalciumcarbonat, Natriumcarbonat, Natriumphosphat, Kaliumphosphat, Natriumhydroxid, Kaliumhydroxid, Calciumhydroxid, Calciumcarbonat, Pyridin, Triethylamin usw.

Die Reaktionstemperatur liegt zwischen -20 und 200°C, bevorzugterweise zwischen 50 und 160°C. Die Reaktionszeiten betragen üblicherweise zwischen einigen Minuten und 50 Stunden, meist 0,5-10 Stunden. Bei der Verwendung niedrig siedender Lösungsmittel ist es manchmal nützlich, die Umsetzung unter Eigendruck im Autoklaven durchzuführen.

Gegebenenfalls können die so erhaltenen Produkte der Formel I mit A=A⁴ oder A⁵ durch weitere Reaktionen in andere Zwischenprodukte der Formel I mit A=A⁴ oder A⁵ überführt werden. So kann es erforderlich sein, Schutzgruppen abzuspalten. Beispielsweise kann die Verbindung Nr. 1.017 aus Tabelle 1 durch hydrolytische Abspaltung des Trimethylsilylrestes aus Verbindung Nr. 1.001 aus Tabelle 1 in Gegenwart von Tetrabutylammoniumfluorid hergestellt werden.

Eine andere Möglichkeit zur Herstellung der Zwischenprodukte I mit A=A⁴ ergibt sich durch Cycloaddition von cyclischen Acetalen I mit A=A⁵ an ein Dien, Heterodien oder 1,3-Dipol gemäß folgender Reaktionsgleichung:

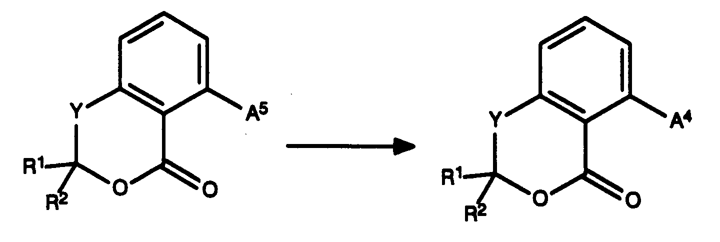

Für A⁵ kommen die voranstehend beschriebenen Alkene und Alkine in Betracht, ganz besonders bevorzugt sind Vinyl und Ethinyl.

Hinsichtlich der (Hetero)Dienkomponente und möglicher 1,3-polarer Verbindungen sei auf G. March, Advanced Organic Chemistry, Second Edition, 1977, pp. 758-798 verwiesen.

Besonders bevorzugte Diene sind Cyclopentadien und 3-Sulfolen. Als 1,3-Dipole sind Nitriloxide besonders geeignet, die durch R³¹ substituiert sind. Auf diese Weise sind verschiedene Isoxazol- und Isoxazolinderivate der Formel I zugänglich (s. Herstellbeispiel Nr. 6).

Die oben beschriebenen Reaktionszeiten, -Temperaturen und Lösungsmittel sind auch hier geeignet, zusätzlich bieten sich unpolare Solventien wie Kohlenwasserstoffe wie Hexane, Heptane o. ä., Aromaten wie Toluol oder Chlorbenzol oder chlorierte Kohlenwasserstoffe an. In manchen Fällen kann es sinnvoll sein als Katalysator eine Protonen- oder Lewis-Säure zuzusetzen.

Die cyclischen Acetale I sind selber herbizid aktiv oder können zur Herstellung von Pflanzenschutzwirkstoffen verwendet werden, z.B. der Formel IV durch die Umsetzung mit einem Salz R⁵⁰-M und einem Heterocyclus III gemäß folgendem Reaktionsschema: 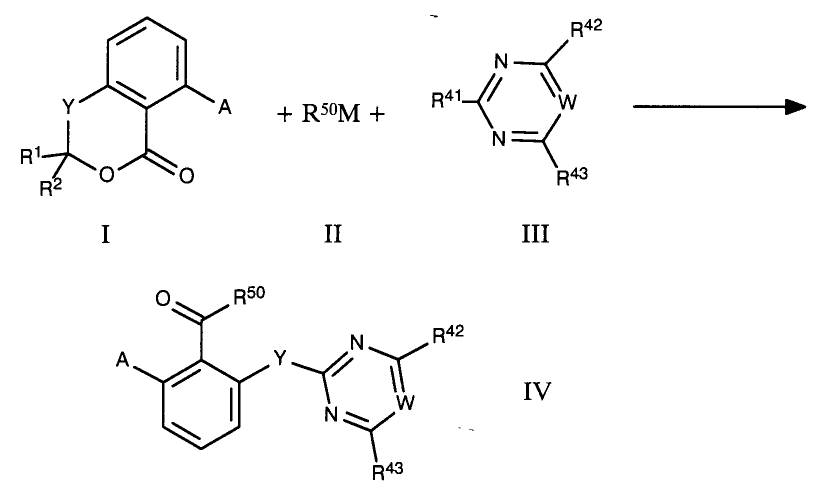 wobei R¹, R², Y und A die oben beschriebenen Bedeutungen haben und die übrigen Reste bedeuten:
M
   ein Alkalimetallkation wie Lithium, Natrium, Kalium oder ein Äquivalent eines Erdalkalimetallkations wie Magnesium, Calcium, Barium;
R⁴¹
   Halogen wie Fluor, Chlor, Brom, Iod, Alkylsulfonyl, besonders Methylsulfonyl oder Halogenalkylsulfonyl, besonders Trifluormethylsulfonyl;
R⁵⁰
   a) ein über ein Stickstoffatom verknüpfter 5-gliedriger Heteroaromat wie Pyrrolyl, Pyrazolyl, Imidazolyl und Triazolyl, welcher ein bis zwei Halogenatome, insbesondere Fluor und Chlor und/oder ein bis zwei der folgenden Reste tragen kann:
      C₁-C₄-Alkyl;
      C₁-C₄-Halogenalkyl, insbesondere C₁-C₂-Halogenalkyl;
      C₁-C₄-Halogenalkoxy, insbesondere C₁-C₂-Halogenalkoxy;
      C₁-C₄-Alkoxy;
      C₁-C₄-Alkylthio;
   b) ein Rest -(O)m-NR⁶R⁷ in dem m für 0 oder 1 steht und R⁶ und R⁷, die gleich oder unterschiedlich sein können, die folgende Bedeutung haben:
      Wasserstoff;
      C₁-C₈-Alkyl, insbesondere C₁-C₄-Alkyl;
      C₃-C₆-Alkenyl, insbesondere 2-Propenyl, 2-Butenyl, 3-Methyl-2-butenyl und 3-Methyl-2-pentenyl; C₃-C₆-Alkinyl, vorzugsweise 2-Propinyl, 2-Butinyl, 1-Methyl-2-propinyl und 1-Methyl-2-butinyl, insbesondere 2-Propinyl;
      C₃-C₈-Cycloalkyl, wie Cyclopropyl, Cyclobutyl, Cyclopentyl, Cyclohexyl, Cycloheptyl und Cyclooctyl, wobei diese Alkyl-, Cycloalkyl-, Alkenyl- und Alkinylgruppen jeweils ein bis fünf, insesondere ein bis drei Halogenatome, bevorzugt Fluor oder Chlor und/oder ein bis zwei der folgenden Gruppen tragen können:
      C₁-C₄-Alkyl, C₁-C₄-Alkoxy, C₁-C₄-Alkylthio, C₁-C₄-Halogenalkoxy, C₃-C₆-Alkenyloxy, C₃-C₆-Alkenylthio, C₃-C₆-Alkinyloxy, C₃-C₆-Alkinylthio, wobei die in diesen Resten vorliegenden Alkenyl- und Alkinylbestandteile vorzugsweise den oben genannten Bedeutungen entsprechen;
      C₁-C₄-Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl;
      C₁-C₄-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl;
      C₃-C₆-Alkenylcarbonyl, C₃-C₆-Alkinylcarbonyl, C₃-C₆-Alkenyloxycarbonyl und C₃-C₆-Alkinyloxycarbonyl, wobei die Alkenyl- bzw. Alkinylreste vorzugsweise wie vorstehend definiert sind;
      Phenyl, gegebenenfalls ein oder mehrfach substituiert durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio wie beispielsweise 2-Fluorphenyl, 3-Chlorphenyl, 4-Bromphenyl, 2-Methylphenyl, 3-Nitrophenyl, 4-Cyanophenyl, 2-Trifluormethylphenyl, 3-Methoxyphenyl, 4-Trifluorethoxyphenyl, 2-Methylthiophenyl, 2,4-Dichlorphenyl, 2-Methoxy-3-methylphenyl, 2,4-Dimethoxyphenyl, 2-Nitro-5-cyanophenyl, 2,6-Difluorphenyl;
      Di-C₁-C₄-alkylamino wie insbesondere Dimethylamino, Diethylamino, Dipropylamino, N-Propyl-N-methylamino, N-Propyl-N-ethylamino, Diisopropylamino, N-Isopropyl-N-methylamino, N-Isopropyl-N-ethylamino, N-Isopropyl-N-propylamino;
      R⁶ und R⁷ ferner Phenyl, das durch einen oder mehrere der folgenden Reste substituiert sein kann: Halogen, Nitro, Cyano, C₁-C₄-Alkyl, C₁-C₄-Halogenalkyl, insbesondere C₁-C₂-Halogenalkyl, C₁-C₄-Alkoxy, C₁-C₄-Halogenalkoxy, insbesondere C₁-C₂-Halogenalkoxy oder C₁-C₄-Alkylthio;
      oder R⁶ und R⁷ bilden gemeinsam eine zu einem Ring geschlossene, gegebenenfalls substituierte C₄-C₇-Alkylenkette, die ein Heteroatum, ausgewählt aus der Gruppe Sauerstoff, Schwefel oder Stickstoff, enthalten kann wie -(CH₂)₄-, -(CH₂)₅-, -(CH₂)₆-, -(CH₂)₇-, -(CH₂)₂-O-(CH₂)₂-, -CH₂-S-(CH₂)₃-, -(CH₂)₂-O-(CH₃)₃-, -NH-(CH₂)₃-, -CH₂-NH-(CH₂)₂-, -CH₂-CH=CH-CH₂-, -CH=CH-(CH₂)₃-, wobei als Substituenten insbesondere C₁-C₄-Alkylreste in Betracht kommen;
   c) ferner eine Gruppe 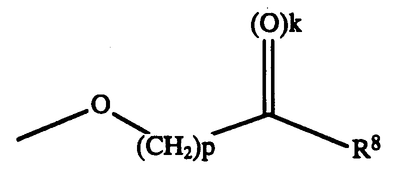 in der k die Werte 0, 1 und 2, p die Werte 1, 2, 3 und 4 annehmen und R⁸ für
      C₁-C₄-Alkyl, C₁-C₄-Halogenalkyl, C₃-C₆-Alkenyl, C₃-C₆-Alkinyl oder gegebenenfalls substituiertes Phenyl steht, wie insbesondere für R⁶ und R⁷ genannt;
   d) ferner ein Rest OR⁹, worin R⁹ bedeutet:
      i) C₃-C₈-Cycloalkyl wie vorstehend genannt, welches ein bis drei C₁-C₄-Alkylgruppen tragen kann, insbesondere Cyclopropyl, Cyclopentyl, Cyclohexyl, Metylcyclohexyl;
      ii) C₁-C₈-Alkyl, welches ein bis fünf Halogenatome, insbesondere Fluor und Chlor und/oder einen der folgenden Reste tragen kann:
         C₁-C₄-Alkoxy, C₁-C₄-Alkylthio, Cyano, C₁-C₄-Alkylcarbonyl, C₃-C₈-Cycloakyl, C₁-C₄-Alkoxycarbonyl, Phenyl, ein- oder mehrfach durch Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio substituiertes Phenyl oder Phenoxy, wie inbesondere oben genannt;
      iii) eine C₁-C₈-Alkylgruppe, welche ein bis fünf, vorzugsweise ein bis drei Halogenatome, insbesondere Fluor und/oder Chlor tragen kann und einen der folgenden Reste trägt: ein 5-gliedriger Heteroaromat, enthaltend ein bis drei Stickstoffatome oder ein 5-gliedriger Heteroaromat, enthaltend ein Stickstoffatom und ein Sauerstoff- oder Schwefelatomt wie Pyrazolyl, Imidazolyl, Benzimidazolyl, Triazolyl, Benztriazolyl, Isooxazolyl, Oxazolyl, Thiazolyl, gebunden über ein C-Atom oder falls möglich N-Atom, wobei der Heteroaromat ein bis vier Halogenatome und/oder ein bis zwei der folgenden Reste tragen kann:
         Nitro, Cyano, C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-l-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3-Isopropylisoxazol-5-yl, 3-Methylisoxazol-5-yl, Oxazol-2-yl, Thiazol-2-yl, Imidazol-2-yl, 3-Ethylisoxazol-5-yl, 3-Phenylisoxazol-5-yl, 3-tert.-Butylisoxazol-5-yl;
      iv) eine C₂-C₆-Alkylgrupe, welche in der 2-Position einen der folgenden Reste trägt: C₁-C₄-Alkoxyimino, C₃-C₆-Alkinyloxyimino, C₃-C₆-Halogenalkenyloxyimino oder Benzyloxyimino;
      v) eine C₃-C₆-Alkenyl- oder eine C₃-C₆-Alkinylgruppe, wobei diese Gruppen ihrerseits ein bis fünf Halogenatome tragen können;
      vi) R⁹ ferner ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wie insbesondere oben genannt;
      vii) ein über ein Stickstoffatom verknüpfter 5-gliedriger Heteroaromat, enthaltend ein bis drei Stickstoffatome wie Pyrazolyl, Imidazolyl, Benzimidazolyl, Triazolyl, Benztriazolyl, vorzugsweise gebunden über die 1-Position, wobei der Heteroaromat ein bis zwei Halogenatome und/oder ein bis zwei der folgenden Reste tragen kann:
         C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3,4-Dichlorimidazol-1-yl;
      viii) R⁹ ferner ein Gruppe -N=CR¹⁰R¹¹, worin R¹⁰ und R¹¹, die gleich oder verschieden sein können, bedeuten:
         C₁-C₁₂-Alkyl, C₃-C₆-Alkenyl, C₃-C₆-Alkinyl, C₃-C₈-Cycloalkyl, wobei diese Reste einen C₁-C₄-Alkoxy, C₁-C₄-Alkylthio und/oder einen gegebenenfalls substituierten Phenylrest, wie insbesondere vorstehend genannt, tragen können;
         Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio;
         oder R¹⁰ und R¹¹ bilden gemeinsam eine C₃-C₁₂ Alkylenkette, welche ein bis drei C₁-C₄-Alkylgruppen tragen und ein Heteroatom aus der Gruppe Sauerstoff, Schwefel und Stickstoff enthalten kann, wie insbesondere bei R⁶ und R⁷ genannt.
   e) oder R⁵⁰ bildet einen Rest -NH-SO₂-R¹², in dem R¹² bedeutet:
      C₁-C₄-Alkyl, C₃-C₆-Alkenyl, C₃-C₆-Alkinyl, C₃-C₈-Cycloalkyl wie insbesondere vorstehend für R¹ genannt, wobei diese Reste einen C₁-C₄-Alkoxy-, C₁-C₄-Alkylthio- und/oder einen Phenylrest wie oben genannt tragen können;
      Phenyl, gegebenenfalls substituiert, insbesondere wie vorstehend genannt;
R⁴² die voranstehend im einzelnen genannten C₁-C₄-Alkyl-, C₁-C₄-Halogenalkyl-, C₁-C₄-Alkoxy-, C₁-C₄-Halogenalkoxy-, C₁-C₄-Alkylthiogruppen und Halogenatome, insbesondere Chlor, Methyl, Methoxy, Ethoxy, Difluormethoxy, Trifluormethoxy, besonders bevorzugt Methoxy;
W Stickstoff oder CR¹³, worin
   - R¹³: bevorzugt Wasserstoff bedeutet oder zusammen mit R⁴³ eine 4-bis 5-gliedrige Alkylen- oder Alkenylenkette bildet, in der jeweils eine Methylengruppe durch Sauerstoff ersetzt ist wie -CH₂-CH₂-O-, -CH=CH-O-, -CH₂-CH₂-CH₂-O-, -CH=CH-CH₂O-, insbesondere Wasserstoff und -CH₂-CH₂-O-;
   - R⁴³: Halogen, C₁-C₄-Alkyl, C₁-C₄-Halogenalkyl, besonders C₁-C₂-Halogenalkyl, C₁-C₄-Alkoxy, C₁-C₄-Halogenalkoxy, besonders C₁-C₂-Halogenalkoxy, C₁-C₄-Alkylthiogruppen, insbesondere Chlor, Fluor, Methyl, Methoxy, Ethoxy, Difluormethoxy, Trifluormethoxy, besonders bevorzugt Methoxy, oder mit R¹³ ist wie oben genannt zu einem 5- oder 6-gliedrigen Ring verknüpft.

Die Umsetzung der cyclischen Acetale I mit dem Salz R⁵⁰M und die weitere Reaktion mit dem Heterocyclus der Formel III kann in einem Reaktionsgefäß direkt bis zum Wirkstoff IV durchgeführt werden. In diesem Fall gibt man zuerst das Salz R⁵⁰M zu, das man auch in situ aus einer Verbindung R⁵⁰H und einer Base herstellen kann. Anschließend fügt man dann den Heterocyclus III hinzu. Die Zugabe von III sollte vorteilhaft erst erfolgen, wenn der erste Schritt der Reaktion, die Addition des Salzes R⁵⁰M an das Zwischenprodukt I, weitgehend abgeschlossen ist. Das kann zwischen wenigen Minuten und etlichen Stunden dauern, wobei die Reaktionstemperatur zwischen -40°C und 200°C, meist zwischen 0°C und 130°C liegt.

Es ist auch möglich, die Reaktion nach der ersten Stufe abzubrechen und das Zwischenprodukt V 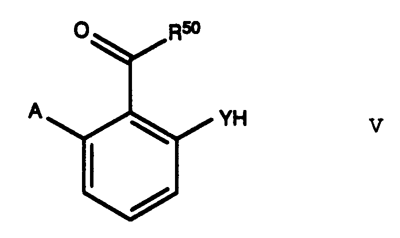 zu isolieren. Die Verbindung V kann man dann nach bekannten Verfahren, z.B. wie in den eingangs genannten Offenlegungsschriften beschrieben, weiter umsetzen.

In beiden Fällen sind die üblichen Lösungsmittel geeignet, vorausgesetzt, sie können von der verwendeten Base oder dem Salz R⁵⁰M nicht selbst deprotoniert werden und an der Reaktion teilnehmen. Besonders gut geeignet sind polare Lösungsmittel, z. B. Ether wie Diethylether, Methyl-tert.-butylether, Dimethoxyethan, Tetrahydrofuran, Dioxan, Amide wie Dimethylformamid, Dimethylacetamid, N-Methylpyrrolidon, Dimethylpropylenharnstoff oder Dimethylsulfoxid. Man kann einen Phasentransfer-Katalysator wie einen Kronenether oder ein quartäres Ammoniumsalz zusetzen, wenn das den Umsatz fördert.

Die Reaktionstemperatur liegt zwischen -40 und 200°C, bevorzugterweise zwischen 0 und 160°C. Die Reaktionszeiten betragen üblicherweise zwischen einigen Minuten und 50 Stunden, meist 0,5-10 Stunden.

Pro Mol Ausgangsstoff I werden im allgemeinen 0,8 bis 3, insbesondere 0,9 bis 1,5 Moläquivalente der Verbindung II (R⁵⁰M) eingesetzt. Die Menge an Heterocyclus III liegt ebenfalls zweckmäßigerweise bei 0,8 bis 3, insbesondere 0,9 bis 1,5 Moläquivalenten, bezogen auf I.

Die Umsetzung kann kontinuierlich oder diskontinuierlich; bei Atmosphärendruck, Über- oder Unterdruck vorgenommen werden.

Die Möglichkeiten der Aufarbeitung sind vielfältig und hängen im Einzelfall von der Löslichkeit des Produktes in den verwendeten Lösungsmitteln sowie von der Mischbarkeit der Lösungsmittel mit Wasser und den Siedepunkten der Lösungsmittel ab. Sowohl wäßrige als auch nicht-wäßrige Aufarbeitungen sind möglich.

Eine geeignete Aufarbeitungsmethode besteht beispielsweise darin, die Reaktionsmischung, aus der man vorher das Lösungsmittel auch teilweise oder ganz verdampfen kann, mit Wasser zu vermischen und das Produkt aufzufiltrieren oder mit einem organischen Lösungsmittel zu extrahieren.

### Synthesebeispiele

### Beispiel 1

### a)

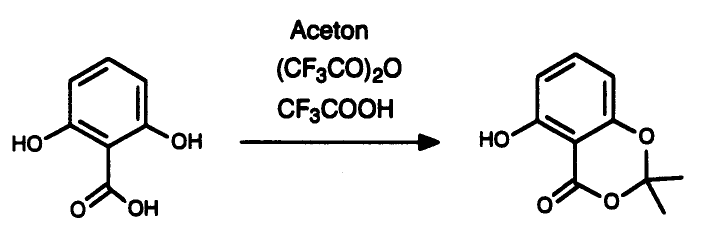

5-Hydroxy-2,2-dimethyl-4H-(1,3)benzodioxin-4-on

Man legt 100 g (0,66 Mol) 2,6-Dihydroxybenzoesäure in 800 ml Trifluoressigsäure vor und gibt 100 ml Aceton sowie 216 g (1,98 Mol) Trifluoracetanhydrid zu. Man kocht 2 h unter Rückfluß, und tropft anschließend während weiterer 5,5 h unter Rückfluß 50 ml Aceton pro Stunde zu (gesamte Reaktionszeit 7,5 h, gesamte Acetonzugabe 375 ml). Die Reaktionsmischung wird im Vakuum bei ca. 55°C eingeengt, dreimal mit Toluol aufgefüllt und wieder eingeengt und schließlich 1 h bei 45°C an der Ölpumpe getrocknet.

Das ölige Produkt wird in 2 l Methyl-tert.-butylether aufgenommen, mit 2 l Wasser und 2 l gesättigter NaHCO₃-Lösung versetzt und ca. 1,5 h gerührt. Man trennt die wäßrige Phase ab, extrahiert einmal mit Methyl-tert.-butylether, wäscht die vereinigten organischen Phasen mit Kochsalzlösung, trocknet und engt im Vakuum ein. Der Rückstand wird mit 200 ml n-Pentan verrührt, abgesaugt und im Vakuum getrocknet.
Ausbeute: 115 g (90%)
Schmp. 60-62°C.

### b)

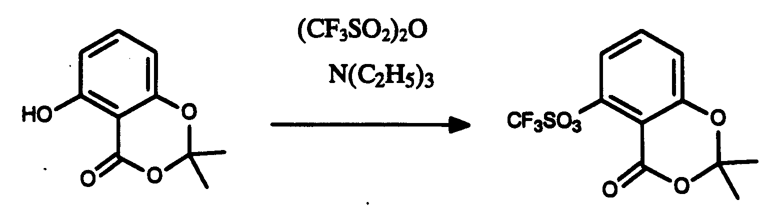

2,2-Dimethyl-5-trifluormethylsulfonyloxy-4H-(1,3)benzodioxin-4-on

Man löst 80 g (0,41 Mol) 5-Hydroxy-2,2-dimethyl-4H-(1, 3)benzodioxin-4-on in 1,5 l Methylenchlorid, gibt bei 0°C 129 g (1,28 Mol) Triethylamin zu und tropft dann innerhalb von 2 h 314 g (1,11 Mol) Trifluormethylsulfonsäureanhydrid zu. Man läßt auf 10°C erwärmen, rührt bei dieser Temperatur 10 min nach und gibt die Mischung dann unter Rühren in 1,5 l Wasser bei 0°C. Man trennt die organische Phase ab, extrahiert mit Methylenchlorid, wäscht die vereinigten organischen Phasen mit Wasser und gesättigter Kochsalzlösung, trocknet über Natriumsulfat und engt im Vakuum ein. Der Rückstand wird mit 200 ml n-Pentan verrührt, abfiltriert, mit n-Pentan nachgewaschen und bei 40°C an der Ölpumpe getrocknet.
Ausbeute: 118 g (88 %)
Schmelzpunkt: 115°C.

### c)

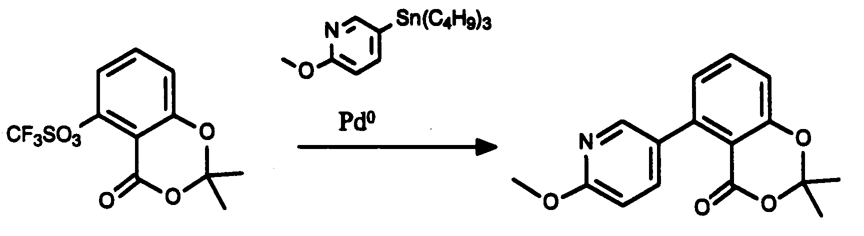

2,2-Dimethyl-5-(2-methoxypyridin-5-yl)-4H-(1,3)benzodioxin-4-on

Man löst 8,7 g 2,2-Dimethyl-5-trifluormethylsulfonyloxy-4H-(1, 3)benzodioxin-4-on, 23,2 g 2-Methoxy-5-tributylstannylpyridin, 3,4 g Lithiumchlorid, 0,6 g Tetrakistriphenylphosphinpalladium⁰ und 70 mg 2,6-Di-t.-butyl-4-methylphenol in 150 ml Dioxan und rührt 3 h bei 140°C im Autoklaven. Man engt im Vakuum ein, verrührt mit 200 ml n-Pentan, filtriert über wenig Kieselgel-60, wäscht mit n-Pentan nach und eluiert das Produkt mit Essigsäureethylester. Nach dem Einengen verbleiben 10,3 g vom Schmp. 160°C.

### d) 2-(4,6-Dimethoxypyrimidin-2-yloxy)-6-(2-methoxypyridin-5-yl)benzoesäureacetonoximester

0,67 g Acetonoxim gelöst in 30 ml Toluol werden mit 1,66 g einer 30 %igen Natriummethylat-Lösung in Methanol versetzt und im Vakuum eingeengt. Man gebt 35 ml Dimethylformamid und dann 2,5 g 2,2-Dimethyl-5-(2-methoxypyridin-5-yl)-4H-(1,3)benzodioxin-4-on zu, rührt 15 min bei Raumtemperatur und fügt schließlich 1,9 g 4,6-Dimethoxy-2-methylsulfonylpyrimidin hinzu. Man rührt über Nacht nach, gießt in 300 ml Wasser, extrahiert mit Methyl-t.-butylether, wäscht gründlich mit Wasser und gesättigter Kochsalzlösung, trocknet über Natriumsulfat und engt im Vakuum ein. Das Rohprodukt wird durch Chromatographie an Kieselgel-60 mit n-Hexan/Essigsäureethylester gereinigt.
¹H-NMR (CDCl₃): δ = 1,65 (s); 1,95 (s); 3,85 (s); 3,98 (s) ; 5,78 (s); 6,77 (d); 7,3-7,7 (m); 8,25 (d).

### Beispiel 2

### a) 2,2-Dimethyl-5-(6-methoxypyridin-2-yl)-4H-(1,3)benzodioxin-4-on

Man löst 10,9 g 2,2-Dimethyl-5-trifluormethylsulfonyloxy-4H-(1, 3)benzodioxin-4-on, 16,0 g 6-Methoxy-2-tributylstannylpyridin, 4,25 g Lithiumchlorid, 0,78 g Tetrakistriphenylphosphinpalladium⁰ und 40 mg 2,6-Di-t.-butyl-4-methylphenol in 120 ml Dioxan und rührt 4 h bei 140°C im Autoklaven. Man engt im Vakuum ein und chromatographiert das Produkt an Kieselgel-60 mit Essigsäureethylester/Toluol. Man erhält 9,1 g (95 %) eines Feststoffes vom Schmp. 130-132°C.

### b) 2-(4,6-Dimethoxypyrimidin-2-yloxy)-6-(6-methoxypyridin-2-yl)benzoesäurebenzylester

1,36 g Benzylalkohol gelöst in 50 ml Dimethylformamid werden mit 380 mg Natriumhydrid (80 %ig in Paraffinöl) versetzt und 1 h bei Raumtemperatur nachgerührt. Man gibt 3,0 g 2,2-Dimethyl-5-(6-methoxypyridin-2-yl)-4H-(1, 3)benzodioxin-4-on zu, rührt 3 h bei Raumtemperatur und fügt schließlich 2,65 g 4,6-Dimethoxy-2-methylsulfonylpyrimidin hinzu. Man rührt 2 h bei Raumtemperatur, 1,5 h bei 80°C und 3 h bei 115°C, gießt in phosphorsaures Eiswasser, extrahiert mit Essigsäureethylester, wäscht mit Wasser, trocknet über Natriumsulfat und engt im Vakuum ein. Das Rohprodukt wird durch Chromatographie an Kieselgel-60 mit Cyclohexan/Toluol/Essigsäureethylester gereinigt.
¹H-NMR (CDCl₃): δ = 3,80 (s); 3,89 (s); 5,02 (s); 5,72 (s); 6,67 (d); 6,9-7,7 (m).

### Beispiel 3

### a) 2,2-Dimethyl-5-phenyl-4H-(1,3)benzodioxin-4-on

Zur Lösung von 2,93 g (9 mmol) 2,2-Dimethyl-5-trifluormethylsulfonyloxy-4H-(1,3)benzodioxin-4-on in 50ml Dimethylformamid gibt man unter Stickstoff 0,23 g (0,2 mmol) Tetrakistriphenylphosphin-Palladium, rührt einige Minuten und fügt dann 2,97g (14 mmol) Kaliumphosphat und 2,04 g (9,9 mmol) Phenylboronsäurediisopropylester hinzu. Man erhitzt auf 100°C bis zum vollständigen Umsatz (ca. 16h). Nach dem Abkühlen gießt man die Reaktionslösung in eine Mischung aus 250 ml Wasser und 6 g Orthophosphorsäure, extrahiert dreimal mit Methyl-tert.-Butylether, wäscht die vereinigten organischen Phasen mi Wasser und gesättigter Kochsalzlösung, trocknet über Natriumsulfat und engt ein. Es verbleiben 2,6 g eines dunklen Öls, welches an Kieselgel mit Toluol/Aceton chromatographiert wird. Man erhält 1,3 g (57 %) eines farblosen Feststoffs, Fp. 158-163°C (aus Hexan umkristallisiert).

### b)

Die unter a) beschriebene Verbindung läßt sich analog auch unter Verwendung von 1,21 g (9 mmol) Phenylboronsäure anstatt des oben verwendeten Boronsäureesters herstellen. Ausbeute 1,7g (74%), physikalische Daten und Reinigung wie unter a).

### Beispiel 4

### 2,2-Dimethyl-5-(3,4-(ethylendioxy)-1-phenyl)-4H-(1,3)benzodioxin-4-on

Zur Lösung von 4,24 g (13,6 mmol) 2,2-Dimethyl-5-trifluormethylsulfonyloxy-4H-(1,3)benzodioxin-4-on in 50 ml Dimethylformamid gibt man unter Stickstoff 0,35 g (0,3 mmol) Tetrakistriphenylphosphin-Palladium, rührt einige Minuten und fügt dann 4,24 g (20 mmol) Kaliumphosphat und 2,70 g (15 mmol) 3,4-(Ethylendioxy)-1-phenylboronsäure hinzu. Man erhitzt auf 100°C bis zum vollständigen Umsatz (ca. 16h). Nach dem Abkühlen gießt man die Reaktionslösung in eine Mischung aus 250 ml Wasser und 8 g Orthophosphorsäure, extrahiert dreimal mit Methyl-tert.-Butylether, wäscht die vereinigten organischen Phasen mit Wasser und gesättigter Kochsalzlösung, trocknet über Natriumsulfat und engt ein. Es verbleiben 7,6 g eines dunklen Öls, welches an Kieselgel mit Toluol/Aceton chromatographiert wird. Man erhält ein farbloses Öl, NMR siehe Tabelle Nr. 1.040.

### Beispiel 5

### 2,2-Dimethyl-5-(3-methyl-3-buten-1-inyl)-4H-(1, 3)benzodioxin-4-on

Zur Lösung von 15,0 g (46 mmol) 2,2-Dimethyl-5-trifluormethylsulfonyloxy-4H-(1,3)benzodioxin-4-on in 150 ml Dimethylformamid gibt man unter Stickstoff 0,64 g (0,92 mmol) Bistriphenylphosphin-Palladiumdichlorid, 4,4 ml (46 mmol) 3-Methyl-3-buten-1-in und 24 ml Triethylamin. Man erhitzt 0,5 h auf 115°C, gibt dann bei dieser Temperatur innerhalb von 1 h nochmals 6,6 ml 3-Methyl-3-buten-1-in zu und rührt 15 min nach. Nach dem Abkühlen gießt man die Reaktionslösung in 500 ml phosphorsaures (pH 3) Wasser bei 0°C, extrahiert mit Methyl-tert.-Butylether, wäscht die vereinigten organischen Phasen sorgfältig mit Wasser und gesättigter Kochsalzlösung, trocknet über Natriumsulfat und engt ein. Es verbleiben 14 g eines dunklen Öls, welches an Kieselgel mit n-Hexan/Ethylacetat chromatographiert wird. Man erhält 7,0 g farblose Kristalle, Fp. 60°C (vgl. Tabelle 1, Nr. 1.009).

### Beispiel 6

### 2,2-Dimethyl-5-(3-methylisoxazol-5-yl)-4H-(1,3)benzodioxin-4-on

Man bereitet zunächst durch Chlorierung von Acetaldoxim mit elementarem Chlor eine Lösung von 1-Chlor-1-(N-hydroxyimino)ethan, die einen Gehalt von ca. 0,18 Mol in 210 ml Methyl-tert.-butylether hat.

Zu einer Lösung von 8,0 g 2,2-Dimethyl-5-(ethinyl)-4H-(1,3)benzodioxin-4-on in 400 ml Methyl-tert.-butylether gibt man unter Rühren bei 0°C 53 ml der oben beschriebenen Lösung von 1-Chlor-1-(N-hydroxyimino)ethan und 6,3 ml Triethylamin, läßt auf Raumtemperatur kommen und rührt über Nacht nach. Man gibt nochmals 53 ml der oben beschriebenen Lösung von 1-Chlor-1-(N-hydroxyimino)ethan und 6,3 ml Triethylamin zu und rührt 24 h bei Raumtemperatur. Anschließend gießt man die Reaktionslösung in phosphorsaures (pH 4) Wasser bei 0°C, extrahiert mit Methyl-tert.-Butylether, wäscht die vereinigten organischen Phasen sorgfältig mit Wasser, trocknet über Natriumsulfat und engt ein. Es verbleiben 8,8 g Rohprodukt, welches an Kieselgel mit n-Hexan/Ethylacetat chromatographiert wird. Man erhält 4,1 g farblose Kristalle, Fp. 74°C (vgl. Tabelle 1, Nr. 1.020).

### Beispiel 7

### a) 5-Chlor-2,2-dimethyl-4H-[3,1]benzoxathiin-4-on

20 g (106 mmol) 2-Chlor-6-mercaptobenzoesäure, 26 ml (212 mmol) 2,2-Dimethoxypropan und 2 g Amberlyst 15 (stark saures Ionenaustauscherharz) werden in 1 l Toluol 3 h unter Rückfluß gekocht. Man destilliert nun 100 ml innerhalb von 2 h ab, fügt noch 10 ml 2,2-Dimethoxypropan zu, kocht eine kurze Zeit weiter, rührt über Nacht bei Raumtemperatur, filtriert und engt im Vakuum ein. Ausbeute: 22,4 g (92 %) Öl, ¹H-NMR (CDCl₃) δ = 1,82 (s); 7,18-7,40 (m).

### b) 5-Chlor-2-phenyl-4H-[3,1]benzoxathiin-4-on

ist unter Verwendung von Benzaldehyddimethylacetal analog Beispiel 7a herstellbar (Fp. 97-103°C).

### Beispiel 8

### 2,2-Dimethyl-5-(pyridin-2-yl)-4H-(1, 3)benzodioxin-4-on

Man löst 6,0 g (18,4 mmol) 2,2-Dimethyl-5-trifluormethylsulfonyloxy-4H-(1,3)benzodioxin-4-on, 7,0 g (19,0 mmol) 2-Tributylstannylpyridin 2,3 g (55,2 mmol) Lithiumchlorid und 420 mg (0,36 mmol) Tetrakistriphenylphosphinpalladium⁰ in 60 ml Dioxan und 90 ml Dimethylformamid und kocht 1,5 h unter Rückfluß (Reaktionstemperatur ca. 120°C). Man rührt bei 0°C in 1,4 l Wasser ein, extrahiert mit Methyl-tert.-butylether, wäscht mit Wasser und Kochsalzlösung, trocknet über Natriumsulfat und engt im Vakuum ein. Man nimmt in 60 ml Essigsäureethylester auf, gibt 8 g Kaliumfluorid zu und rührt über Nacht. Es wird abfiltriert, mit reichlich Essigsäureethylester nachgewaschen und das Filtrat im Vakuum eingeengt. Das Rohprodukt wird durch Chromatographie an Kieselgel-60 mit Hexan/Essigsäureethylester gereinigt. Ausbeute: 2,39 g (51 %). Schmelzpunkt: 142°C.

### Beispiel 9

### 2-(4,6-Dimethoxypyrimidin-2-yloxy)-6-(ethinyl)-benzoesäure(2,2,2-trifluorethyl)ester

Man legt 2,0 g 2,2,2-Trifluorethanol in 80 ml Dimethylformamid vor und gibt bei Raumtemperatur 0,60 g 80%iges Natriumhydrid zu. Nach 1 h hat sich eine klare Lösung gebildet und man gibt 5,0 g 2,2-Dimethyl-5-(trimethylsilylethinyl)-4H-(1, 3)benzodioxin-4-on bei Raumtemperatur zu. Nach 20 min gibt man 4,0 g 4,6-Dimethoxy-2-methylsulfonylpyrimidin zu und rührt über Nacht bei Raumtemperatur. Anschließend gießt man die Reaktionslösung in 400 ml phosphorsaures (pH 3) Wasser bei 0°C, extrahiert mit Methyl-tert.-Butylether, wäscht die vereinigten organischen Phasen mit Wasser, trocknet über Natriumsulfat und engt ein. Es verbleiben 6,4 g Rohprodukt, welches an Kieselgel mit n-Hexan/Ethylacetat chromatographiert wird. Man erhält ein farbloses Öl (1,2 g). ¹H-NMR (CDCl₃): δ = 3,33 (s); 3,83 (s); 4,55 (q); 5,80 (s); 7,25-7,50 (m).

### Beispiel 10

### 2,2-Dimethyl-5-(2-chlorpyridin-4-yl)-4H-(1,3)benzodioxin-4-on

### a) 2,2-Dimethyl-5-(1-oxopyridin-4-yl)-4H-(1,3)benzodioxin-4-on

Man legt 300 mg 2,2-Dimethyl-5-(pyridin-4-yl)-4H-[1,3]benzodioxin-4-on in 20 ml Methylenchlorid vor, gibt bei Raumtemperatur 260 mg 3-Chlorperbenzoesäure zu und rührt über Nacht. Die Reaktionsmischung wird direkt durch Chromatographie an Kieselgel-60 mit Methylenchlorid/Methanol aufgearbeitet. Ausbeute 200 mg. ¹H-NMR (CDCl₃): δ: 1,80 (s); 6,95 (d); 7,07 (d); 7,25 (d); 7,60 (t); 8,25 (d).

### b) 2,2-Dimethyl-5-(2-chlorpyridin-4-yl)-4H-(1,3)benzodioxin-4-on

Man legt 850 mg 2,2-Dimethyl-5-(1-oxopyridin-4-yl)-4H-(1,3)benzodioxin-4-on in 20 ml POCl₃ vor und rührt 4 h bei 105°C. Man entfernt die flüchtigen Anteile im Vakuum, gießt in eine Mischung von 50 ml Eiswasser und 30 ml Methyl-tert.-butylether, stellt mit Natriumhydrogencarbonat-Lösung auf einen pH-Wert von 7,5 ein und extrahiert mit Methyl-tert.-butylether. Der Extrakt wird mit Kochsalz-Lösung gewaschen, über Natriumsulfat getrocknet und im Vakuum eingeengt. Ausbeute: 490 mg. ¹H-NMR (CDCl₃): δ: 1,80 (s); 6,95 (d); 7,07 (d); 7,16 (d); 7,27 (s); 7,59 (t); 8,40 (d).

Die in Tabelle 1 aufgeführten Verbindungen I können beispielsweise analog zu den jeweils angegebenen Synthesebeispielen hergestellt werden.

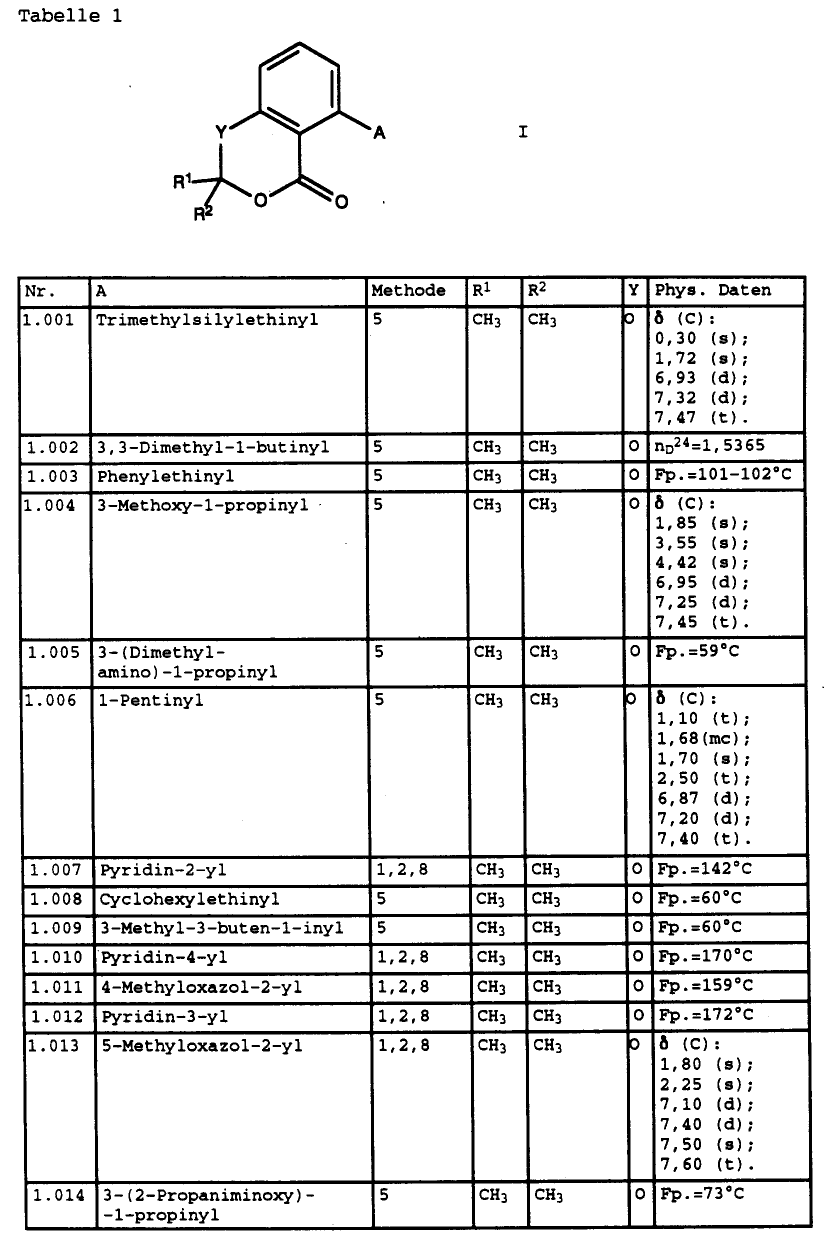

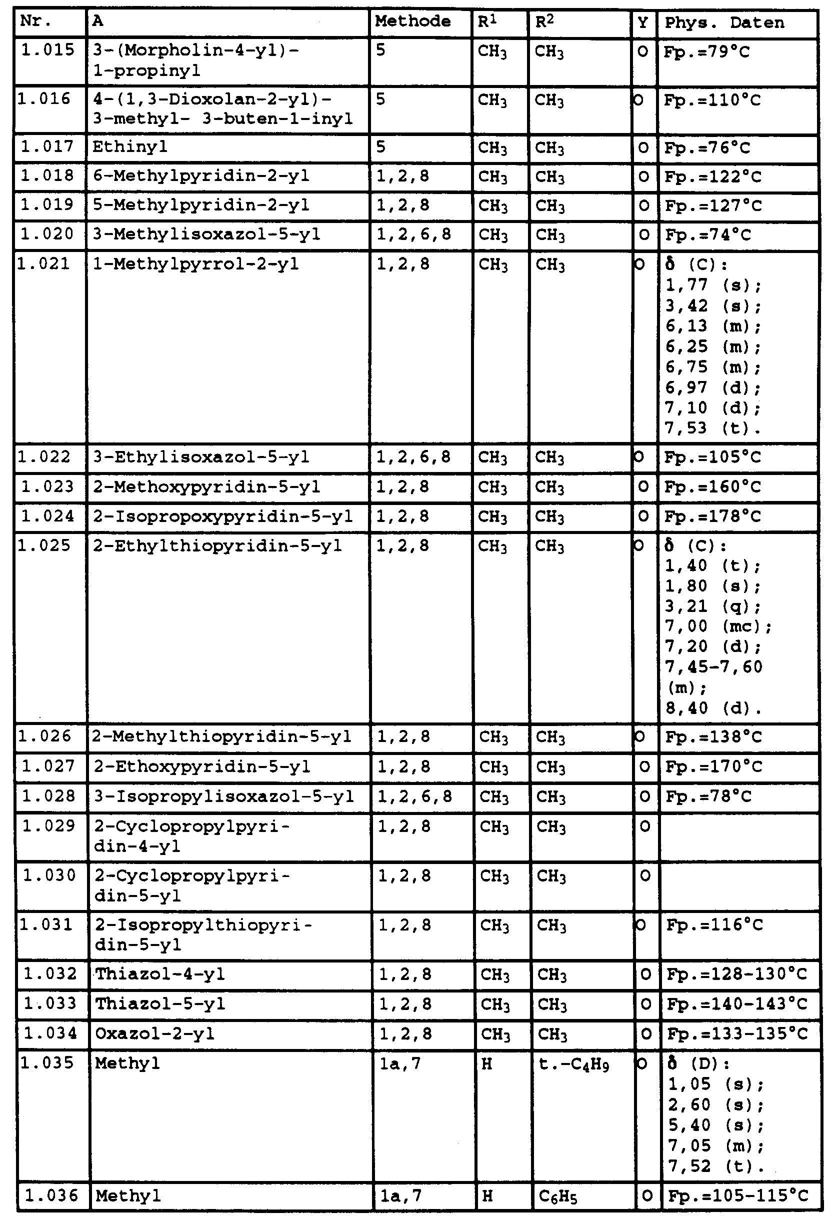

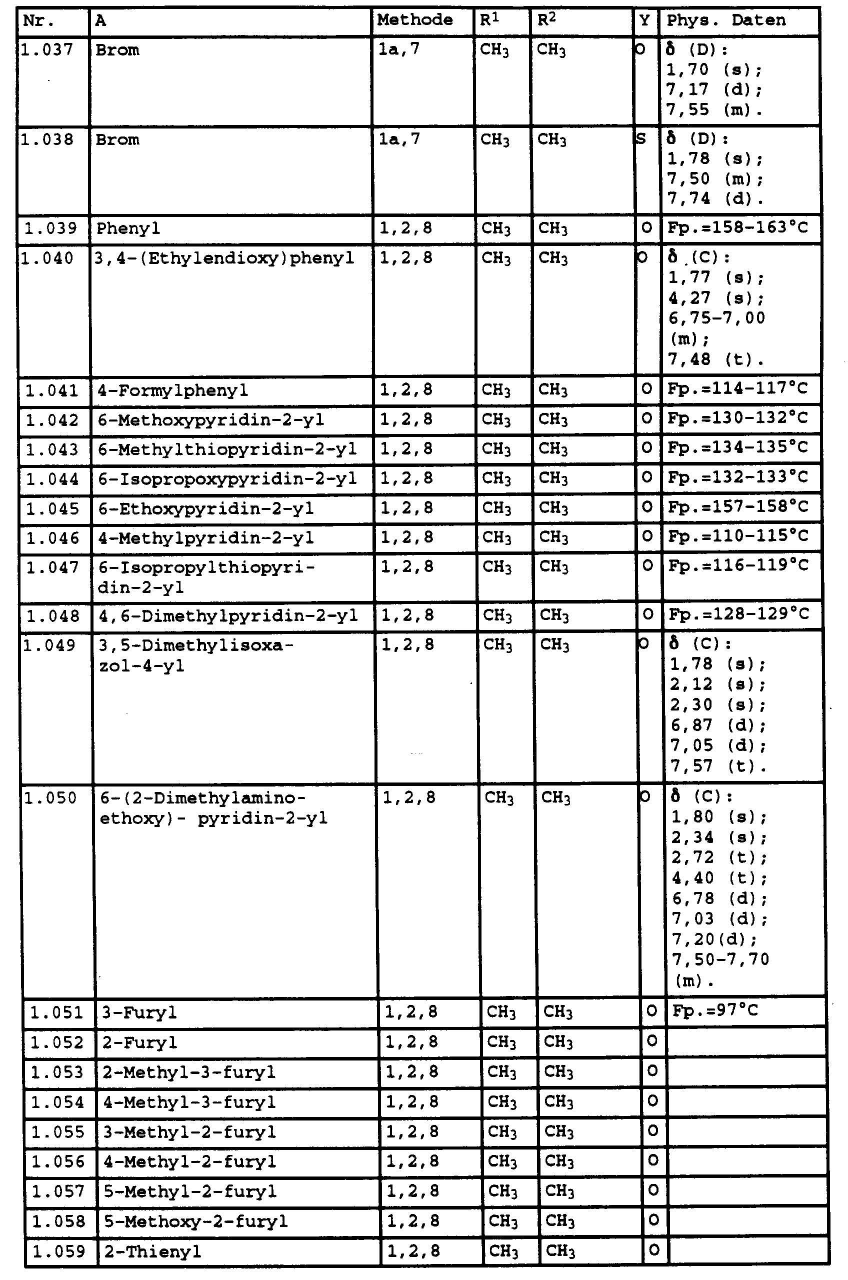

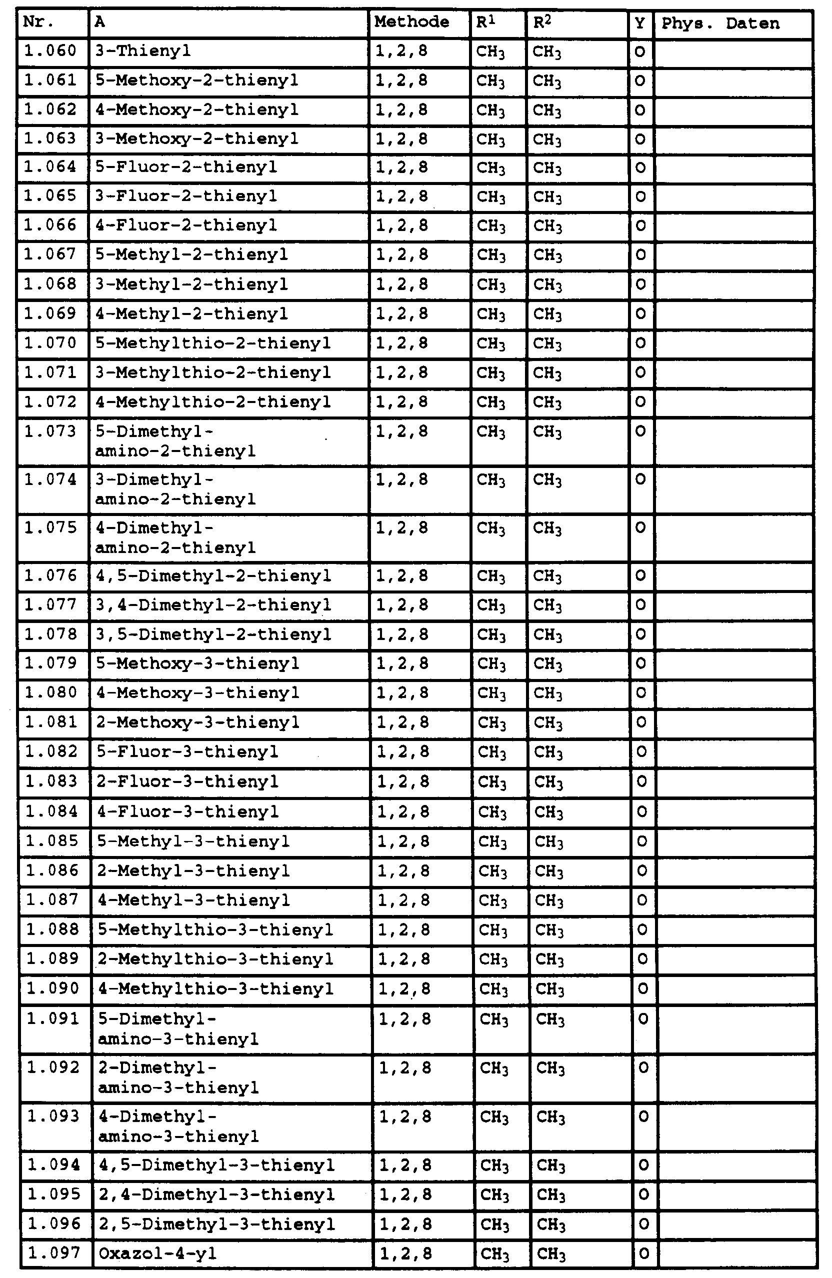

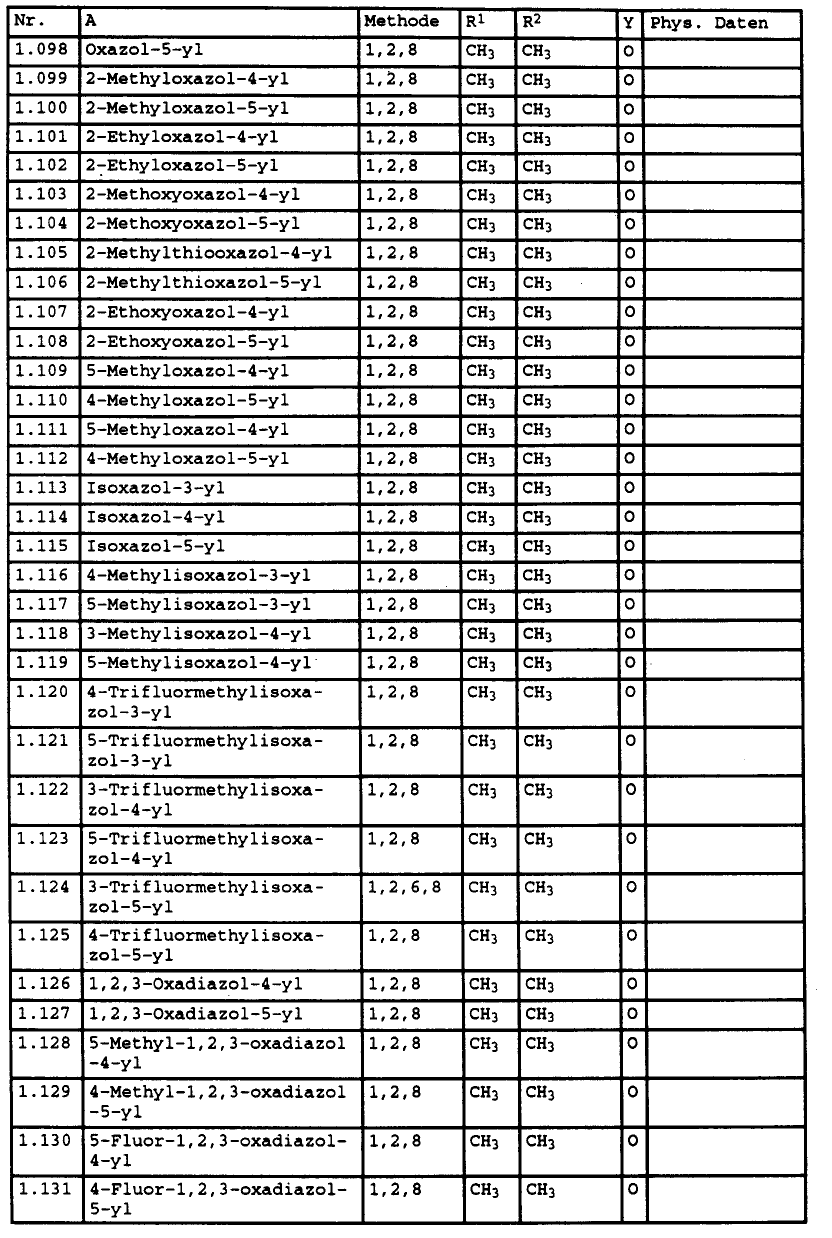

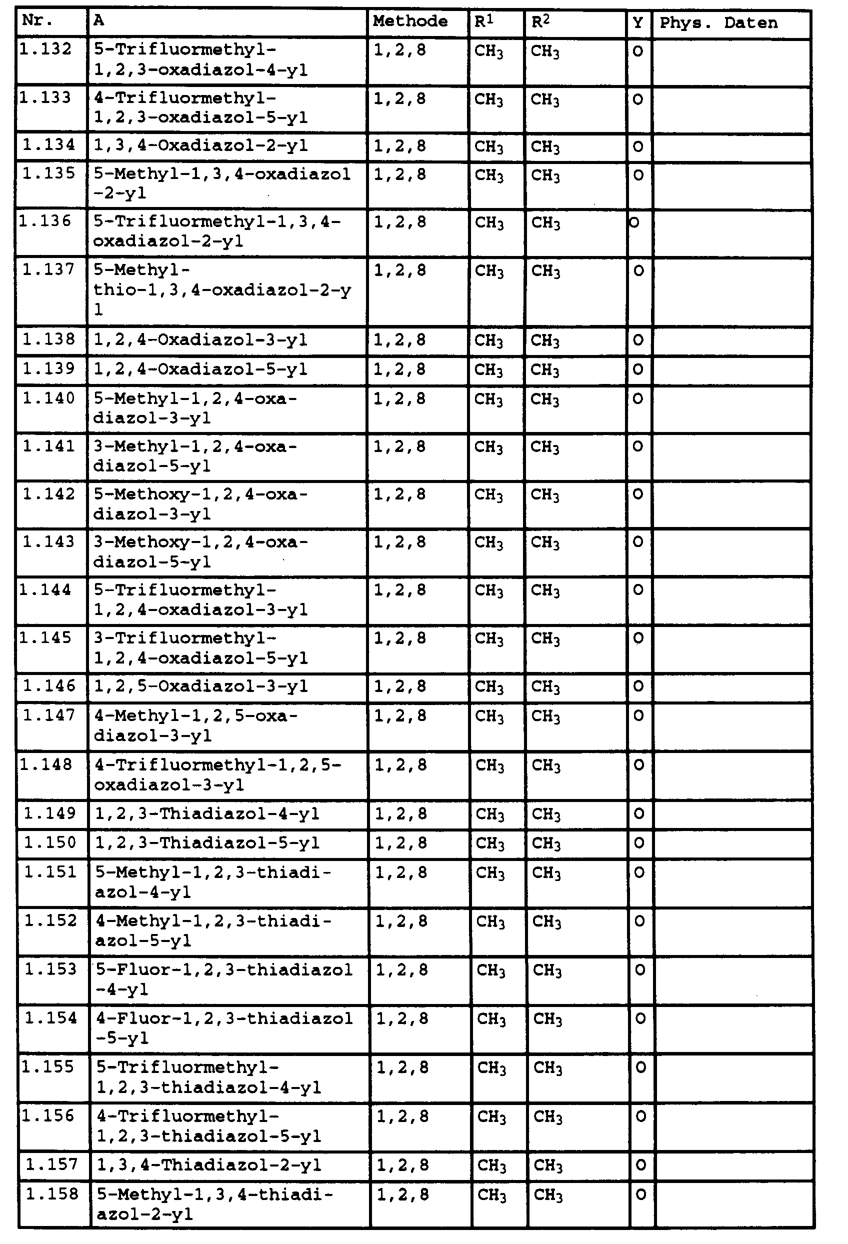

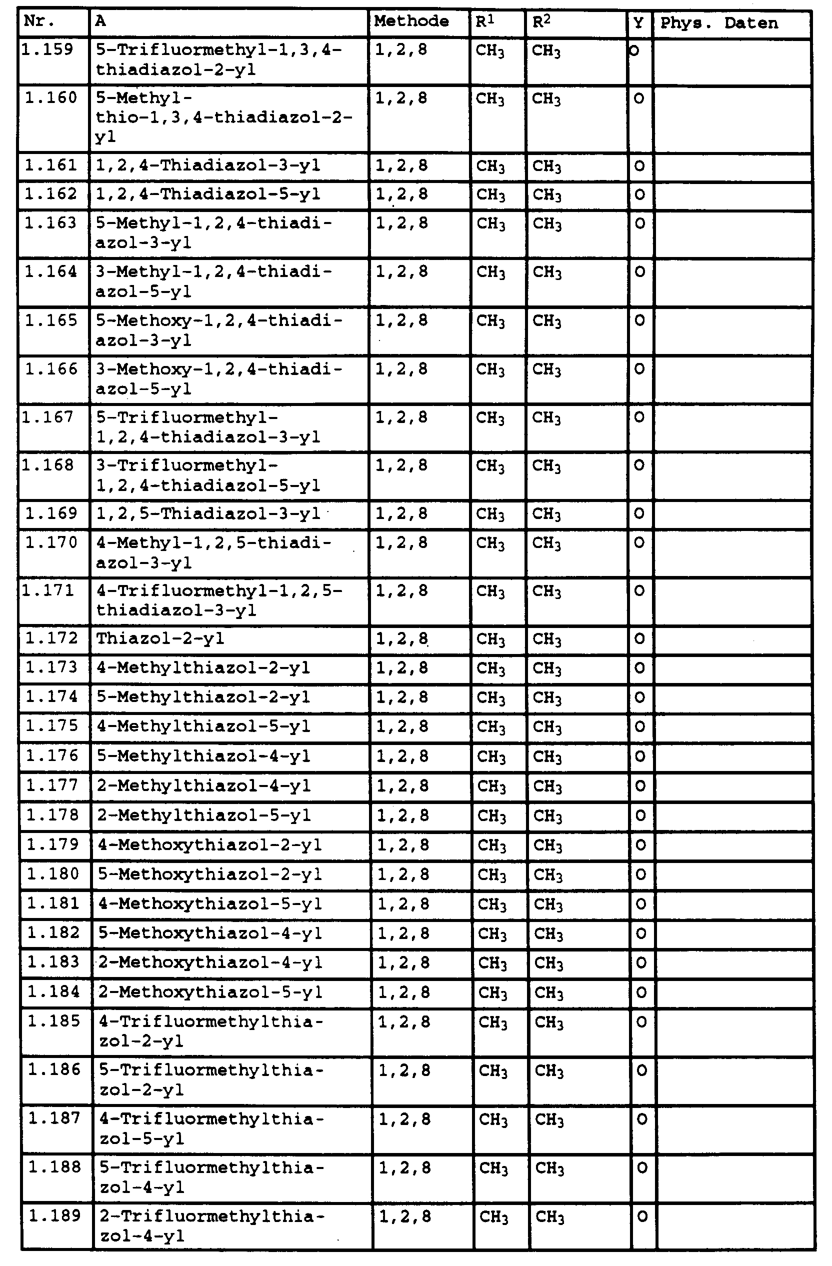

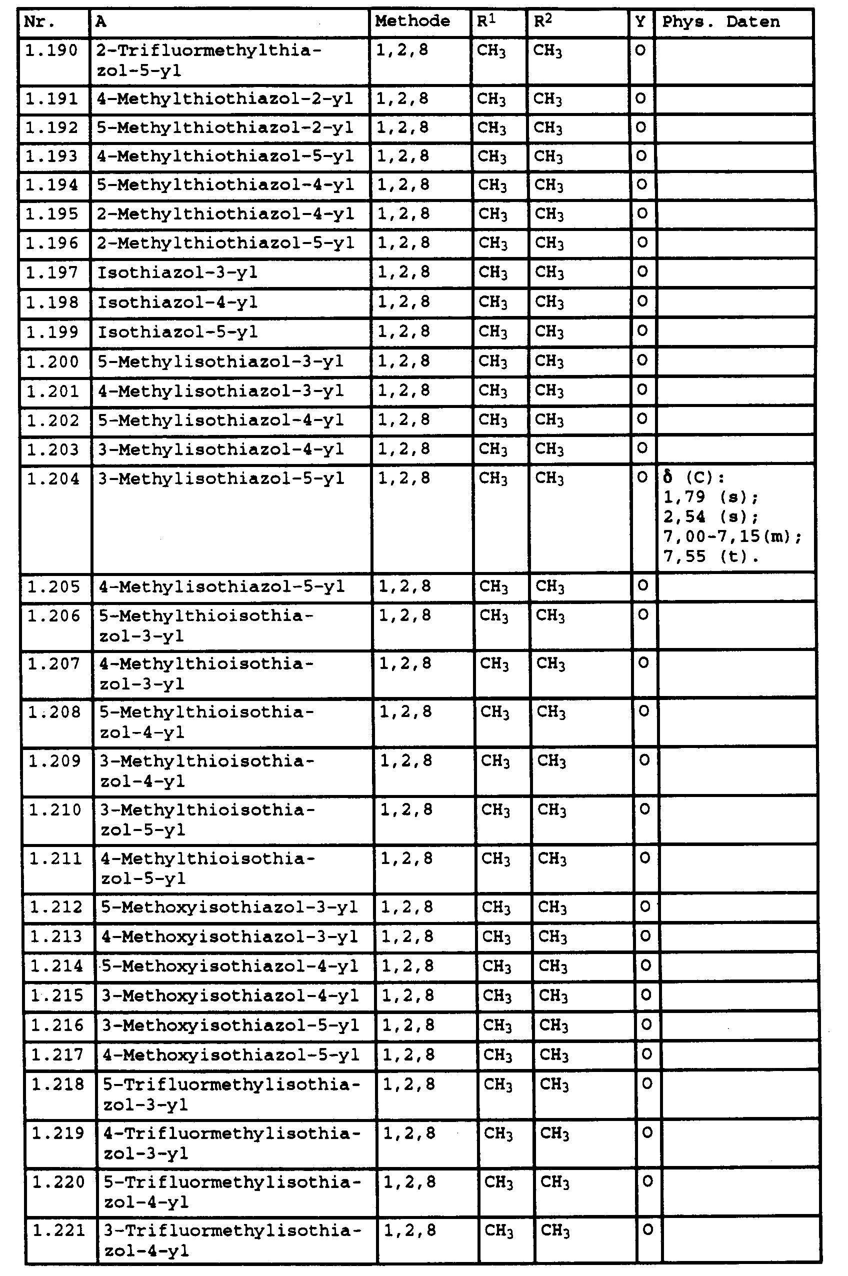

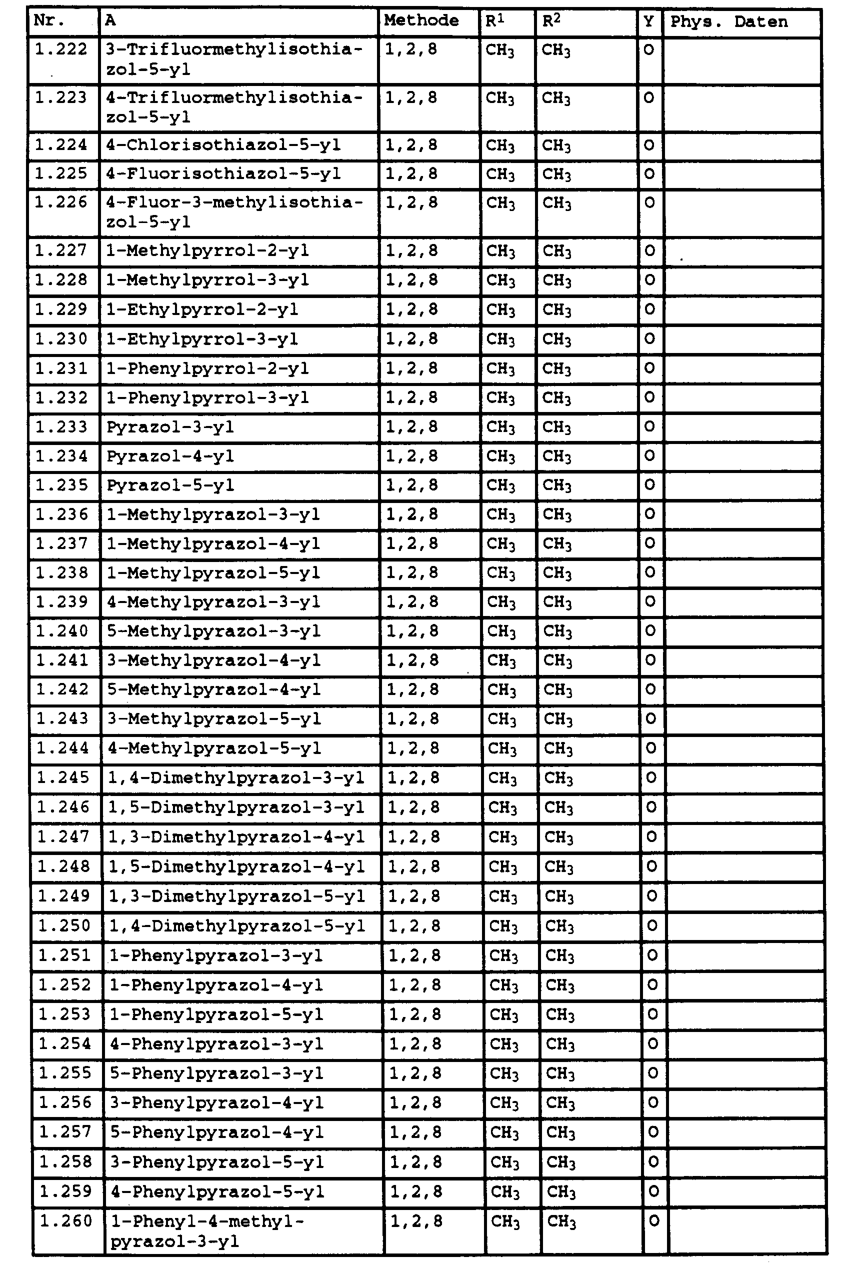

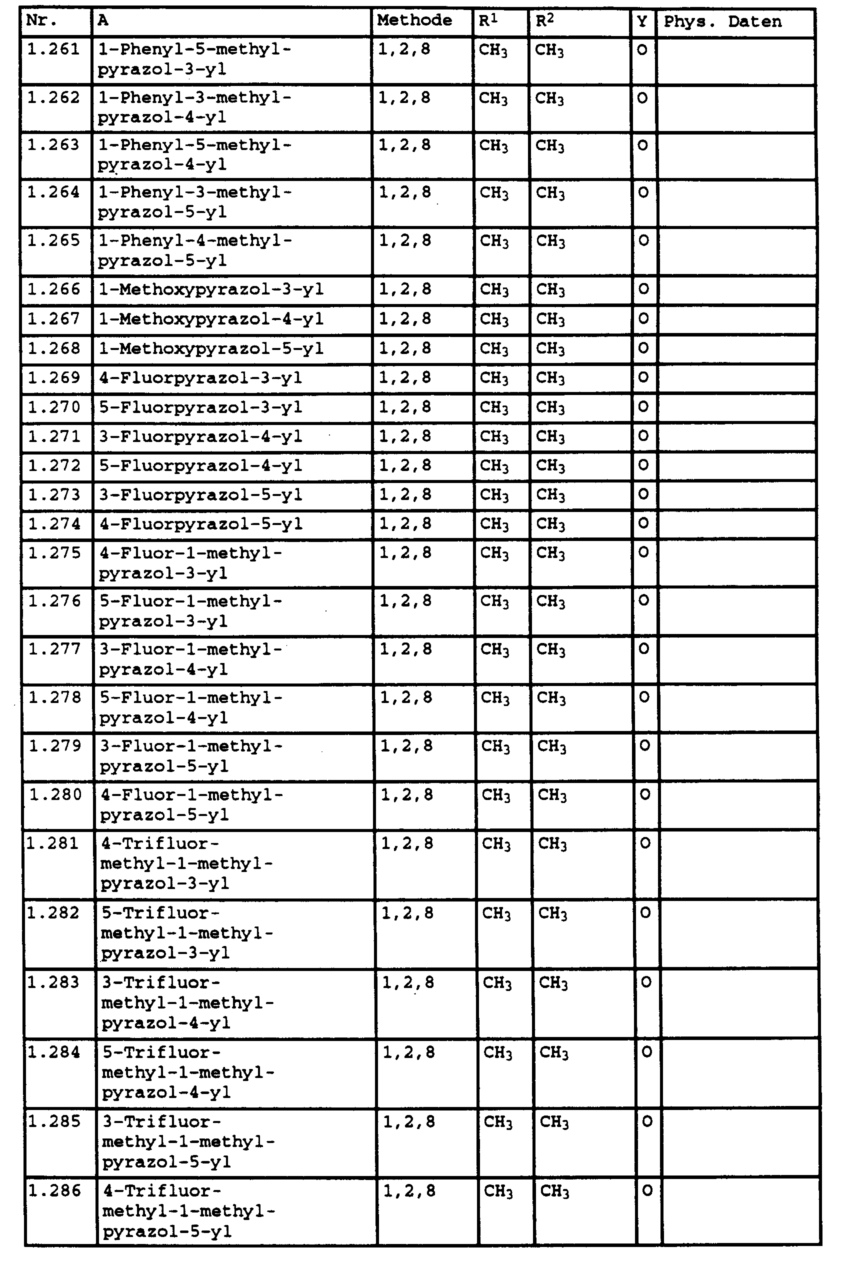

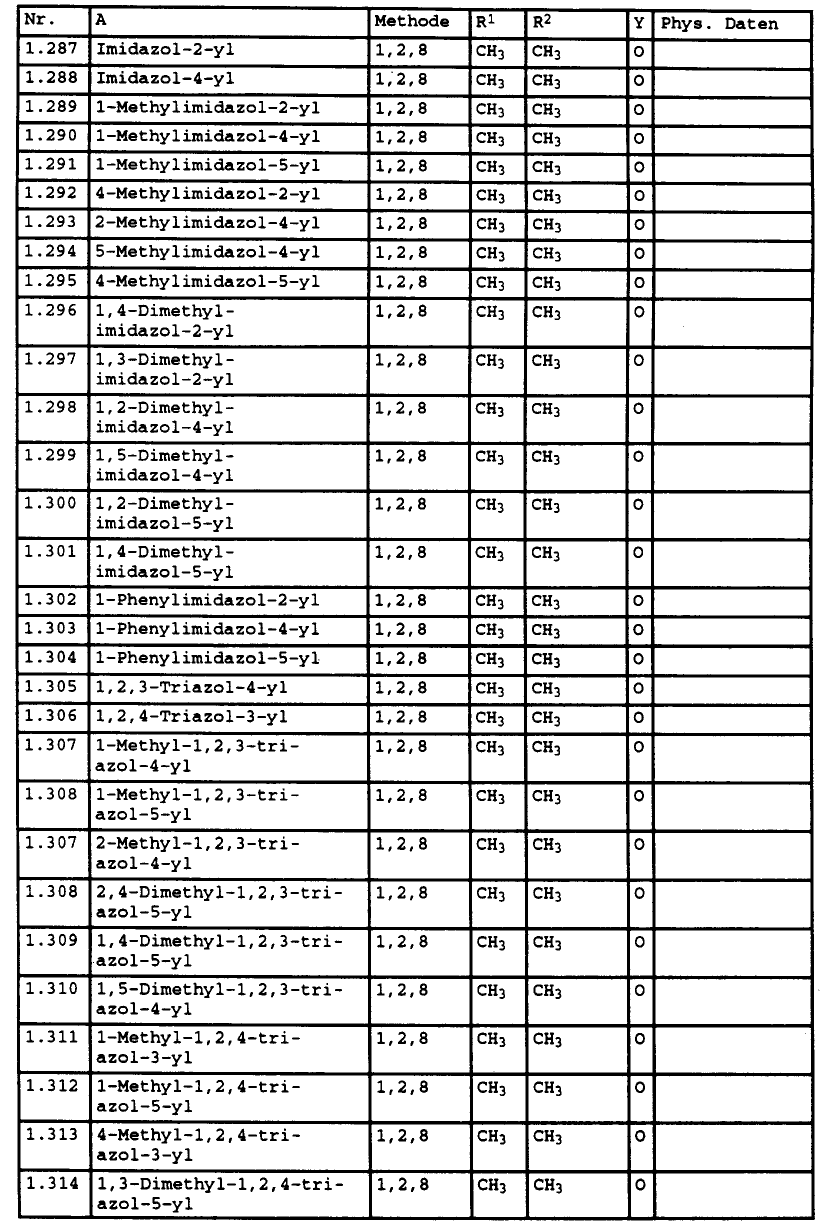

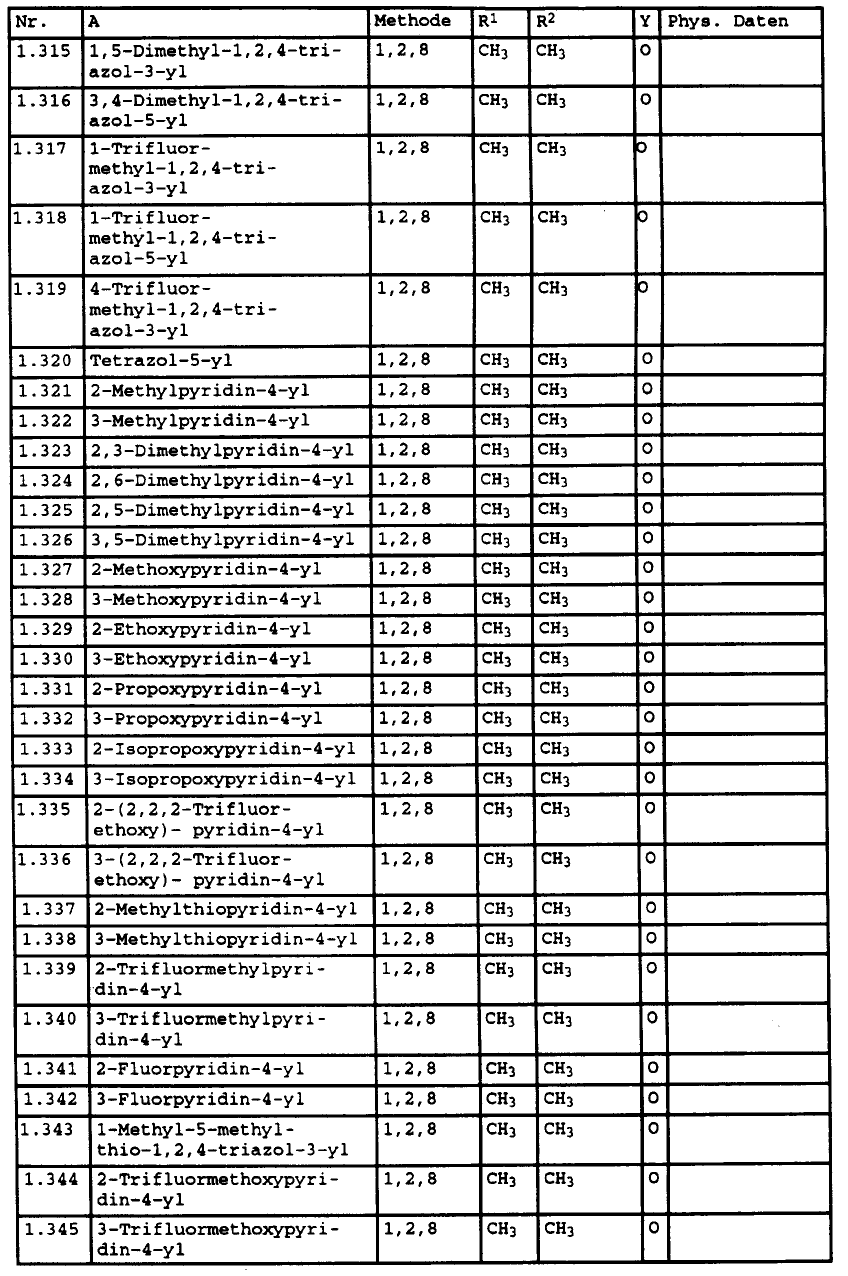

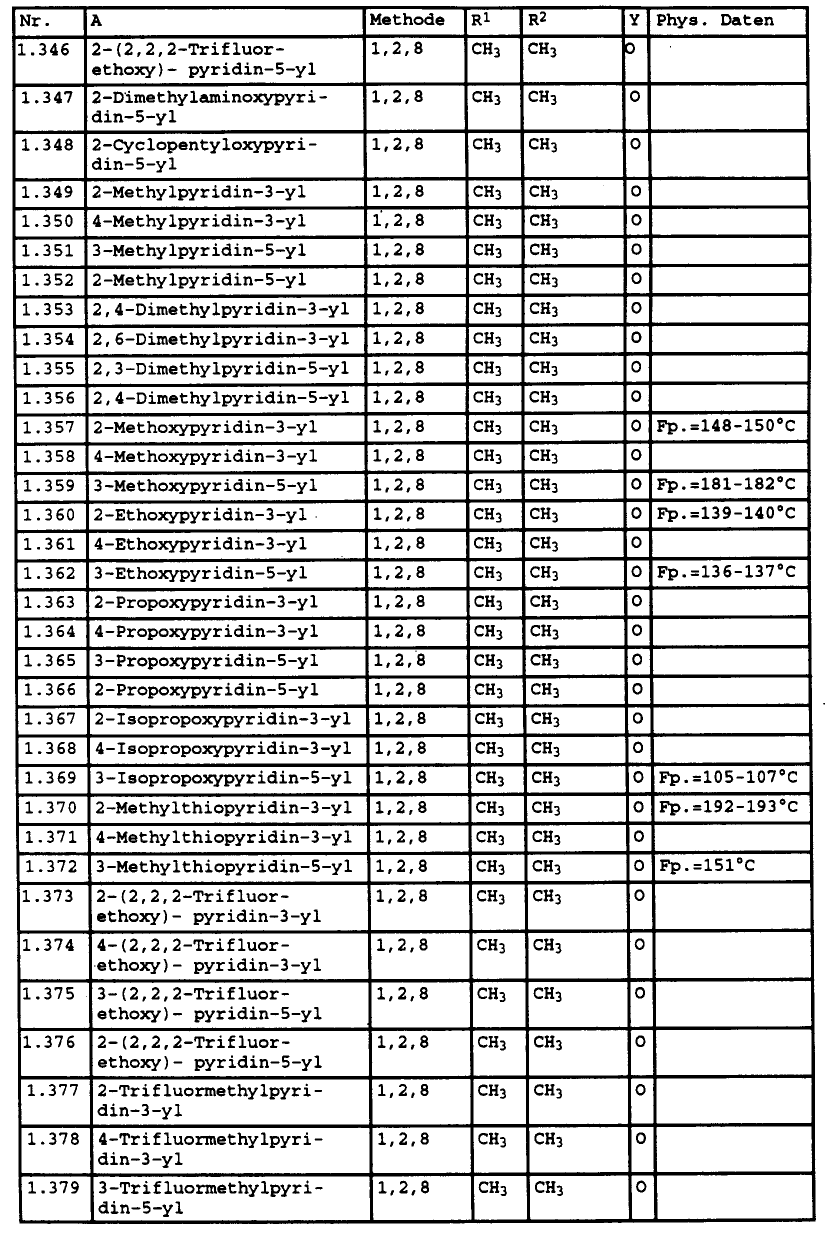

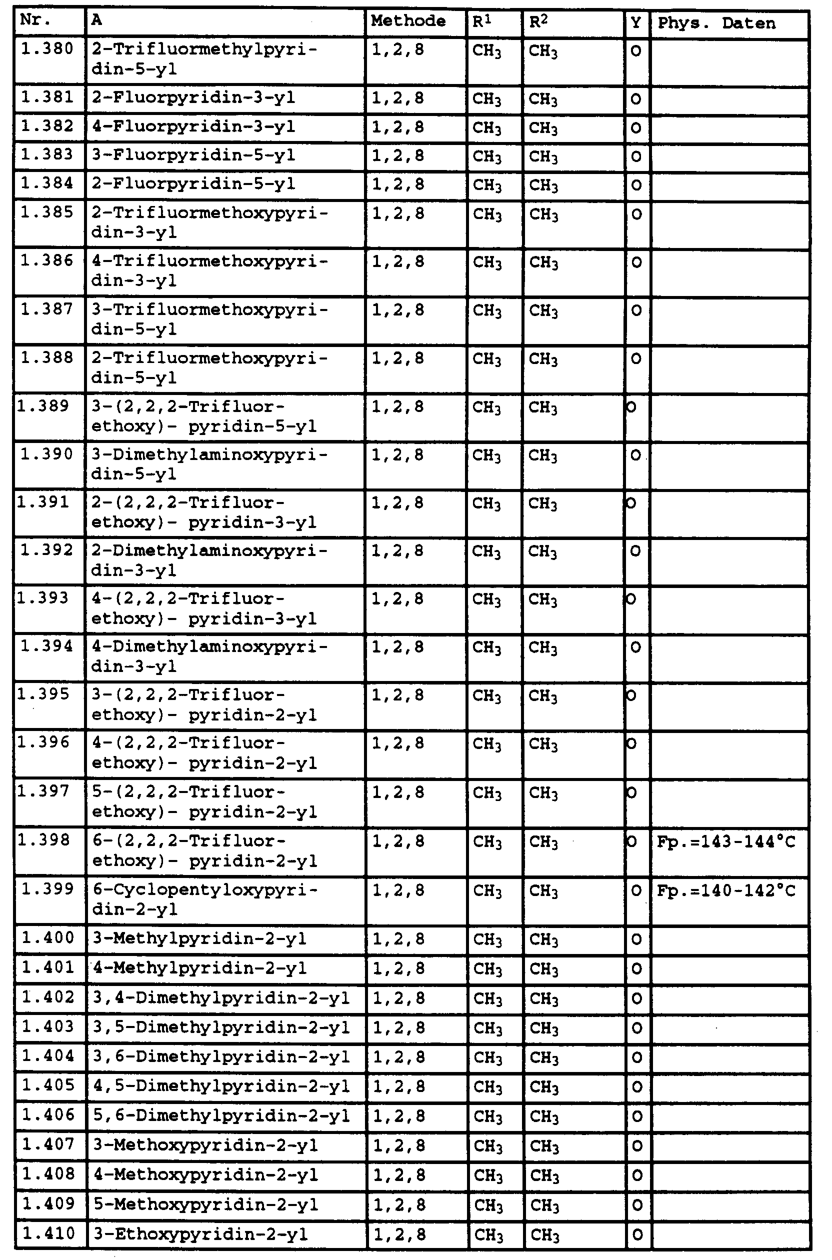

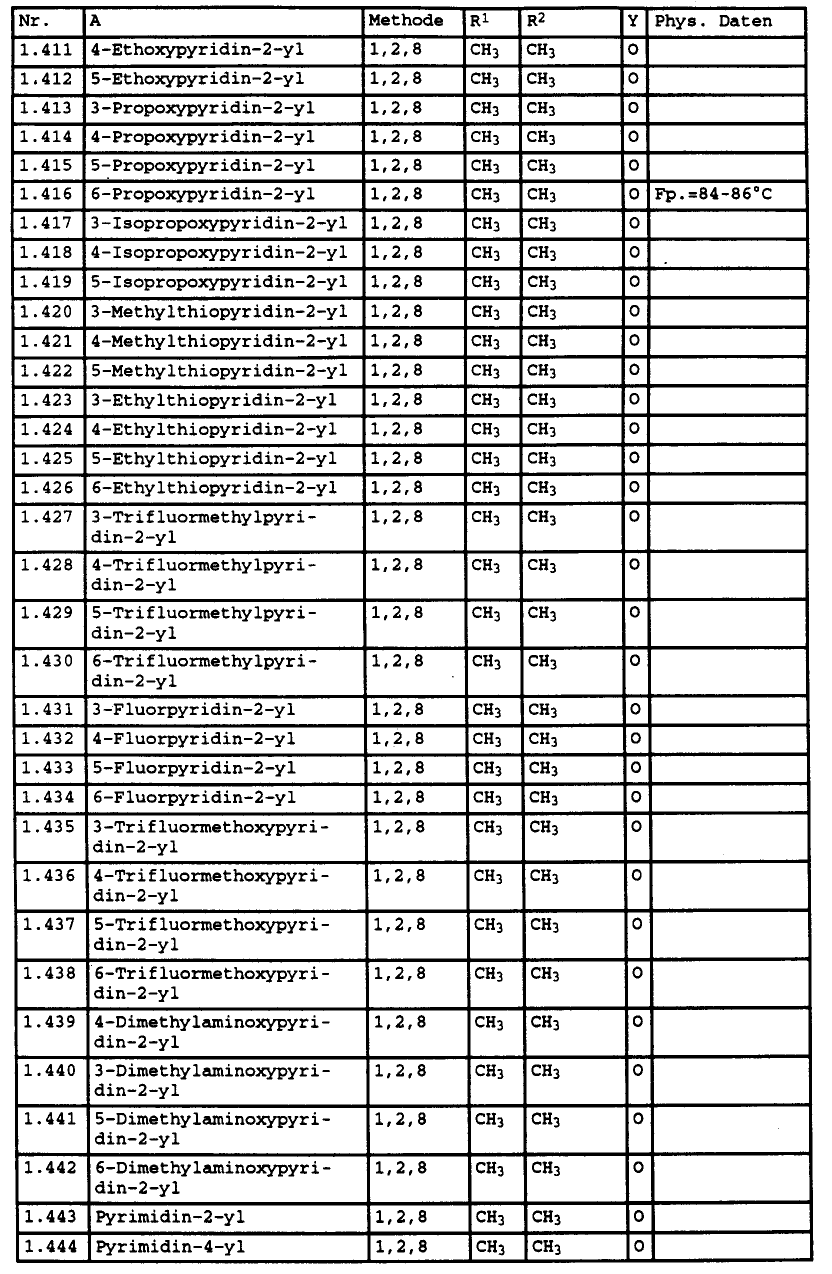

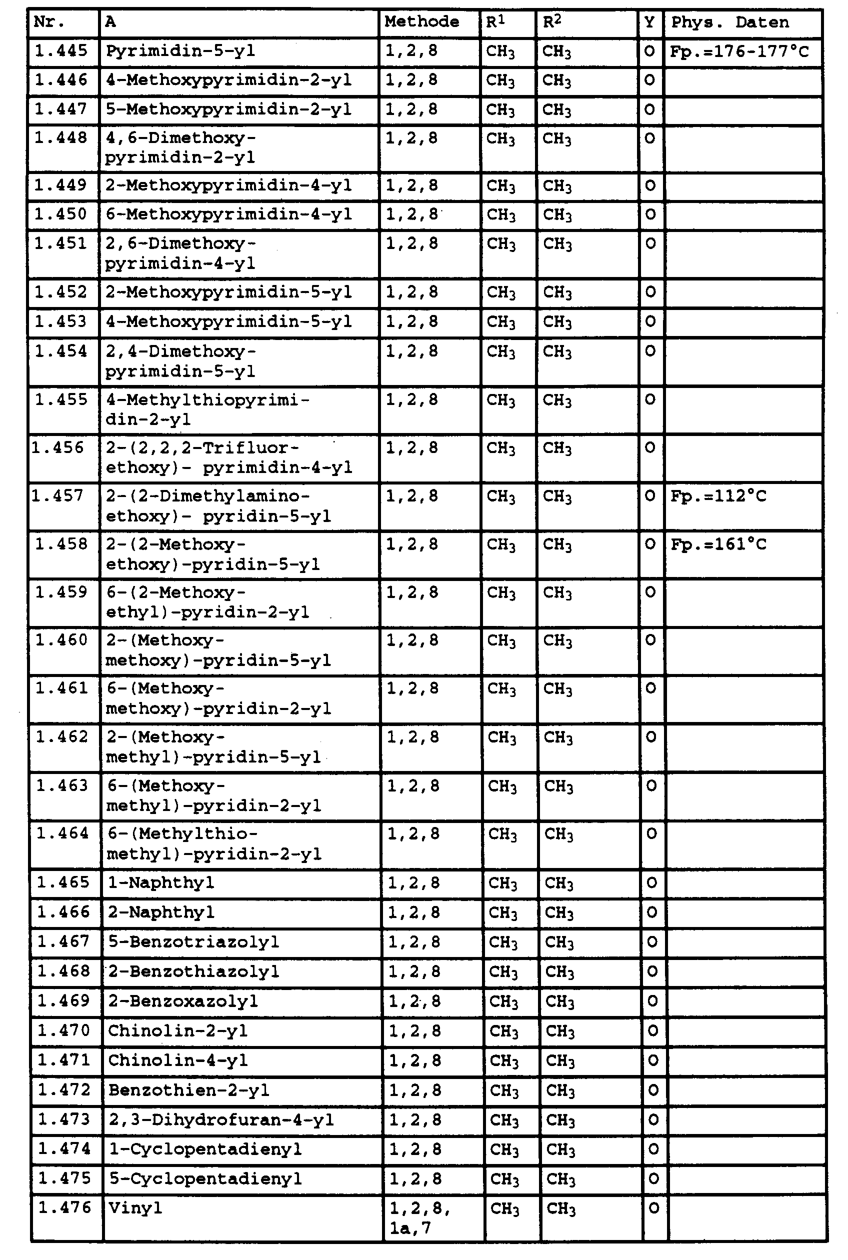

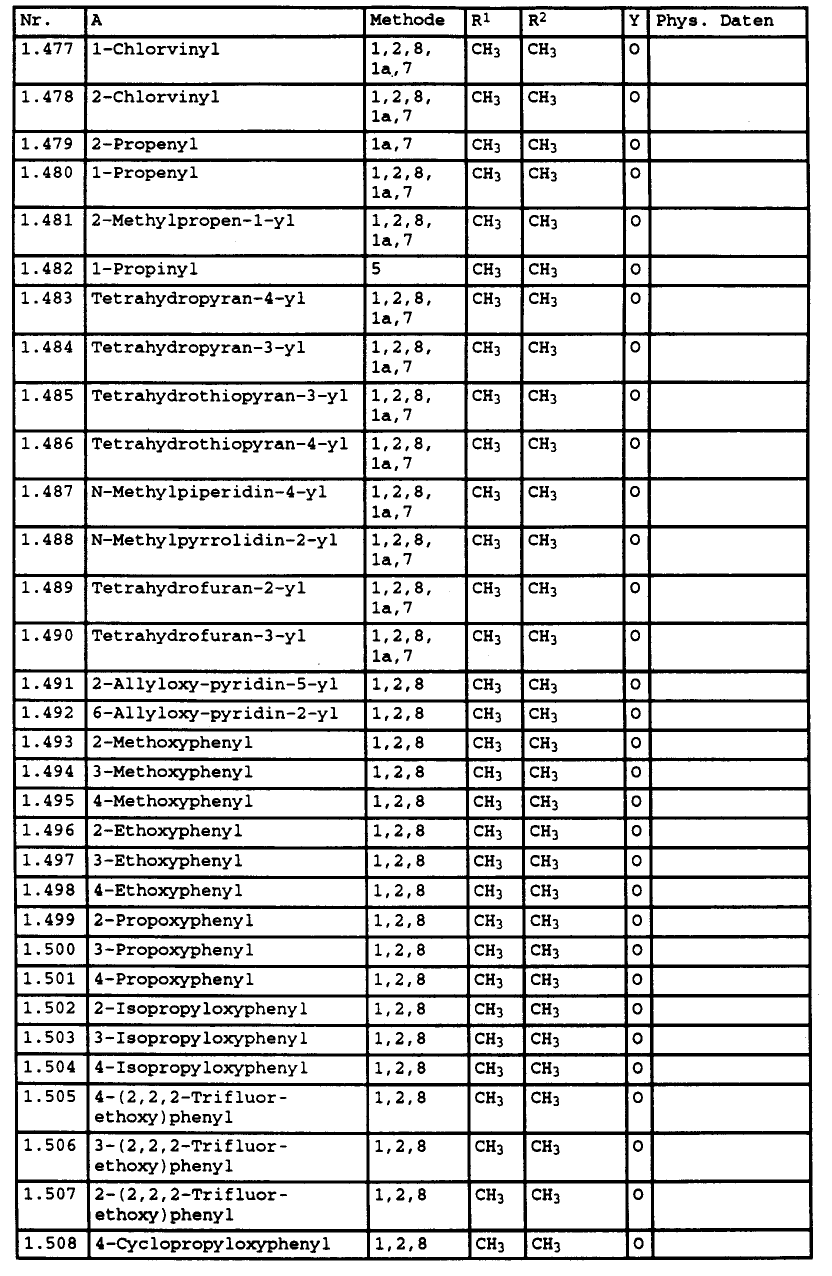

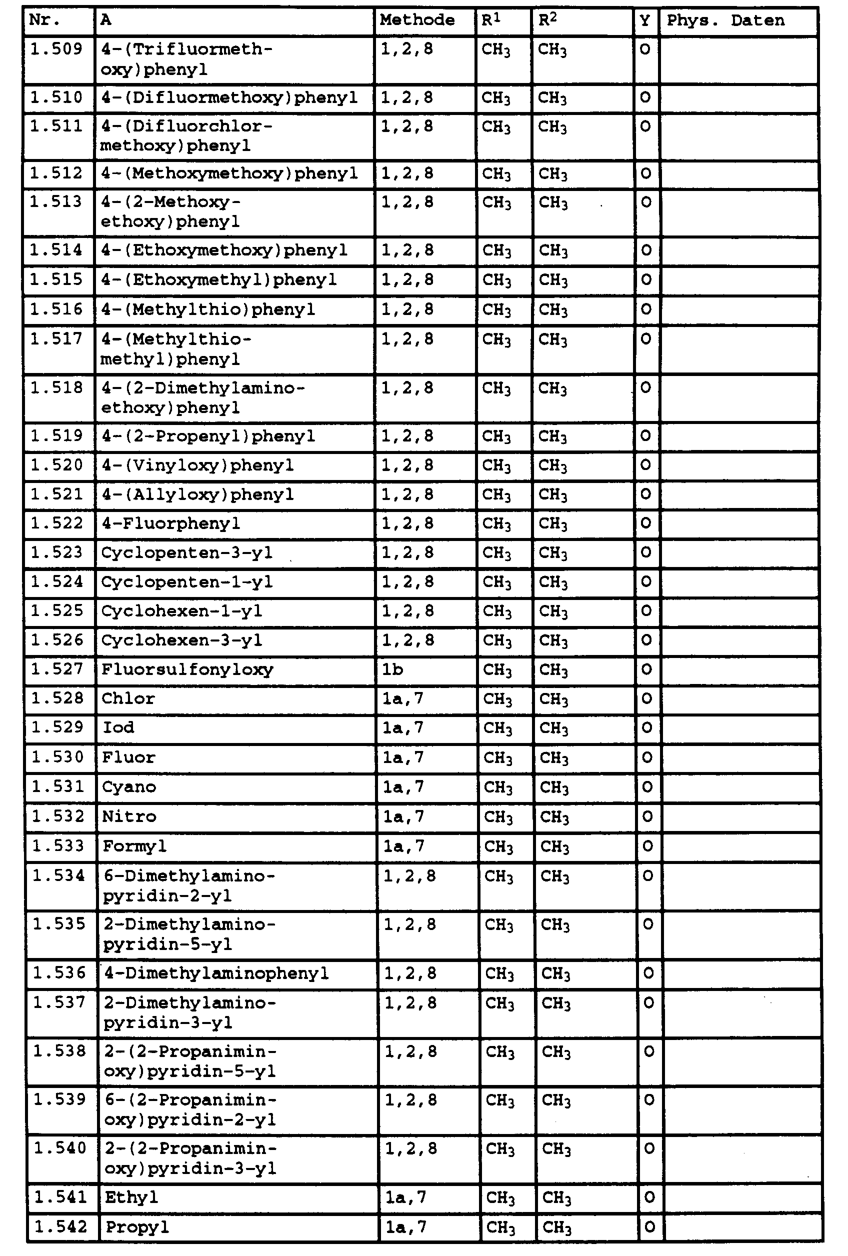

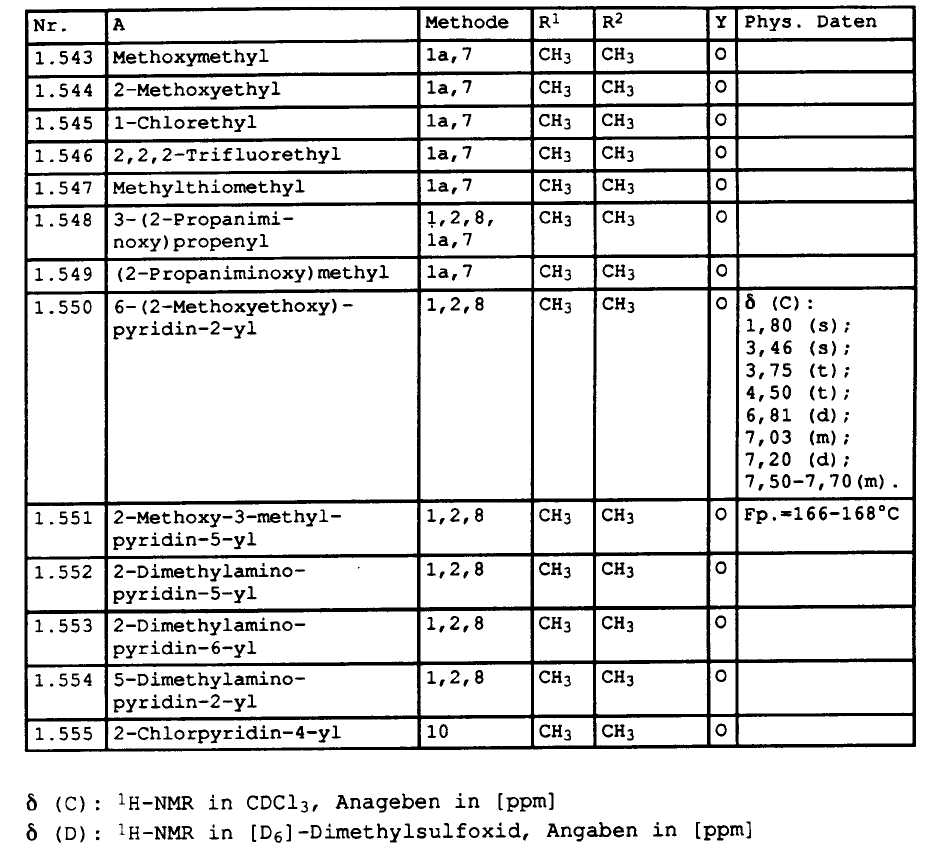

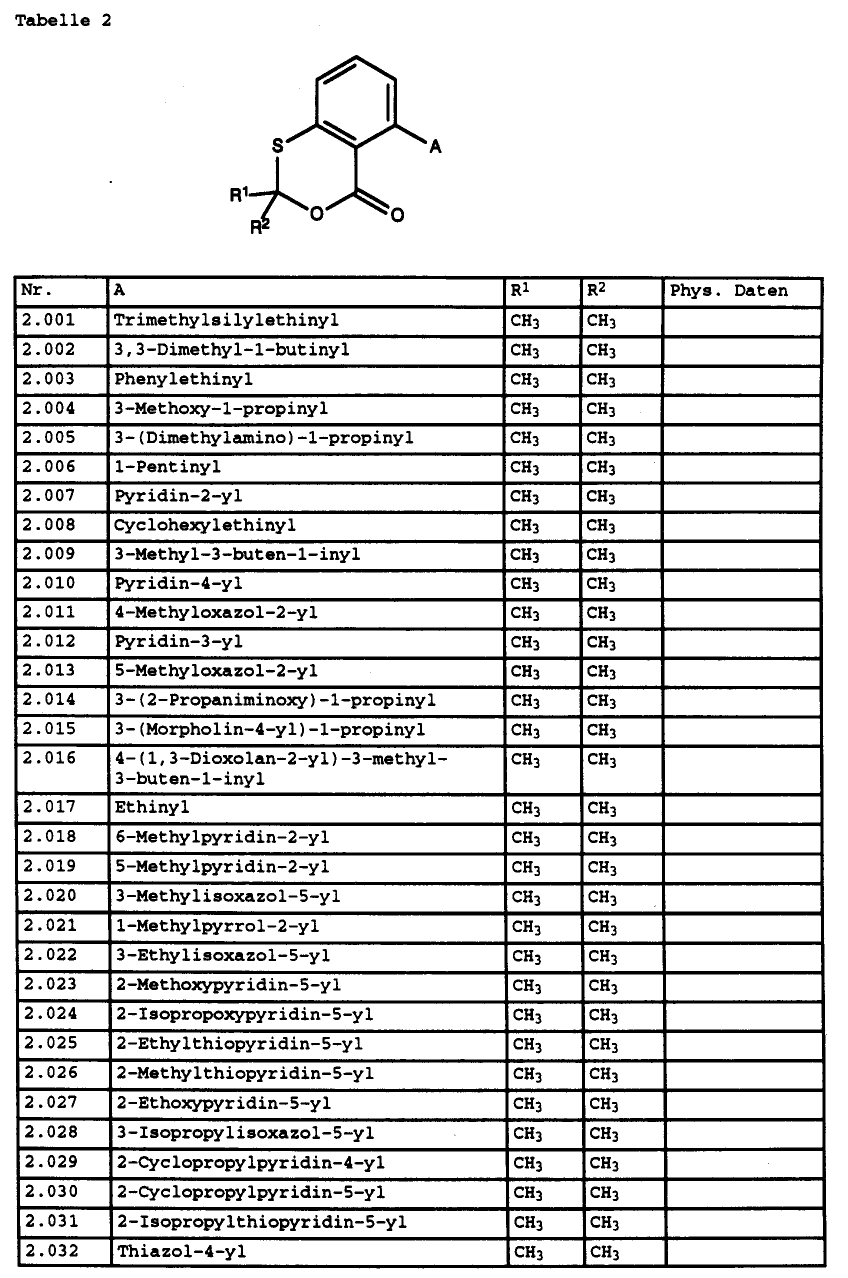

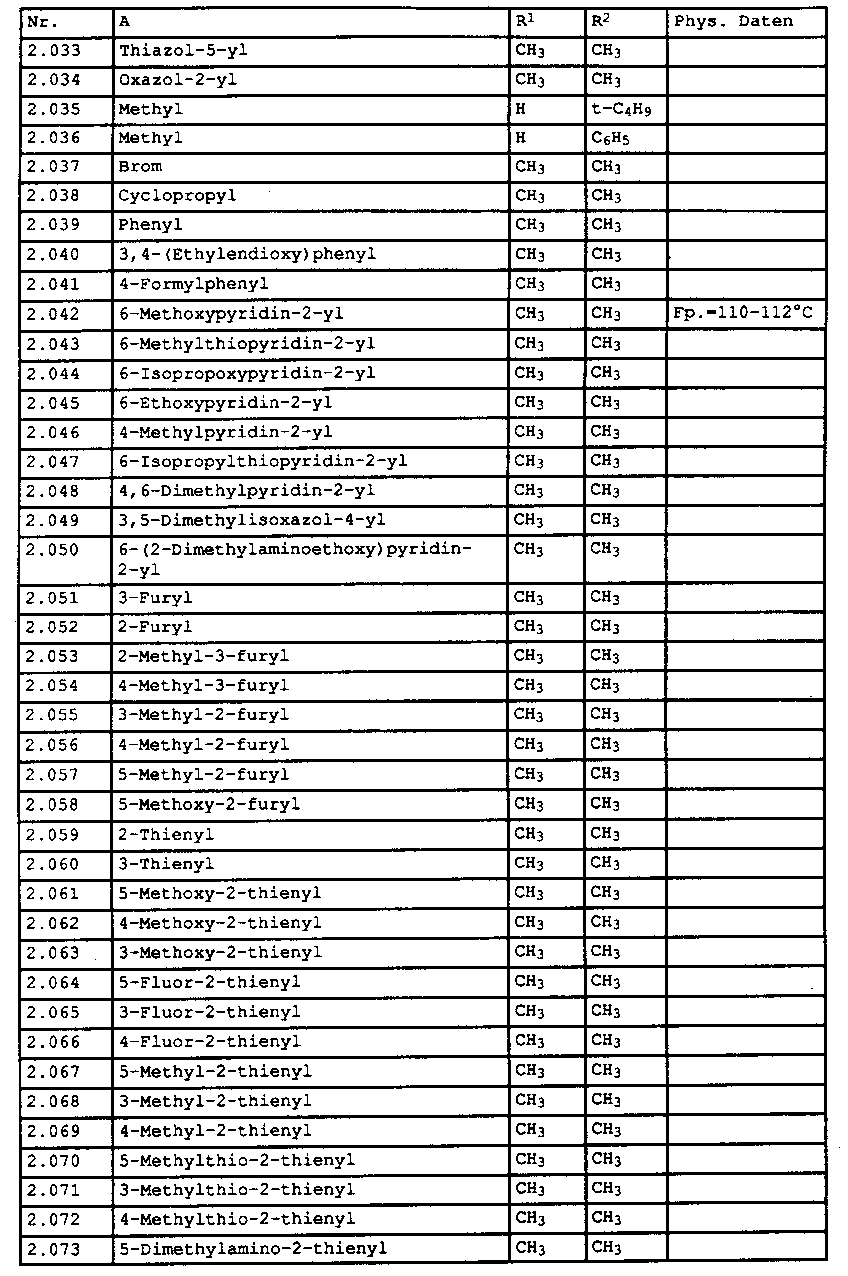

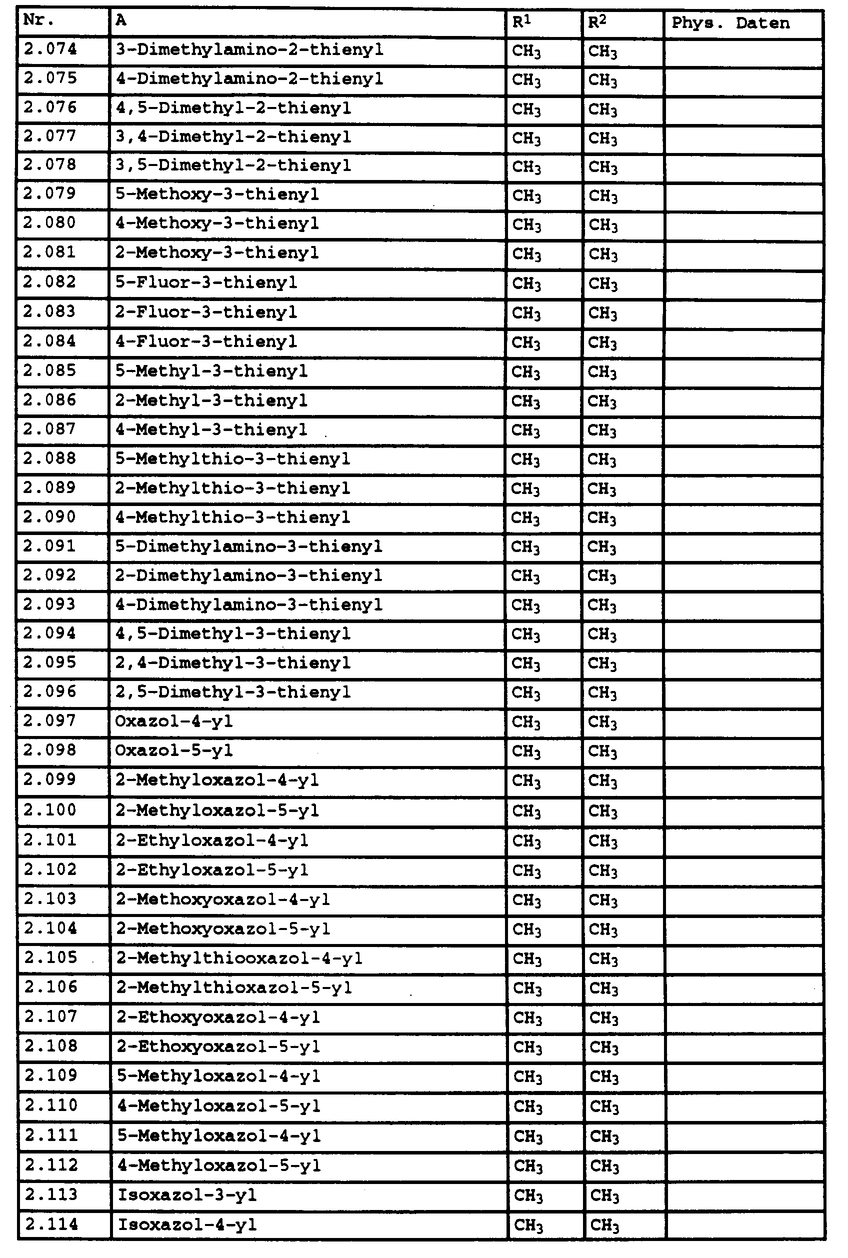

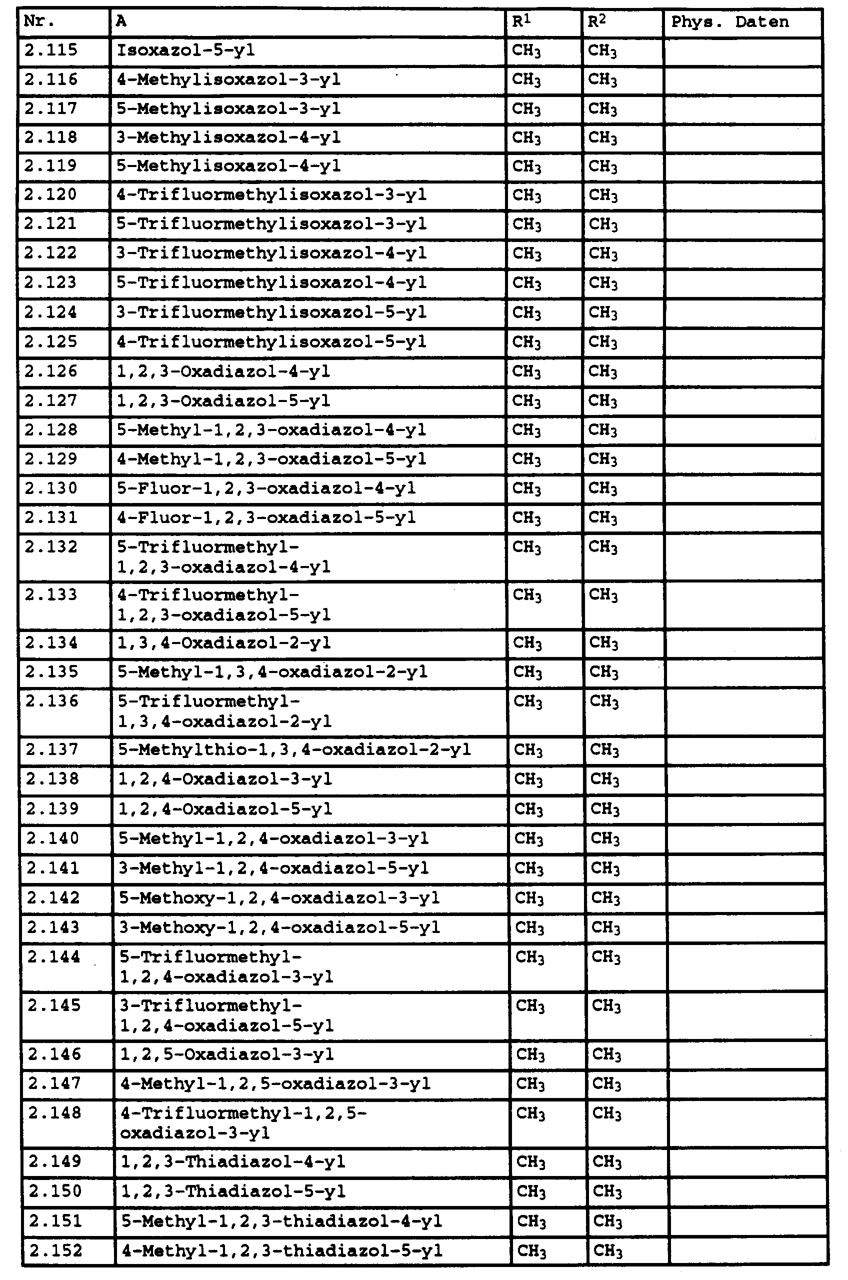

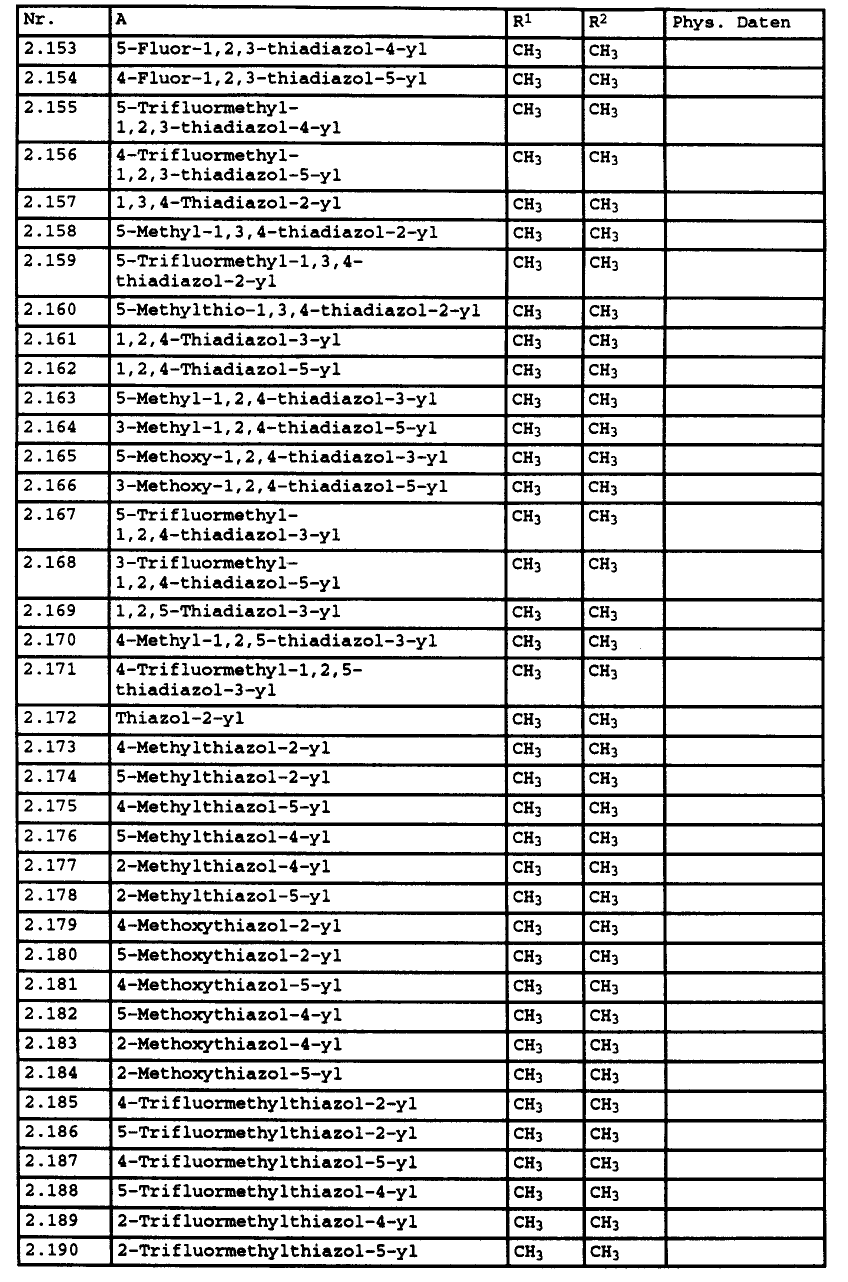

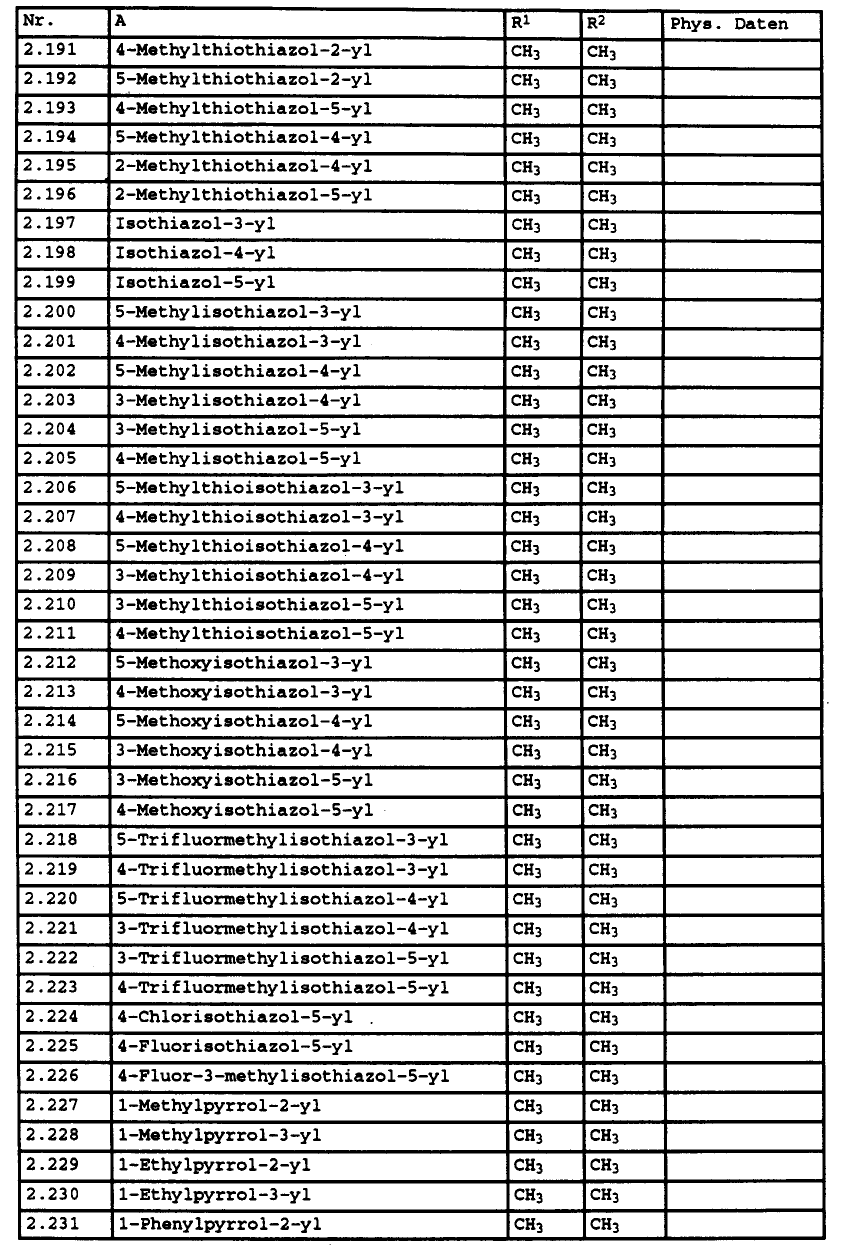

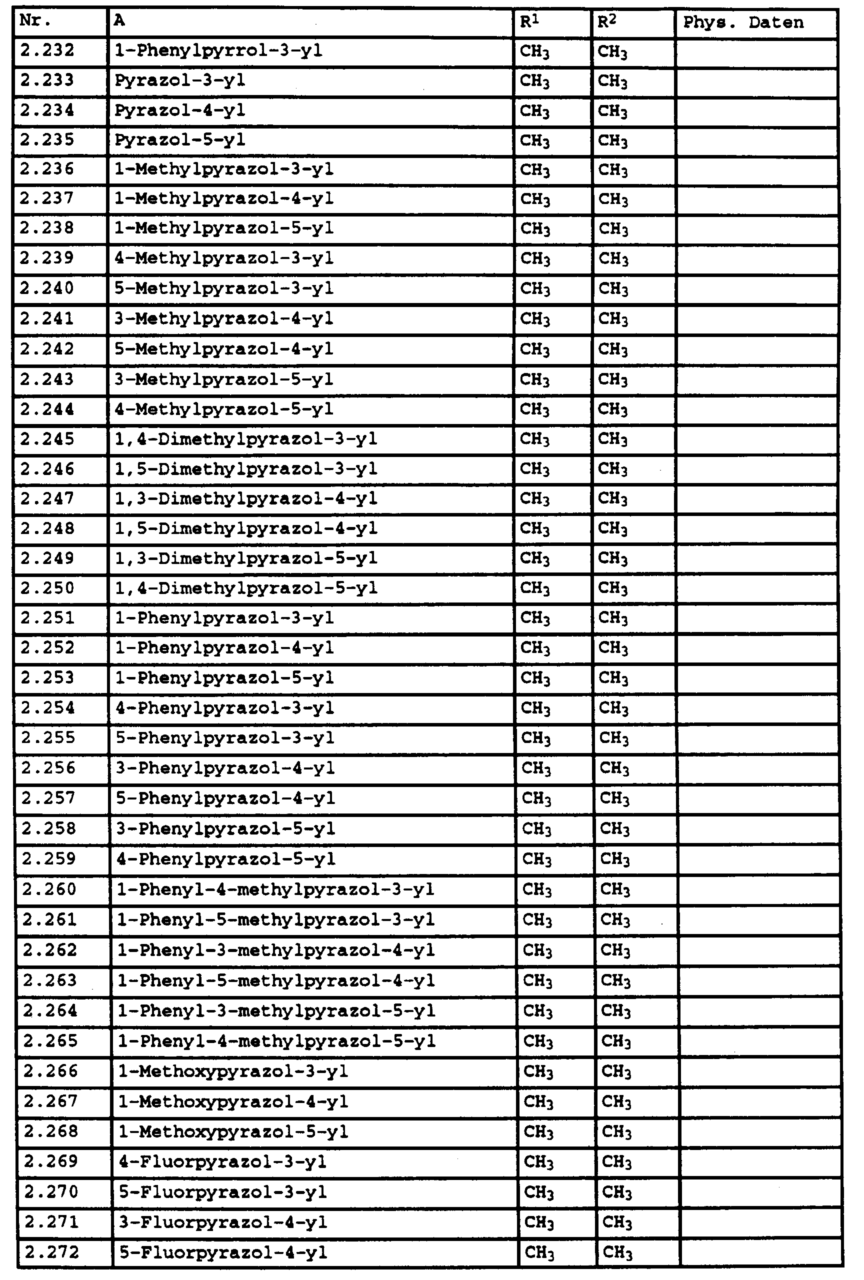

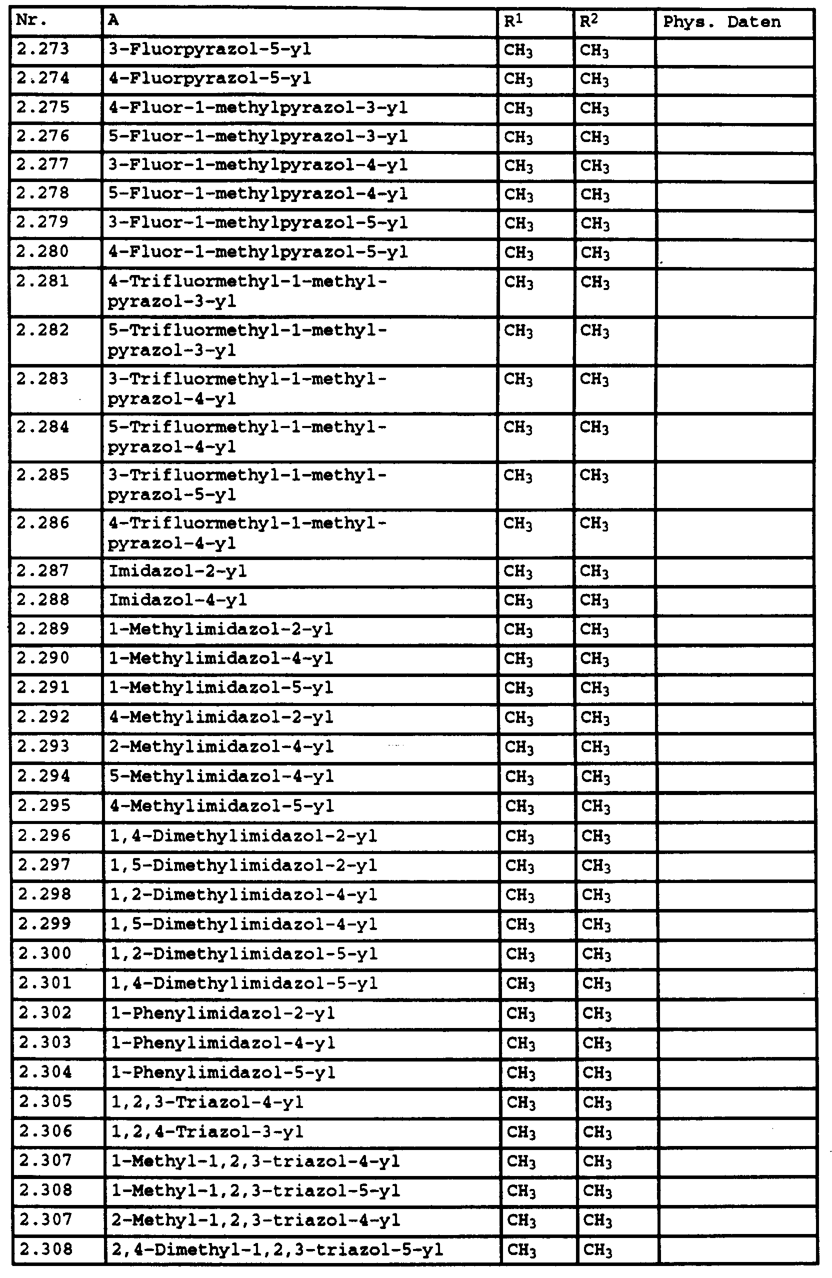

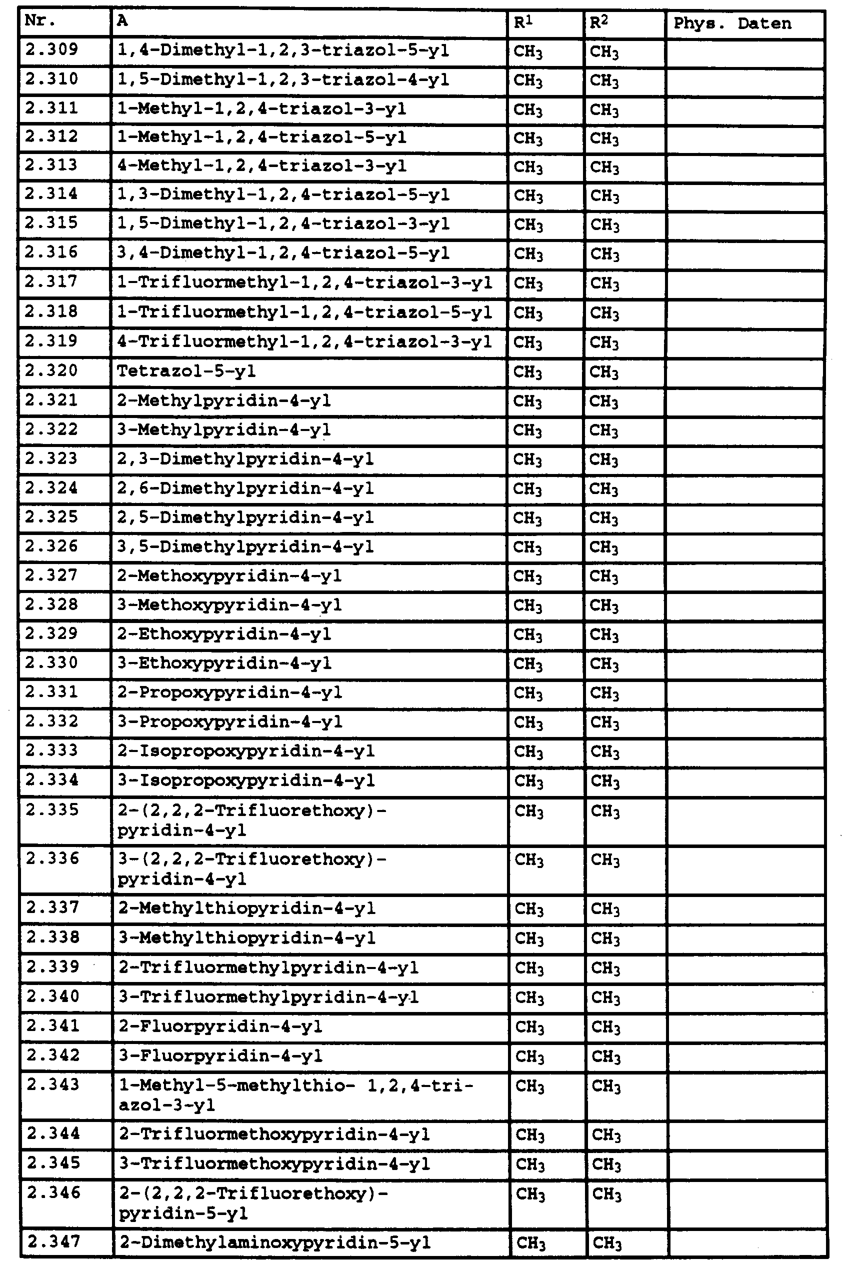

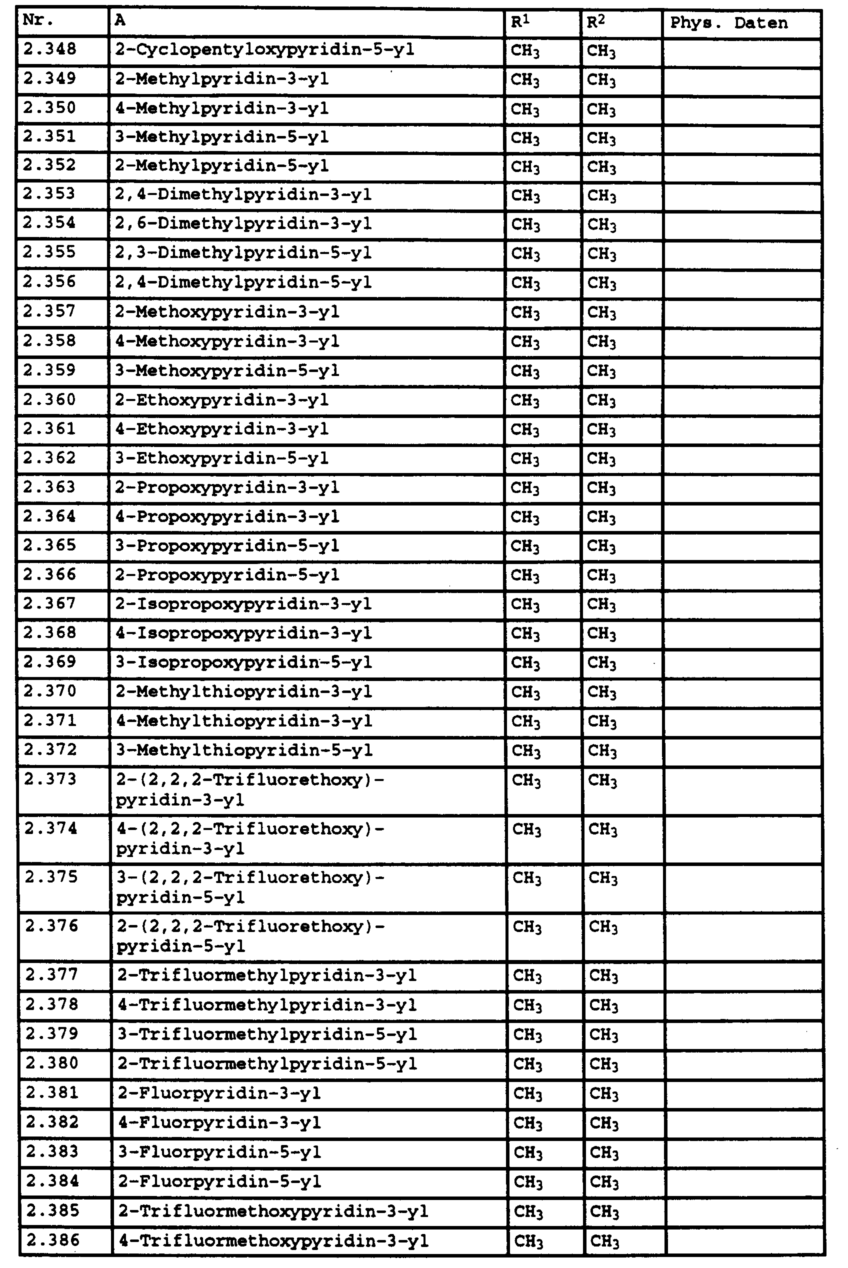

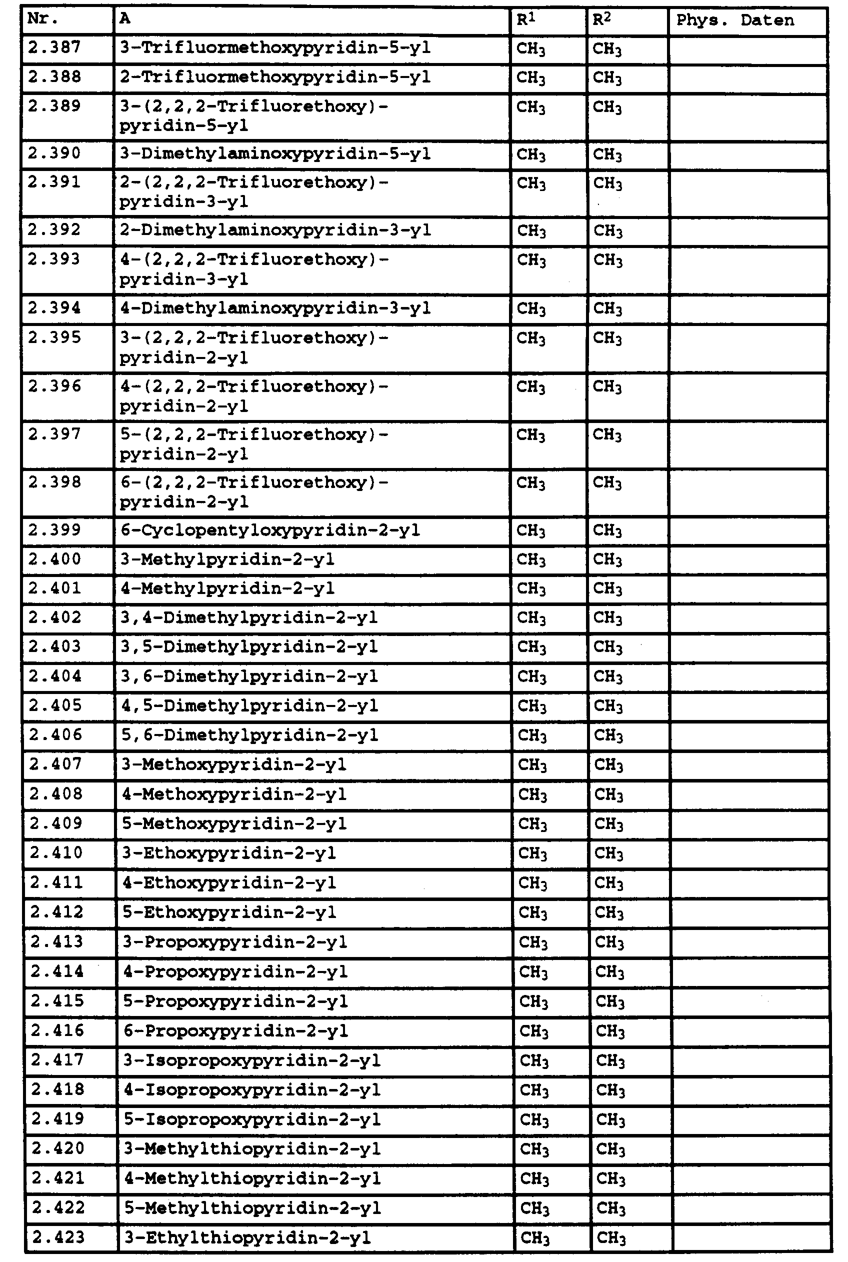

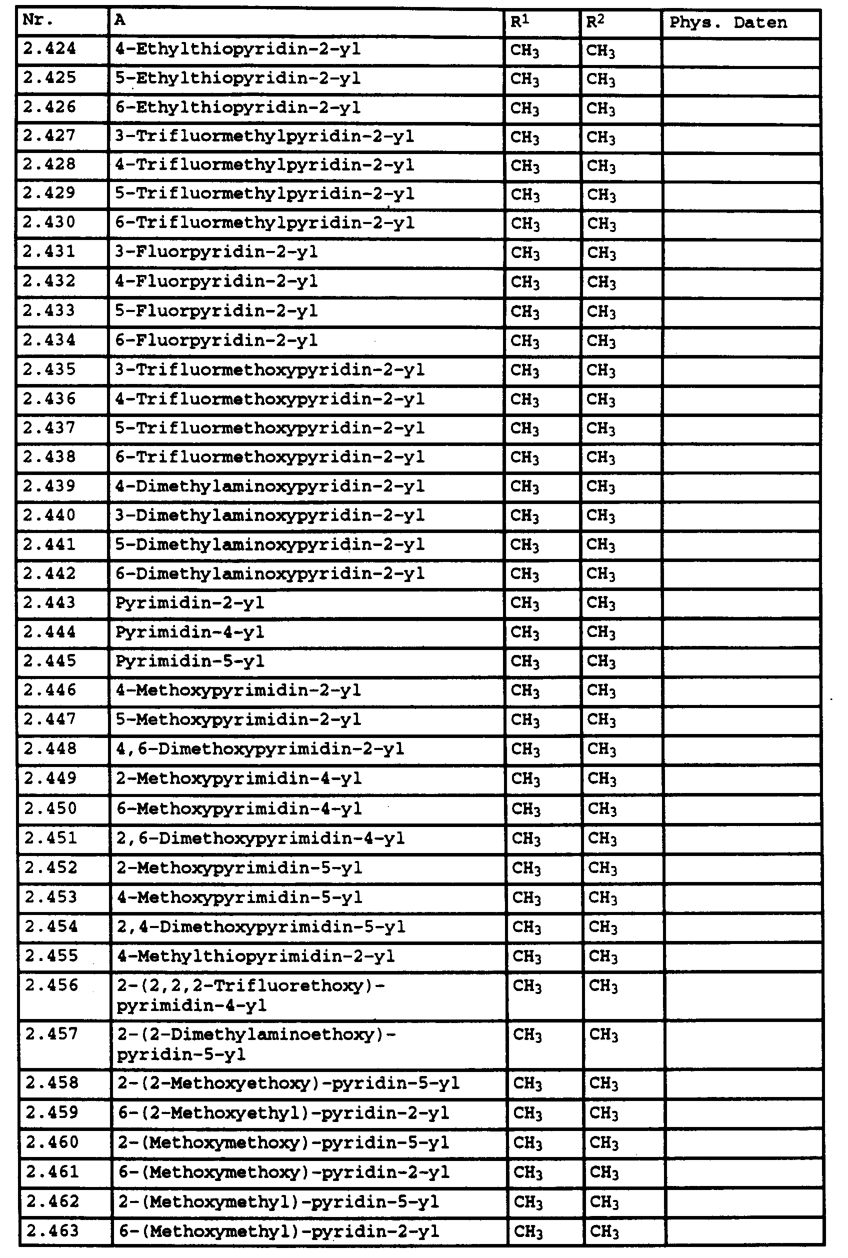

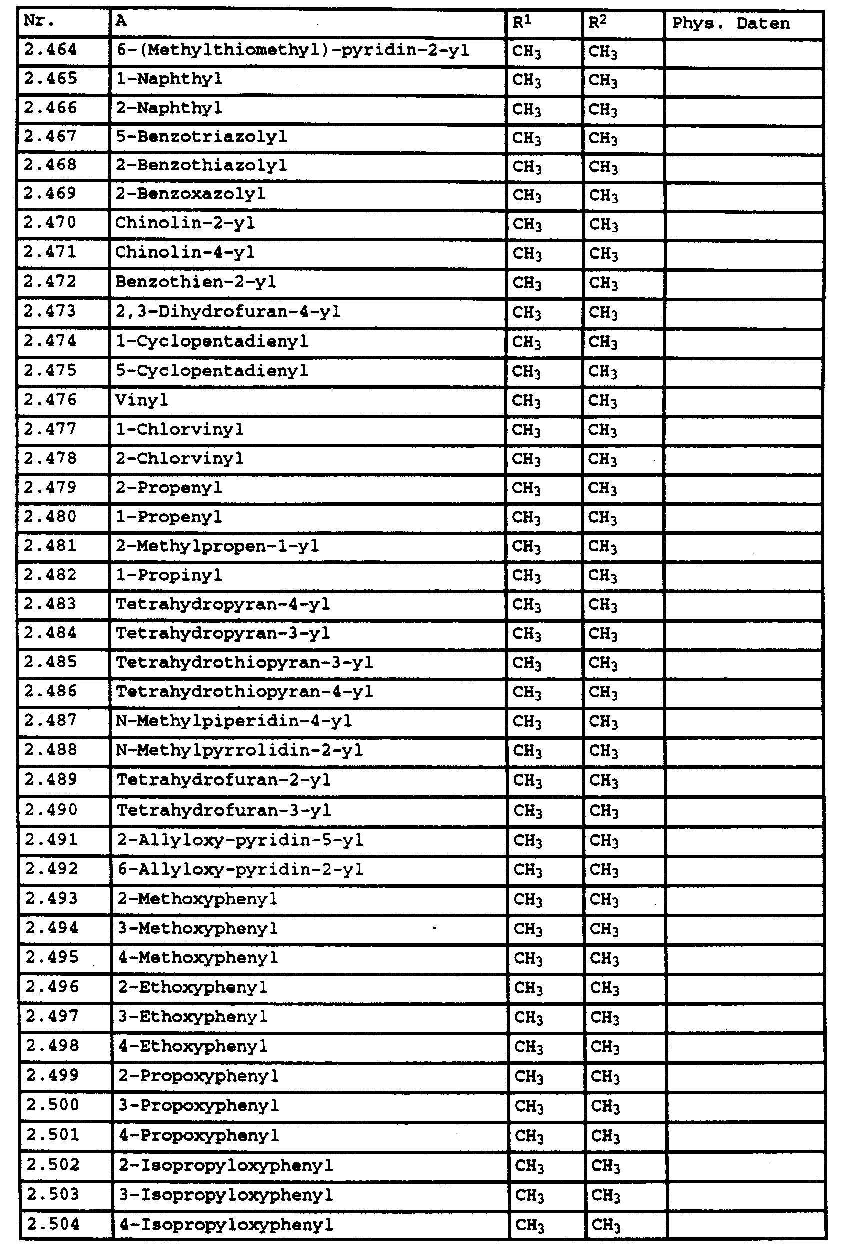

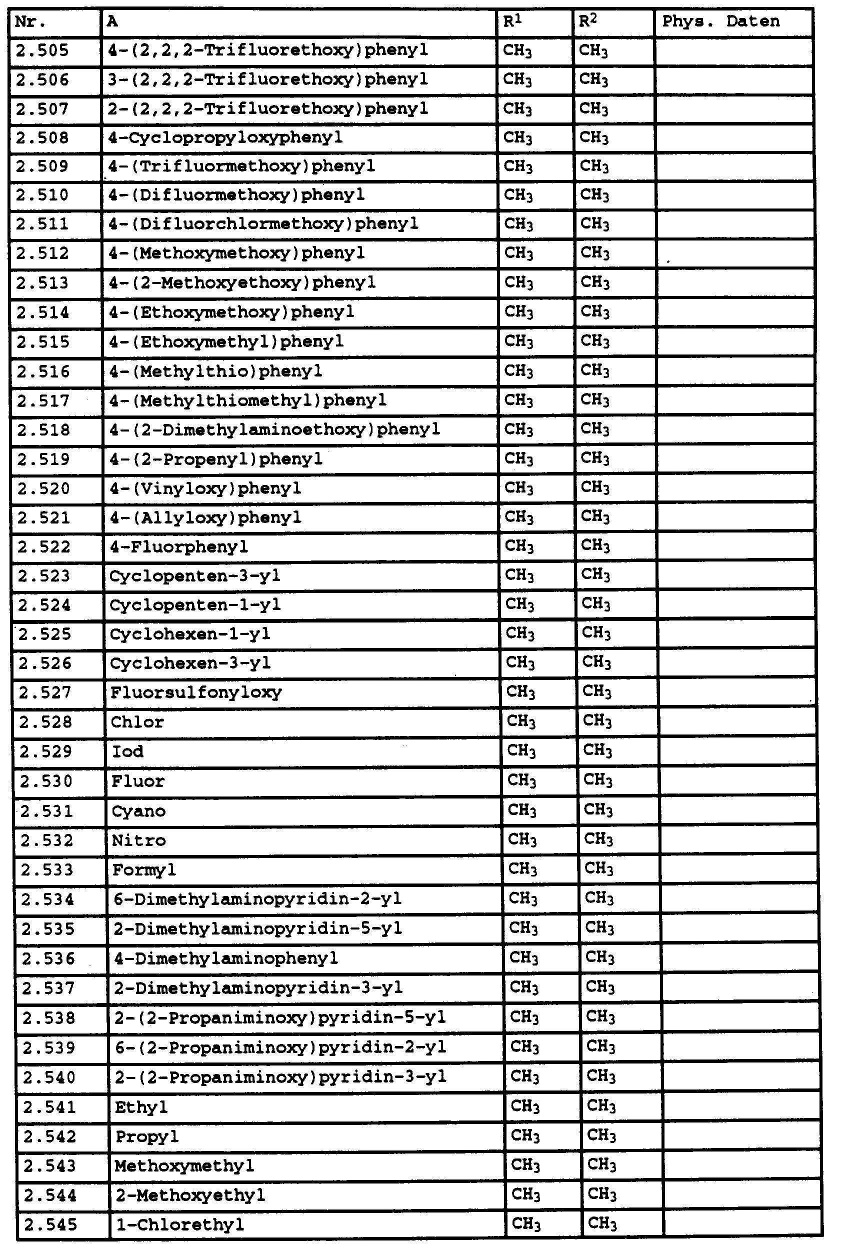

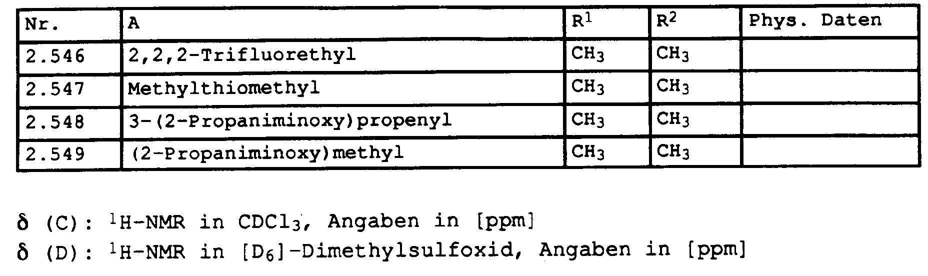

Die Verbindungen I bzw. die sie enthaltenden herbiziden Mittel sowie deren umweltverträgliche Salze von beispielsweise Alkalimetallen, Erdalkalimetallen oder Ammoniak und Aminen bzw. die sie enthaltenden herbiziden Mittel können in Kulturen wie Weizen, Reis, Mais, Soja und Baumwolle Unkräuter und Schadgräser sehr gut bekämpfen, ohne die Kulturpflanzen nennenswert zu schädigen. Dieser Effekt tritt vor allem bei niedrigen Aufwandmengen auf.

Unter Berücksichtigung der Vielseitigkeit der Applikationsmethoden können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus lassen sich die Verbindungen I auch in Kulturen, die durch Züchtung und/oder mittels gentechnischer Methoden gegen die Wirkung von I oder anderen Herbiziden weitgehend resistent gemacht wurden, einsetzen.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Öldispersionen kommen im wesentlichen in Betracht: Mineralölfraktionen von mittlerem bis hohem Siedepunkt wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline oder deren Derivate, alkylierte Benzole und deren Derivate, Alkohole wie Methanol, Ethanol, Propanol, Butanol und Cyclohexanol, Ketone wie Cyclohexanon oder stark polare Lösungsmittel, z.B. Amine wie N-Methylpyrrolidon, oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.-%, vorzugsweise zwischen 0,5 und 90 Gew.-%, Wirkstoff. Die Wirkstoffe werden dabei in einer Reinheit von 90 % bis 100 %, vorzugsweise 95 % bis 100 % (nach NMR-Spektrum) eingesetzt. Die erfindungsgemäßen Verbindungen I können beispielsweise wie folgt formuliert werden:
I. 20 Gewichtsteile der Verbindung Nr. I werden in einer Mischung gelöst, die aus 80 Gewichtsteilen alkyliertem Benzol; 10 Gewichtsteilen des Anlagerungsproduktes von 8 bis 10 Mol Ethylenoxid an 1 Mol Ölsäure-H-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
II. 20 Gewichtsteile der Verbindung Nr. I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
III. 20 Gewichtsteile des Wirkstoffs Nr. I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
IV. 20 Gewichtsteile des Wirkstoffs Nr. I werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
V. 3 Gewichtsteile des Wirkstoffs Nr. I werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. I werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Zur Verbreiterung des Wirkungsspektrums und zur Erzielung synergistischer Effekte können die cyclischen Acetale I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam aus zubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0 kg/ha aktive Substanz (a.S.)

### Anwendungsbeispiele

Die herbizide Wirkung der cyclischen Acetale der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung betrug 3,0 kg/ha a.S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

## Patentansprüche

1. Cyclische Acetale der Formel I 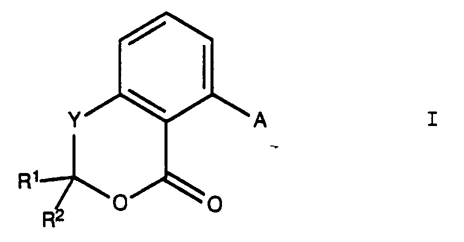 wobei die Substituenten folgende Bedeutung haben:
R¹, R²
Wasserstoff;
C₁-C₄-Alkyl, wobei dieser Rest jeweils ein bis fünf Halogenatome und/oder ein bis zwei C₁-C₄-Alkoxy-Gruppen tragen kann;
Phenyl, wobei dieser Rest jeweils ein bis fünf Halogenatome und/oder ein bis zwei der folgenden Gruppen tragen kann: C₁-C₄-Alkyl, C₁-C₄-Alkoxy, C₁-C₄-Halogenalkyl oder Nitro;
ferner können die beiden Reste gemeinsam eine C₂-C₆-Alkylenkette sein, die die durch ein bis fünf Halogenatome und/oder C₁-C₄-Alkylreste substituiert sein kann;
Y
Sauerstoff oder Schwefel;
A
ein Rest A¹ bis A⁶;
A¹
Hydroxy;
A²
ein Halogenatom, C₁-C₄-Halogenalkylsulfonyloxy, C₁-C₄-Alkylsulfonyloxy oder Fluorsulfonyloxy;
A³
Cyano, Nitro, Formyl;
A⁴
ein Phenylring oder ein 5- oder 6-gliedriger gesättigter oder ungesättigter Heterocyclus mit bis zu vier Heteroatomen aus der Gruppe: Stickstoff, Schwefel, Sauerstoff im Ring, die jeweils durch bis zu fünf Reste R³¹ bis R³⁵ substituiert sein können;
Naphthyl oder ein benzokondensierter 5- oder 6-gliedriger Heteroaromat mit 1 bis 3 Heteroatomen aus der Gruppe: Stickstoff, Schwefel, Sauerstoff im Ring, der durch bis zu fünf Reste R³¹ bis R³⁵ substituiert sein kann;
A⁵
eine C₂-C₆-Alkenyl-, C₃-C₈-Cycloalkenyl-, C₅-C₈-Cycloalkadienyl- oder C₂-C₆-Alkinyl-Gruppe, die jeweils bis zu sieben Substituenten R³¹ bis R³⁷ tragen können;
A⁶
eine C₁-C₈-Alkyl- oder C₃-C₈-Cycloalkyl-Gruppe, die jeweils bis zu sieben Substituenten R³¹ bis R³⁷ tragen können;
R³¹, R³², R³³, R³⁴, R³⁵, R³⁶, R³⁷
a) eine C₃-C₈-Cycloalkylgruppe, welche ein bis drei C₁-C₄-Alkylreste tragen kann;
b) eine C₁-C₈-Alkylgruppe, welche ein bis fünf Halogenatome und/oder einen der folgenden Reste tragen kann:
C₁-C₄-Alkoxy, C₁-C₄-Halogenalkoxy, C₁-C₄-Alkylthio, Cyano, C₃-C₈-Cycloalkyl oder Di-C₁-C₄-Alkylamino;
c) eine C₁-C₈-Alkoxygruppe oder eine C₃-C₅-Cycloalkoxygruppe, welche jeweils ein bis fünf Halogenatome und/oder einen der folgenden Reste tragen können:
C₁-C₄-Alkoxy, C₁-C₄-Halogenalkoxy, C₁-C₄-Alkylthio, C₃-C₈-Cycloalkyl oder Di-C₁-C₄-Alkylamino;
d) eine C₁-C₄-Alkylthiogruppe, welche ein bis fünf Halogenatome und/oder einen der folgenden Reste tragen kann:
C₁-C₄-Alkoxy, C₁-C₄-Halogenalkoxy, C₁-C₄-Alkylthio, C₃-C₈-Cycloalkyl oder Di-C₁-C₄-Alkylamino;
e) eine Di-C₁-C₄-Alkylamino-, eine Di-C₁-C₄-Alkylaminoxygruppe, eine C₅-C₈-Cycloalkaniminoxygruppe oder eine C₁-C₁₀-Alkaniminoxygruppe;
f) eine C₂-C₆-Alkenyl- oder eine C₂-C₆-Alkinylgruppe, welche ein bis fünf Halogenatome und/oder einen der folgenden Reste tragen kann:
C₁-C₄-Alkoxy, C₁-C₄-Halogenalkoxy oder C₁-C₄-Alkylthio;
g) Wasserstoff;
h) Nitro, Halogen, Cyano, Tri-C₁-C₄-alkylsilyl;
mit der Maßgabe, daß A nicht für eine Hydroxy-Gruppe steht, wenn R¹ und R² beide Wasserstoff bedeuten oder wenn R¹ Wasserstoff und R² Phenyl bedeutet.

2. Verfahren zur Herstellung der cyclischen Acetale gemäß Anspruch 1 mit A=A⁴ oder A⁵, dadurch gekennzeichnet, daß man Acetale I mit A=A² mit Verbindungen der Formel X-A⁴ oder X-A⁵, wobei R¹, R², Y, A², A⁴ und A⁵ die in Anspruch 1 genannte Bedeutung haben, und X Wasserstoff, Trialkylstannyl, Dihydroxyboranyl, Dialkoxyboranyl oder Alkylendioxyboranyl bedeutet, in Gegenwart einer katalytisch wirksamen Palladiumverbindung umsetzt.

3. Verfahren zur Herstellung der cyclischen Acetale gemäß Anspruch 1 mit A=A⁴, dadurch gekennzeichnet, daß man eine Cycloaddition zwischen den Acetalen I mit A=A⁵ und einem entsprechenden Dien, Heterodien oder 1,3-Dipol vornimmt.

4. Verwendung der cyclischen Acetale gemäß Anspruch 1 zur Herstellung von Pflanzenschutzwirkstoffen der Formel IV 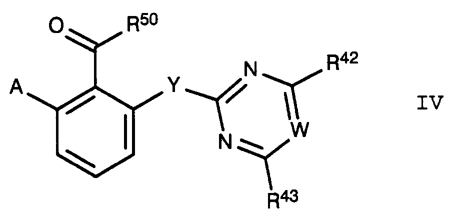 wobei Y und A die unter Anspruch 1 angegebenen Bedeutungen haben und die übrigen Reste die folgenden Bedeutungen haben:
R⁵⁰
a) ein über ein Stickstoffatom verknüpfter 5-gliedriger Heteroaromat, enthaltend zwei bis drei Stickstoffatome, welcher ein bis zwei Halogenatome und/oder ein bis zwei der folgenden Reste tragen kann:
C₁-C₄-Alkyl, C₁-C₄-Halogenalkyl, C₁-C₄-Alkoxy, C₁-C₄-Halogenalkoxy und/oder C₁-C₄-Alkylthio;
b) ein Rest -(O)m-NR⁶R⁷,
in dem m für 0 oder 1 steht und R⁶ und R⁷, die gleich oder unterschiedlich sein können, die folgende Bedeutung haben:
Wasserstoff;
C₁-C₈-Alkyl, C₃-C₆-Alkenyl, C₃-C₆-Alkinyl, C₃-C₈ Cycloalkyl, wobei diese Reste jeweils ein bis fünf Halogenatome und/oder ein bis zwei der folgenden Gruppen tragen können: C₁-C₄ Alkoxy, C₃-C₆ Alkenyloxy, C₃-C₆-Alkinyloxy, C₁-C₄-Alkylthio, C₃-C₆-Alkenylthio, C₃-C₆-Alkinylthio, C₁-C₄-Halogenalkoxy, C₁-C₄-Alkylcarbonyl, C₃-C₆-Alkenylcarbonyl, C₃-C₆-Alkinylcarbonyl, C₁-C₄-Alkoxycarbonyl, C₃-C₆-Alkenyloxycarbonyl, C₃-C₆-Alkinyloxycarbonyl, Di-C₁-C₄-alkylamino, C₃-C₈-Cycloalkyl, Phenyl, ein oder mehrfach durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl;
Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio;
R⁶ und R⁷ gemeinsam eine zu einem Ring geschlossene, optionell substituierte C₄-C₇-Alkylenkette oder gemeinsam eine zu einem Ring geschlossene, optionell substituierte C₃-C₆-Alkylenkette mit einem Heteroatom, ausgewählt aus der Gruppe Sauerstoff, Schwefel oder Stickstoff;
c) R⁵⁰ ferner eine Gruppe 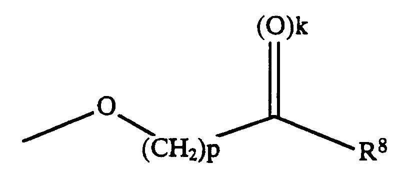 in der R⁸ für C₁-C₄-Alkyl, Phenyl, ein- oder mehrfach durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl, C₁-C₄-Halogenalkyl, C₃-C₆-Alkenyl oder C₃-C₆-Alkinyl steht, p die Werte 1, 2, 3 oder 4 und k die Werte 0, 1 oder 2 annehmen;
d) einen Rest OR⁹, worin R⁹ bedeutet:
I) eine C₃-C₈-Cycloalkylgruppe, welche ein bis drei C₁-C₄-Alkylreste tragen kann;
II) eine C₁-C₈-Alkylgruppe, welche ein bis fünf Halogenatome und/oder einen der folgenden Reste tragen kann:
C₁-C₄-Alkoxy, C₁-C₄-Alkylthio, Cyano, C₁-C₄-Alkylcarbonyl, C₃-C₈-Cycloalkyl, C₁-C₄-Alkoxycarbonyl, Phenyl, ein- oder mehrfach durch Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio substituiertes Phenyl oder Phenoxy;
III) eine C₁-C₈-Alkylgruppe welche ein bis fünf Halogenatome tragen kann und einen der folgenden Reste trägt: ein 5-gliedriger Heteroaromat, enthaltend ein bis drei Stickstoffatome, oder ein 5-gliedriger Heteroaromat enthaltend ein Stickstoffatom und ein Sauerstoff- oder Schwefelatom, welche ein bis vier Halogenatome und/oder ein bis zwei der folgenden Reste tragen können: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
IV) eine C₂-C₆-Alkylgruppe, welche in der 2-Position einen der folgenden Reste trägt: C₁-C₄-Alkoxyimino, C₃-C₆-Alkenyloxyimino, C₃-C₆-Halogenalkenyloxyimino oder Benzyloxyimino;
V) eine C₃-C₆-Alkenyl- oder eine C₃-C₆-Alkinylgruppe, wobei diese Gruppen ihrerseits ein bis fünf Halogenatome tragen können;
VI) ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
VII) ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
VIII) R⁹ ferner eine Gruppe -N=CR¹⁰R¹¹, worin R¹⁰ und R¹¹, die gleich oder verschieden sind, bedeuten:
C₁-C₁₂-Alkyl, C₃-C₆-Alkenyl, C₃-C₆-Alkinyl, C₃-C₈-Cycloalkyl, wobei diese Reste einen C₁-C₄-Alkoxy-, C₁-C₄-Alkylthio und/oder einen Phenylrest tragen können;
Phenyl, das durch einen oder mehrere der folgenden Reste substituiert sein kann:
Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio;
oder R¹⁰ und R¹¹ bilden gemeinsam eine C₃-C₁₂-Alkylenkette, welche ein bis drei C₁-C₄-Alkylgruppen tragen kann;
e) oder R⁵⁰ bildet einen Rest -NH-SO₂-R¹², in dem R¹² bedeutet:
C₁-C₄-Alkyl, C₃-C₆-Alkenyl, C₃-C₆-Alkinyl, C₃-C₈-Cycloalkyl, wobei diese Reste einen C₁-C₄-Alkoxy-, C₁-C₄-Alkylthio und/oder einen Phenylrest tragen können;
Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio.

5. Herbizides Mittel, gekennzeichnet durch einen Gehalt an mindestens einem cyclischen Acetal der Formel I gemäß Anspruch 1 und üblichen inerten Trägerstoffen.

6. Verfahren zur Bekämpfung unerwünschten Pflanzenwuchses , dadurch gekennzeichnet, daß man eine herbizid wirksame Menge eines cyclischen Acetals der Formel I gemäß Anspruch 1 einschließlich von Verbindungen mit A = Hydroxy, wenn R¹ und R² beide Wasserstoff bedeuten oder wenn R¹ Wasserstoff und R² Phenyl bedeutet.

## Claims

1. A cyclic acetal of the formula I 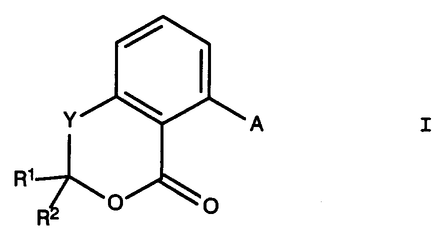 where the substituents have the following meanings:
R¹ and R² are
hydrogen;
C₁-C₄-alkyl, this radical in each case being able to carry one to five halogen atoms and/or one to two C₁-C₄-alkoxy groups;
phenyl, this radical in each case being able to carry one to five halogen atoms and/or one to two of the following groups: C₁-C₄-alkyl, C₁-C₄-alkoxy, C₁-C₄-haloalkyl or nitro;
in addition the two radicals together can be a C₂-C₆-alkylene chain which can be substituted by one to five halogen atoms and/or C₁-C₄-alkyl radicals;
Y is
oxygen or sulfur;
A is
a radical A¹ to A⁶;
A¹ is
hydroxyl;
A² is
a halogen atom, C₁-C₄-haloalkylsulfonyloxy, C₁-C₄-alkylsulfonyloxy or fluorosulfonyloxy;
A³ is
cyano, nitro or formyl;
A⁴ is
a phenyl ring or a 5- or 6-membered saturated or unsaturated heterocycle having up to four heteroatoms from the group consisting of nitrogen, sulfur and oxygen in the ring, each of which can be substituted by up to five radicals R³¹ to R³⁵;
naphthyl or a benzo-fused 5- or 6-membered heteroaromatic having 1 to 3 heteroatoms from the group consisting of nitrogen, sulfur and oxygen in the ring, which can be substituted by up to five radicals R³¹ to R³⁵;
A⁵ is
a C₂-C₆-alkenyl, C₃-C₈-cycloalkenyl, C₅-C₈-cycloalkadienyl or C₂-C₆-alkynyl group, each of which can carry up to seven substituents R³¹ to R³⁷;
A⁶ is
a C₁-C₈-alkyl or C₃-C₈-cycloalkyl group, each of which can carry up to seven substituents R³¹ to R³⁷;
R³¹, R³², R³³, R³⁴, R³⁵, R³⁶ and R³⁷ are
a) a C₃-C₈-cycloalkyl group which can carry one to three C₁-C₄-alkyl radicals;
b) a C₁-C₈-alkyl group which can carry one to five halogen atoms and/or one of the following radicals:
C₁-C₄-alkoxy, C₁-C₄-haloalkoxy, C₁-C₄-alkylthio, cyano, C₃-C₈-cycloalkyl or di-C₁-C₄-alkylamino;
c) a C₁-C₈-alkoxy group or a C₃-C₅-cycloalkoxy group, each of which can carry one to five halogen atoms and/or one of the following radicals:
C₁-C₄-alkoxy, C₁-C₄-haloalkoxy, C₁-C₄-alkylthio, C₃-C₈-cycloalkyl or di-C₁-C₄-alkylamino;
d) a C₁-C₄-alkylthio group which can carry one to five halogen atoms and/or one of the following radicals:
C₁-C₄-alkoxy, C₁-C₄-haloalkoxy, C₁-C₄-alkylthio, C₃-C₈-cycloalkyl or di-C₁-C₄-alkylamino;
e) a di-C₁-C₄-alkylamino or di-C₁-C₄-alkylaminoxy group, a C₅-C₈-cycloalkaniminoxy group or a C₁-C₁₀-alkaniminoxy group;
f) a C₂-C₆-alkenyl or a C₂-C₆-alkynyl group, which can carry one to five halogen atoms and/or one of the following radicals:
C₁-C₄-alkoxy, C₁-C₄-haloalkoxy or C₁-C₄-alkylthio;
g) hydrogen;
h) nitro, halogen, cyano or tri-C₁-C₄-alkylsilyl;
with the proviso that A is not a hydroxyl group if R¹ and R² are both hydrogen or if R¹ is hydrogen and R² is phenyl.

2. A process for preparing the cyclic acetals as claimed in claim 1 where A=A⁴ or A⁵, which comprises reacting acetals I where A=A² with compounds of the formula X-A⁴ or X-A⁵, where R¹, R², Y, A², A⁴ and A⁵ have the meanings mentioned in claim 1, and X is hydrogen, trialkylstannyl, dihydroxyboranyl, dialkoxyboranyl or alkylenedioxyboranyl, in the presence of a catalytically active palladium compound.

3. A process for preparing the cyclic acetals as claimed in claim 1 where A=A⁴, which comprises carrying out a cycloaddition between the acetal I where A=A⁵ and an appropriate diene, heterodiene or 1,3-dipolar compound.

4. The use of the cyclic acetals as claimed in claim 1 for preparing crop protection active compounds of the formula IV 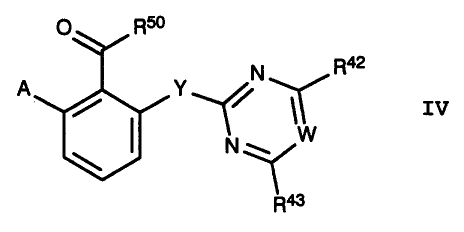 where Y and A have the meanings indicated under claim 1 and the other radicals have the following meanings:
R⁵⁰ is
a) a 5-membered heteroaromatic linked via a nitrogen atom, containing two to three nitrogen atoms, which can carry one to two halogen atoms and/or one to two of the following radicals:
C₁-C₄-alkyl, C₁-C₄-haloalkyl, C₁-C₄-alkoxy, C₁-C₄-halo-alkoxy and/or C₁-C₄-alkylthio;
b) a radical -(O)ₘ-NR⁶R⁷,
in which m is 0 or 1 and R⁶ and R⁷, which can be identi-cal or different, have the following meanings:
hydrogen;
C₁-C₈-alkyl, C₃-C₆-alkenyl, C₃-C₆-alkynyl or C₃-C₈-cycloalkyl, these radicals in each case being able to carry one to five halogen atoms and/or one to two of the following groups: C₁-C₄-alkoxy, C₃-C₆-alkenyloxy, C₃-C₆-alkynyloxy, C₁-C₄-alkylthio, C₃-C₆-alkenylthio, C₃-C₆-alkynylthio, C₁-C₄-haloalkoxy, C₁-C₄-alkylcarbonyl, C₃-C₆-alkenyl-carbonyl, C₃-C₆-alkynylcarbonyl, C₁-C₄-alkoxycarbonyl, C₃-C₆-alkenyloxycarbonyl, C₃-C₆-alkynyloxycarbonyl, di-C₁-C₄-alkylamino, C₃-C₈-cycloalkyl, phenyl or phenyl which is mono- or polysubstituted by halogen, nitro, cyano, C₁-C₄-alkyl, C₁-C₄-haloalkyl, C₁-C₄-alkoxy, C₁-C₄-halo-alkoxy or C₁-C₄-alkylthio;
phenyl which can be substituted by one or more of the following radicals: halogen, nitro, cyano, C₁-C₄-alkyl, C₁-C₄-haloalkyl, C₁-C₄-alkoxy, C₁-C₄-haloalkoxy or C₁-C₄-alkylthio;
R⁶ and R⁷ together are an unsubstituted or substituted C₄-C₇-alkylene chain which is closed to give a ring or together are an unsubstituted or substituted C₃-C₆-alkyl-ene chain which is closed to give a ring and contains a heteroatom selected from the group consisting of oxygen, sulfur and nitrogen;
c) R⁵⁰ is additionally a group 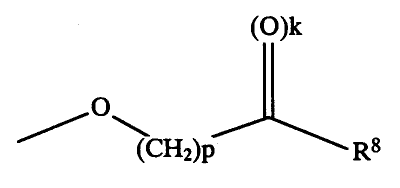 where R⁸ is C₁-C₄-alkyl, phenyl, phenyl which is mono- or polysubstituted by halogen, nitro, cyano, C₁-C₄-alkyl, C₁-C₄-haloalkyl, C₁-C₄-alkoxy, C₁-C₄-haloalkoxy or C₁-C₄-alkylthio, or is C₁-C₄-haloalkyl, C₃-C₆-alkenyl or C₃-C₆-alkynyl, p assumes the values 1, 2, 3 or 4 and k assumes the values 0, 1 or 2;
d) a radical OR⁹, where R⁹ is:
I) a C₃-C₈-cycloalkyl group which can carry one to three C₁-C₄-alkyl radicals;
II) a C₁-C₈-alkyl group which can carry one to five halogen atoms and/or one of the following radicals:
C₁-C₄-alkoxy, C₁-C₄-alkylthio, cyano, C₁-C₄-alkylcarbonyl, C₃-C₈-cycloalkyl, C₁-C₄-alkoxycarbonyl, phenyl, or phenyl or phenoxy which is mono- or polysubstituted by halogen, nitro, cyano, C₁-C₄-alkyl, C₁-C₄-haloalkyl, C₁-C₄-alkoxy, C₁-C₄-haloalkoxy and/or C₁-C₄-alkylthio;
III)a C₁-C₈-alkyl group which can carry one to five halogen atoms and carries one of the following radicals: a 5-membered heteroaromatic, containing one to three nitrogen atoms, or a 5-membered
heteroaromatic containing a nitrogen atom and an oxygen or sulfur atom which can carry one to four halogen atoms and/or one to two of the following radicals: nitro, cyano, C₁-C₄-alkyl, C₁-C₄-haloalkyl, C₁-C₄-alkoxy, C₁-C₄-haloalkoxy and/or C₁-C₄-alkylthio;
IV) a C₂-C₆-alkyl group which in the 2-position carries one of the following radicals: C₁-C₄-alkoxyimino, C₃-C₆-alkenyloxyimino, C₃-C₆-haloalkenyloxyimino or benzyloxyimino;
V) a C₃-C₆-alkenyl group or a C₃-C₆-alkynyl group, where these groups in turn can carry one to five halogen atoms;
VI) a phenyl radical which can carry one to five halogen atoms and/or one to three of the following radicals: nitro, cyano, C₁-C₄-alkyl, C₁-C₄-haloalkyl, C₁-C₄-alkoxy, C₁-C₄-haloalkoxy and/or C₁-C₄-alkylthio;
VII) a 5-membered heteroaromatic linked via a nitrogen atom, containing one to three nitrogen atoms, which can carry one to two halogen atoms and/or one to two of the following radicals: nitro, cyano, C₁-C₄-alkyl, C₁-C₄-haloalkyl, C₁-C₄-alkoxy, C₁-C₄-haloalkoxy and/or C₁-C₄-alkylthio;
VIII) R⁹ is additionally a group -N=CR¹⁰R¹¹, where R¹⁰ and R¹¹, which are identical or different, are:
C₁-C₁₂-alkyl, C₃-C₆-alkenyl, C₃-C₆-alkynyl, C₃-C₈-cycloalkyl, where these radicals can carry a C₁-C₄-alkoxy or C₁-C₄-alkylthio radical and/or a phenyl radical;
phenyl, which can be substituted by one or more of the following radicals:
halogen, nitro, cyano, C₁-C₄-alkyl, C₁-C₄-haloalkyl, C₁-C₄-alkoxy, C₁-C₄-haloalkoxy or C₁-C₄-alkylthio;
or R¹⁰ and R¹¹ together form a C₃-C₁₂-alkylene chain, which can carry one to three C₁-C₄-alkyl groups;
e) or R⁵⁰ forms a radical -NH-SO₂-R¹², where R¹² is:
C₁-C₄-alkyl, C₃-C₆-alkenyl, C₃-C₆-alkynyl or C₃-C₈-cycloalkyl, where these radicals can carry a C₁-C₄-alkoxy or C₁-C₄-alkylthio radical and/or a phenyl radical;
phenyl, which can be substituted by one or more of the following radicals: halogen, nitro, cyano, C₁-C₄-alkyl, C₁-C₄-haloalkyl, C₁-C₄-alkoxy, C₁-C₄-haloalkoxy or C₁-C₄-alkylthio.

5. A herbicidal composition, which contains at least one cyclic acetal of the formula I as claimed in claim 1 and customary inert carriers.

6. A process for controlling undesired plant growth, which comprises applying a herbicidally active amount of a cyclic acetal of the formula I as claimed in claim 1 including compounds where A = hydroxyl if R¹ and R² are both hydrogen or if R¹ is hydrogen and R² is phenyl.

## Revendications

1. Acétals cycliques de formule I 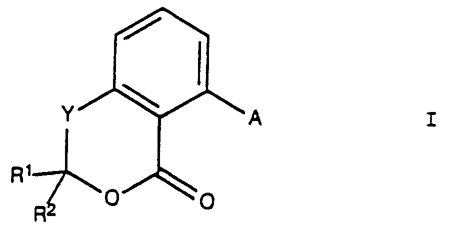 dans laquelle les symboles ont les significations suivantes :
R¹, R²
l'hydrogène ;
un groupe alkyle en C1-C4 qui peut porter un à cinq atomes d'halogènes et/ou un à deux groupes alcoxy en C1-C4 ;
un groupe phényle, qui peut porter un à cinq atomes d'halogènes et/ou un à deux des substituants suivants : alkyle en C1-C4, alcoxy en C1-C4, halogénoalkyle en C1-C4 ou nitro ;
en outre, les deux symboles peuvent représenter ensemble une chaîne alkylène en C2-C6 qui peut elle-même être substituée par un à cinq atomes d'ahlogènes et/ou groupes alkyle en C1-C4 ;
Y
l'oxygène ou le soufre ;
A
l'un des substituants A¹ à A⁶ ;
A¹
un groupe hydroxy ;
A²
un atome d'halogène, un groupe halogénoalkylsulfonyloxy en C1-C4, alkylsulfonyloxy en C1-C4 ou fluorosulfonyloxy ;
A³
un groupe cyano, nitro, formyle ;
A⁴
un cycle phényle ou un hétérocycle saturé ou insature à cinq ou six chaînons contenant dans le cycle jusqu'à quatre hétéroatomes choisis parmi l'azote, le soufre et l'oxygène, chacun de ces substituants pouvant lui-même porter jusqu'à cinq substituants R³¹ à R³⁵ ;
un groupe naphtyle ou un groupe hétéroaromatique à cinq ou six chaînons benzocondensé contenant dans le cycle à un à trois hétéroatomes choisi parmi l'azote, le soufre et l'oxygène et qui peut lui-même porter jusqu'à cinq substituants R³¹ à R³⁵ ;
A⁵
un groupe alcényle en C2-C6, cycloalcényle en C3-C8, cycloalcadiényle en C5-C8 ou alcynyle en C2-C6, chacun d'eux pouvant porter jusqu'à sept substituants R³¹ à R³⁷ ;
A⁶
un groupe alkyle en C1-C8 ou cycloalkyle en C3-C8 qui peut porter jusqu'à sept substituants R³¹ à R³⁷ ;
R³¹, R³², R³³, R³⁴, R³⁵, R³⁶, R³⁷
a) un groupe cycloalkyle en C3-C8 qui peut porter un à trois groupes alkyle en C1-C4 ;
b) un groupe alkyle en C1-C8 qui peut porter un à cinq atomes d'halogènes et/ou un des substituants suivants :
alcoxy en C1-C4, halogénoalcoxy en C1-C4, alkylthio en C1-C4, cyano, cycloalkyle en C3-C8 ou di-(alkyle en C1-C4)amino ;
c) un groupe alcoxy en C1-C8 ou cycloalcoxy en C3-C5 qui peut porter un à cinq atomes d'halogènes et/ou un des substituants suivants :
alcoxy en C1-C4, halogénoalcoxy en C1-C4, alkylthio en C1-C4, cycloalkyle en C3-C8 ou di-(alkyle en C1-C4)amino ;
d) un groupe alkylthio en C1-C4 qui peut porter un à cinq atomes d'halogènes et/ou un des substituants suivants :
alcoxy en C1-C4, halogénoalcoxy en C1-C4, alkylthio en C1-C4, cycloalkyle en C3-C8 ou di-(alkyle en C1-C4)amino ;
e) un groupe di-(alkyle en C1-C4)amino, un groupe di-(alkyle en C1-C4)aminoxy, un groupe (cycloalcane en C5-C8)-iminoxy ou un groupe (alcane en C1-C10)-iminoxy ;
f) un groupe alcényle en C2-C6 ou alcynyle en C2-C6 qui peut porter un à cinq atomes d'halogènes et/ou un des substituants suivants :
alcoxy en C1-C4, halogénoalcoxy en C1-C4 ou alkylthio en C1-C4 ;
g) l'hydrogène ;
h) un groupe nitro, un halogène, un groupe cyano, tri-(alkyle en C1-C4)silyle ;
sous réserve que A ne peut représenter un groupe hydroxy lorsque R¹ et R² représentent tous deux l'hydrogène ou lorsque R¹ représente l'hydrogène et R² un groupe phényle.

2. Procédé de préparation des acétals'cycliques selon la revendication 1 pour lesquels A = A⁴ ou A⁵, caractérisé par le fait que l'on fait réagir des acétals I pour lesquels A = A² avec des composés de formule X - A⁴ ou X - A⁵, R², Y, A², A⁴ et A⁵ ayant les significations indiquées dans la revendication 1 et X représentant l'hydrogène, un groupe trialkylstannyle, dihydroxyboranyle, dialcoxyboranyle ou alkylènedioxybaranyle, en présence d'un composé du palladium possédant une activité catalytique.

3. Procédé pour la préparation des acétals cycliques selon la revendication 1 pour lesquels A = A⁴, caractérisé par le fait que l'on procède à une cycloaddition entre les acétals I pour lesquels A = A⁵ et un diène, hétérodiène ou 1,3-dipôle correspondant.

4. Utilisation des acétals cycliques selon la revendication 1 pour la préparation des substances actives phytosanitaires de formule IV 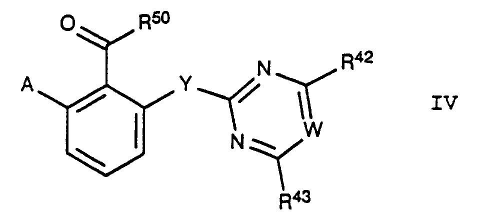 dans laquelle Y et A ont les significations indiquées dans la revendication 1 et les autres symboles ont les significations suivantes :
R⁵⁰
a) un radical hétéroaromatique à cinq chaînons relié par l'intermédiaire d'un atome d'azote, qui contient un à trois atomes d'azote, et qui peut porter un à deux atomes d'halogènes et/ou un à deux des substituants suivants :
alkyle en C1-C4, halogénoalkyle en C1-C4, alcoxy en C1-C4, halogénoalcoxy en C1-C4, et/ou alkylthio en C1-C4 ;
b) un groupe -(O)ₘ-NR⁶R⁷,
dans lequel m est égal à 0 ou 1 et R⁶ et R⁷, identiques ou différents, ont les significations suivantes :
l'hydrogène ;
un groupe alkyle en C1-C8, alcényle en C3-C6, alcynyle en C3-C6, cycloalkyle en C3-C8, chacun de ces groupes pouvant porter un à cinq atomes d'halogènes et/ou un à deux des substituants suivants : alcoxy en C1-C4, alcényloxy en C3-C6, alcynyloxy en C3-C6, alkylthio en C1-C4, alcénylthio en C3-C6, alcynylthio en C3-C6, halogénoalcoxy en C1-C4, (alkyle en C1-C4)carbonyle, (alcényle en C3-C6)carbonyle, (alcynyle en C3-C6)carbonyle, (alcoxy en C1-C4)carbonyle, (alcényloxy en C3-C6)carbonyle, (alcynyloxy en C3-C6)carbonyle, di-(alkyle en C1-C4)amino, cycloalkyle en C3-C8, phényle, phényle portant un ou plusieurs substituants halogéno, nitro, cyano, alkyle en C1-C4, halogénoalkyle en C1-C4, alcoxy en C1-C4, halogénoalcoxy en C1-C4 ou alkylthio en C1-C4 ;
phényle qui peut porter un ou plusieurs des substituants suivants : halogéno, nitro, cyano, alkyle en C1-C4, halogénoalkyle en C1-C4, alcoxy en C1-C4, halogénoalcoxy en C1-C4 ou alkylthio en C1-C4 ;
R⁶ et R⁷ représentent ensemble une chaîne alkylène en C4-C7 refermée en un cycle, éventuellement substituée, ou bien une chaîne alkylène en C3-C6 refermée en un cycle et éventuellement substituée, contenant un hétéroatome choisi parmi l'oxygène, le soufre et l'azote ;
c) R⁵⁰ peut en outre représenter un groupe 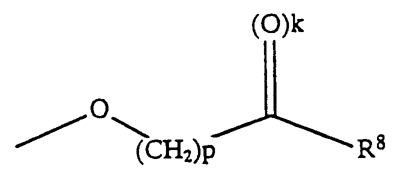 dans laquelle R⁸ représente un groupe alkyle en C1-C4, phényle, phényle portant un ou plusieurs substituants halogéno, nitro, cyano, alkyle en C1-C4, halogénoalkyle en C1-C4, alcoxy en C1-C4, halogénoalcoxy en C1-C4 ou alkylthio en C1-C4 ; halogénoalkyle en C1-C4, alcényle en C3-C6 ou alcynyle en C3-C6, p étant égal à 1, 2, 3 ou 4 et k à 0, 1 ou 2 ;
d) un groupe OR⁹ dans lequel R⁹ représente :
I) un groupe cycloalkyle en C3-C8 qui peut porter un à trois groupes alkyle en C1-C4 ;
II) un groupe alkyle en C1-C8 qui peut porter un à cinq atomes d'halogènes et/ou l'un des substituants suivants :
alcoxy en C1-C4, alkylthio en C1-C4, cyano, (alkyle en C1-C4)carbonyle, cycloalkyle en C3-C8, (alcoxy en C1-C4)carbonyle, phényle, phényle ou phénoxy portant un ou plusieurs substituants halogéno, nitro, cyano, alkyle en C1-C4, halogénoalkyle en C1-C4, alcoxy en C1-C4, halogénoalcoxy en C1-C4 et/ou alkylthio en C1-C4 ;
III) un groupe alkyle en C1-C8 qui peut porter un à cinq atomes d'halogènes et porte l'un des substituants suivants : un radical hétéroaromatique à cinq chaînons contenant un à trois atomes d'azote ou un radical hétéroaromatique à cinq chaînons contenant un atome d'azote et un atome d'oxygène ou de soufre, chacun d'eux pouvant porter un à quatre atomes d'halogènes et/ou un à deux des substituants suivants : nitro, cyano, alkyle en C1-C4, halogénoalkyle en C1-C4, alcoxy en C1-C4, halogénoalcoxy en C1-C4 et/ou alkylthio en C1-C4 ;
IV) un groupe alkyle en C2-C6 qui porte en position 2 l'un des substituants suivants : alcoxyimino en C1-C4, alcényloxyimino en C3-C6, halogénoalcényloxyimino en C3-C6 ou benzyloxyimino ;
V) un groupe alcényle en C3-C6 ou alcynyle en C3-C6 qui peut lui-même porter un à cinq atomes d'halogènes ;
VI) un groupe phényle qui peut porter un à cinq atomes d'halogènes et/ou un à trois des substituants suivants : nitro, cyano, alkyle en C1-C4, halogénoalkyle en C1-C4, alcoxy en C1-C4, halogénoalcoxy en C1-C4 et/ou alkylthio en C1-C4 ;
VII) un radical hétéroaromatique à cinq chaînons relié par l'intermédiaire d'un atome d'azote, qui contient un à trois atomes d'azote et peut porter un à deux atomes d'halogènes et/ou un à deux des substituants suivants : nitro, cyano, alkyle en C1-C4, halogénoalkyle en C1-C4, alcoxy en C1-C4, halogénoalcoxy en C1-C4 et/ou alkylthio en C1-C4 ;
VIII) R⁹ peut en outre représenter un groupe -N=CR¹⁰R¹¹ dans lequel R¹⁰ et R¹¹, qui peuvent avoir des significations identiques ou différentes, représentent chacun :
un groupe alkyle en C1-C12, alcényle en C3-C6, alcynyle en C3-C6, cycloalkyle en C3-C8, chacun d'eux pouvant porter un groupe alcoxy en C1-C4, alkylthio en C1-C4 et/ou un groupe phényle ;
un groupe phényle qui peut porter un ou plusieurs des substituants suivants : halogéno, nitro, cyano, alkyle en C1-C4, halogénoalkyle en C1-C4, alcoxy en C1-C4, halogénoalcoxy en C1-C4 ou alkylthio en C1-C4 ;
ou bien R¹⁰ et R¹¹ représentent ensemble une chaîne alkylène en C3-C12 qui peut porter un à trois groupes alkyle en C1-C4 ;
e) ou bien R⁵⁰ forme un groupe -NH-SO₂-R¹² dans lequel R¹² représente :
un groupe alkyle en C1-C4, alcényle en C3-C6, alcynyle en C3-C6, cycloalkyle en C3-C8, chacun d'eux pouvant porter un groupe alcoxy en C1-C4, alkylthio en C1-C4 et/ou phényle ;
un groupe phényle qui peut porter un ou plusieurs des substituants suivants : halogéno, nitro, cyano, alkyle en C1-C4, halogénoalkyle en C1-C4, alcoxy en C1-C4, halogénoalcoxy en C1-C4 ou alkylthio en C1-C4.

5. Produit herbicide caractérisé par le fait qu'il contient au moins un acétal cyclique de formule I de la revendication 1 et des véhicules inertes usuels.

6. Procédé pour combattre les croissances de végétaux indésirables, caractérisé par le fait que l'on utilise une quantité herbicide efficace d'un acétal cyclique de formule I de la revendication 1, y compris de composés pour lesquels A = hydroxy lorsque R¹ et R² représentent tous deux l'hydrogène ou lorsque R¹ représente l'hydrogène et R² un groupe phényle.
